# EUROPEAN PATENT APPLICATION

(11) **EP 3 369 431 A1**
(43) Date of publication of application: **05.09.2018**
(21) Application number: 17159242.1
(22) Date of filing: 03.03.2017
(51) Int. Cl.: A61K 39/00, G01N 33/50, G01N 33/569

(54) **VACCINE**

(71) Applicant: TREOS BIO KFT, 8200 Veszprem (HU)
(72) Inventor: LISZIEWICZ, Julianna, 8220 Balatonalmadi (HU); MOLNAR, Levente, 2013 Pomáz (HU); TOKE, Eniko R., 1089 Budapest (HU); TOTH, József, 9012 Gyor (HU); LORINCZ, Orsolya, 1047 Budapest (HU); CSISZOVSZKI, Zsolt, 1119 Budapest (HU); SOMOGYI, Eszter, 8220 Balatonalmádi (HU); PANTYA, Katalin, 1063 Budapest (HU); MEGYESI, Mónika, 3441 Mezokeresztes (HU)
(74) Representative: J A Kemp

(57) **Abstract**

The invention relates to peptides and pharmaceutical compositions comprising peptides that find use in the prevention or treatment of cancer, in particular breast cancer and colorectal cancer. The invention also relates to methods of inducing a cytotoxic T cell response in a subject or treating cancer by administering pharmaceutical compositions comprising the peptides, and companion diagnostic methods of identifying subjects for treatment. The peptides comprise T cell epitopes that are immunogenic in a high percentage of patients.

## Description

### Field of Invention

The invention relates to peptide vaccines that find use in the prevention or treatment of cancer, in particular breast cancer and colorectal cancer. We invented the process to make HLA-specific cancer vaccines for a large patient population. This invention discloses two vaccine composition suitable for most colorectal and breast cancer patients.

### Background to the invention

Cancer is killing millions of people worldwide, because existing drugs do not enable effective prevention or treatment. Current checkpoint inhibitor immunotherapies that re-activate existing immune responses can provide clinical benefit for a fraction of cancer patients. Current cancer vaccines that induce new immune responses are poorly immunogenic and fail to benefit most patients.

Recent analyses of 63,220 unique tumors revealed that cancer vaccines need to be generated specifically for each patient because extensive inter-individual tumor genomic heterogeneity (Hartmaier et al. Genome Medicine 2017 9:16). Using state of art technologies it is currently not feasible to scale HLA-specific cancer vaccines to large populations.

### Summary of the invention

In antigen presenting cells (APC) protein antigens are processed into peptides. These peptides bind to human leukocyte antigen molecules (HLAs) and are presented on the cell surface as peptide-HLA complexes to T cells. Different individuals express different HLA molecules and different HLA molecules present different peptides. Therefore, according to the state of the art, a peptide, or a fragment of a larger polypeptide, is identified as immunogenic for a specific human subject if it is presented by a HLA molecule that is expressed by the subject. In other words, the state of the art describes immunogenic peptides as HLA-restricted epitopes. However, HLA restricted epitopes induce T cell responses in only a fraction of individuals who express the HLA molecule. Peptides that activate a T cell response in one individual are inactive in others despite HLA allele matching. Therefore, it was previously unknown how an individual's HLA molecules present the antigen-derived epitopes that positively activate T cell responses.

The inventors have discovered that multiple HLAs expressed by an individual need to present the same peptide in order to trigger a T cell response. The fragments of a polypeptide antigen that are immunogenic for a specific individual are those that can bind to multiple class I (activate cytotoxic T cells) or class II (activate helper T cells) HLAs expressed by that individual. For example, the inventors have discovered that the presence of a T cell epitope that binds to at least three HLA type I of a subject predicts an immune response in the subject to a polypeptide.

Based on this discovery the inventors have identified the T cell epitopes of certain breast and/or colorectal cancer associated polypeptide antigens (cancer testis antigens (CTA)) that are capable of binding to at least three class I HLA in a high proportion of individuals. These T cell epitopes, or fragments of the antigens comprising the T cell epitopes, are useful for inducing cancer specific immune responses and for treating cancer.

In a first aspect the invention provides a peptide that
(a) is a fragment of a breast cancer associated antigen preferably selected from SPAG9, AKAP-4, BORIS, NY-SAR-35, NY-BR-1, SURVIVIN, MAGE-A11, PRAME, MAGE-A9, and HOM-TES-85, optionally wherein the fragment comprises or consists of the amino acid sequence of any one of SEQ ID NOs: 1 to 20; or
(b) consists of two or more fragments of one or more breast cancer associated antigens preferably selected from SPAG9, AKAP-4, BORIS, NY-SAR-35, NY-BR-1, SURVIVIN, MAGE-A11, PRAME, MAGE-A9 and HOM-TES-8 wherein the fragments overlap or are arranged end to end in the peptide and optionally wherein each fragment comprises or consists of a different amino acid sequence selected from SEQ ID NOs: 1 to 20; or
(c) is a fragment of a colorectal cancer-associated antigen preferably selected from TSP50, EpCAM, SPAG9, CAGE1, FBXO39, SURVIVIN, MAGE-A8 and MAGE-A6, optionally wherein the fragment comprises or consists of the amino acid sequence of any one of SEQ ID NOs: 21 to 40; or
(d) consists of two or more fragments of one or more colorectal cancer associated antigens preferably selected from TSP50, EpCAM, SPAG9, CAGE1, FBXO39, SURVIVIN, MAGE-A8 and MAGE-A6, wherein the fragments overlap or are arranged end to end in the peptide and optionally wherein each fragment comprises or consists of a different amino acid sequence selected from SEQ ID NOs: 21 to 40.

In some cases the peptide comprises or consists of fragments of
(a) at least two different breast cancer-associated antigens preferably selected from SPAG9, AKAP-4, BORIS, NY-SAR-35, NY-BR-1, SURVIVIN, MAGE-A11, PRAME, MAGE-A9 and HOM-TES-8, optionally wherein each fragment comprises or consists of a different amino acid sequence selected from SEQ ID NOs: 1 to 20; or
(b) at least at least two different colorectal cancer-associated antigens preferably selected from TSP50, EpCAM, SPAG9, CAGE1, FBXO39, SURVIVIN, MAGE-A8 and MAGE-A6, optionally wherein each fragment comprises or consists of a different amino acid sequence selected from SEQ ID NOs: 21 to 40.

In some cases the peptide comprises or consists of one or more amino acid sequences selected from SEQ ID NOs: 41-80.

In some cases the invention provides a peptide that comprises or consists of the amino acid sequence of any one of SEQ ID NOs: 81-142.

In a further aspect the invention provides a panel of two or more peptides as described above, wherein each peptide comprises or consists of a different amino acid sequence selected from SEQ ID NOs: 1 to 20; or each peptide comprises or consists of a different amino acid sequence selected from SEQ ID NOs: 21 to 40. In some cases the panel of peptides comprises or consists of nine peptides having the amino acid sequences of SEQ ID NOs: 92, 93, 98, 99, 100, 101, 103, 104, and 105. In some cases the panel of peptides comprises nine peptides having the amino acid acid sequences of SEQ ID NOs: 121, 124, 126, 127, 130, 131, 132, 133 and 134.

In a further aspect the invention provides a pharmaceutical composition having a peptide or a panel of peptides as described above as active ingredients.

In a further aspect the invention provides a method of inducing a cytotoxic T cell response in a subject, the method comprising administering to the subject a pharmaceutical composition as described above. The method may be a method of treating cancer, such as breast cancer or colorectal cancer.

In further aspects, the invention provides
- the pharmaceutical composition described above for use in a method of inducing a cytotoxic T cell response or for use in a method of treating cancer, optionally breast cancer or colorectal cancer; and
- use of a peptide or a panel of peptides as described above in the manufacture of a medicament for inducing a cytotoxic T cell response or for treating cancer, optionally breast cancer or colorectal cancer.

In a further aspect the invention provides a method of identifying a human subject who will likely have a cytotoxic T cell response to administration of a pharmaceutical composition as described above, the method comprising
(i) determining the HLA class I genotype of a human subject;
(ii) determining that the active ingredient peptide(s) of the pharmaceutical composition comprise a sequence that is a T cell epitope capable of binding to at least three HLA class I of the subject; and
(iii) identifying the subject as likely to have a cytotoxic T cell response to administration of the pharmaceutical composition.

In a further aspect the invention provides a method of identifying a subject who will likely have a clinical response to a method of treatment as described above, the method comprising
(i) determining the HLA class I genotype of the subject;
(ii) determining that one or more cancer-associated antigen selected from SPAG9, AKAP-4, BORIS, NY-SAR-35, NY-BR-1, SURVIVIN, MAGE-A11, PRAME, MAGE-A9, HOM-TES-85, TSP50, EpCAM, CAGE1, FBXO39, MAGE-A8 and MAGE-A6 is expressed by cancer cells of the subject;
(iii) determining that the active ingredient peptide(s) of the pharmaceutical composition comprise two or more different amino acid sequences each of which is
   a. a fragment of a cancer-associated antigen expressed by cancer cells of the subject as determined in step (ii); and
   b. a T cell epitope capable of binding to at least three HLA class I of the subject; and
(iv) identifying the subject as likely to have a clinical response to the method of treatment.

In some cases administration of the pharmaceutical composition may then be selected as a method of treatment for the subject. The subject further be treated by administration of the pharmaceutical composition.

In a further aspect the invention provides a method of treatment as described above, wherein the subject has been identified as likely to have a clinical response to the treatment by the method described above.

In a further aspect the invention provides a method of identifying a human subject who will likely not have a clinical response to a method of treatment as described above, the method comprising
(i) determining the HLA class I genotype of a human subject;
(ii) determining that the active ingredient peptide(s) of the pharmaceutical composition do not comprise two or more different amino acid sequences each of which is a T cell epitope capable of binding to at least three HLA class I of the subject; and
(iii) identifying the subject as likely not to have a clinical response to the method of treatment.

The invention will now be described in more detail, by way of example and not limitation, and by reference to the accompanying drawings. Many equivalent modifications and variations will be apparent, to those skilled in the art when given this disclosure. Accordingly, the exemplary embodiments of the invention set forth are considered to be illustrative and not limiting. Various changes to the described embodiments may be made without departing from the scope of the invention. All documents cited herein, whether supra or infra, are expressly incorporated by reference in their entirety.

The present invention includes the combination of the aspects and preferred features described except where such a combination is clearly impermissible or is stated to be expressly avoided. As used in this specification and the appended claims, the singular forms "a", "an", and "the" include plural referents unless the content clearly dictates otherwise. Thus, for example, reference to "a peptide" includes two or more such peptides.

Section headings are used herein for convenience only and are not to be construed as limiting in any way.

### Description of the Figures

**Fig.1**
   ROC curve of HLA restricted PEPI biomarkers.
**Fig**. **2**
   ROC curve of ≥1 PEPI3+ Test for the determination of the diagnostic accuracy.
**Fig**. **3**
   Distribution of HLA class I PEPI3+ compared to CD8+ T cell responses measured by a state of art assay among peptide pools used in the CD8+ T cell response assays. **A:** HLA class I restricted PEPI3+s. The 90% Overall Percent of Agreement (OPA) among the T cell responses and PEPI3+ peptides demonstrate the utility of the invented peptides for prediction of vaccine induced T cell response set of individuals. **B**: Class I HLA restricted epitopes (PEPI1+). The OPA between predicted epitopes and CD8+ T cell responses was 28% (not significant). Darkest grey: True positive (TP), both peptide and T cell responses were detected; Light grey: False negative (FN), only T cell responses were detected; Lightest grey: False positive (FP), only peptide were detected; Dark grey: True negative (TN): neither peptides nor T cell responses were detected.
**Fig**. **4**
   Distribution of HLA class II PEPIs compared to CD4+ T cell responses measured by a state of art assay among peptide pools used in the assays. **A:** HLA class II restricted PEPI4+s. 67% OPA between PEPI4+ and CD4+ T-cell responses (p=0.002). **B**: The class II HLA restricted epitopes. OPA between class II HLA restricted epitopes and CD4+ T cell responses was 66% (statistically not significant). Darkest grey: True positive (TP), both peptide and T cell responses were detected; Light grey: False negative (FN), only T cell responses were detected; Lightest grey: False positive (FP), only peptide were detected; Dark grey: True negative (TN): neither peptides nor T cell responses were detected.
**Fig**. **5**
   Multiple HLA binding peptides that define the HPV-16 LPV vaccine specific T cell response set of 18 VIN-3 and 5 cervical cancer patients. HLA class I restricted PEPI3 counts (**A** and **B**) and HLA class II restricted PEPI3 counts (**C** and **D**) derived from LPV antigens of each patient. Light grey: immune responders measured after vaccination in the clinical trial; Dark grey: Immune non-responders measured after vaccination in the clinical trial. Results show that ≧ 3 HLA class I binding peptides predict the CD8+ T cell reactivity and ≧4 HLA class II binding peptides predict the CD4+ T cell reactivity.
**Fig. 6**
   The multiple HLA class I binding peptides that define the HPV vaccine specific T cell response set of 2 patients. **A:** Four HPV antigens in the HPV vaccine. Boxes represent the length of the amino acid sequences from the N terminus to the C terminus. **B:** Process to identify the multiple HLA binding peptides of two patients: HLA sequences of the patients labelled as 4-digit HLA genotype right from the patient's ID. The location of the 1^{st} amino acid of the 54 and 91 epitopes that can bind to the patient 12-11 and patient 14-5 HLAs (PEPI1+) respectively are depicted with lines. PEPI2 represents the peptides selected from PEPI1+s that can bind to multiple HLAs of a patient (PEPI2+). PEPI3 represent peptides that can bind to ≧3 HLAs of a patient (PEPI3+). PEPI4 represent peptides that can bind to ≧4 HLAs of a patient (PEPI4+). PEPI5 represent peptides that can bind to ≧5 HLAs of a patient (PEPI5+). PEPI6 represent peptides that can bind to 6 HLAs of a patient (PEPI6). **C**: The DNA vaccine specific PEPI3+ set of two patients characterizes their vaccine specific T cell responses.
**Fig**. **7**
   Correlation between the ≧ 1 PEPI3+ Score and CTL response rates of peptide targets determined in clinical trials.
**Fig. 8**
   Correlation between the ≧1 PEPI3+ Score and the clinical Immune Response Rate (IRR) of immunotherapy vaccines. Dashed lines: 95% confidence band.
**Fig. 9**
   Correlation between the ≧2 PEPI3+ Score and Disease Control Rate (DCR) of immunotherapy vaccines. Dashed lines: 95% confidence band.
**Fig**. **10**
   Peptide hotspot analysis example: PRAME antigen hotspot on 433 patients of the Model Population. On the y axis are the 433 patients of the Model Population, on the x axis is the amino acid sequence of the PRAME antigen (CTA). Each data point represents a PEPI presented by ≧ 3 HLA class I of one patient starting at the specified amino acid position. The two most frequent PEPIs (called bestEPIs) of the PRAME antigen are highlighted in dark gray (peptide hotspots = PEPI Hotspots).
**Fig**. **11**
   CTA Expression Curve calculated by analyzing expression frequency data of tumor specific antigens (CTAs) in human breast cancer tissues. (No cell line data were included.)
**Fig. 12**
   Antigen expression distribution for breast cancer based on the calculation of multi-antigen responses from expression frequencies of the selected 10 different CTAs. **A:** non-cumulative distribution to calculate the expected value for the number of expressed antigens (AG50). This value shows that probably 6.14 vaccine antigens will be expressed by breast tumor cells. **B**: cumulative distribution curve of the minimum number of expressed antigens (CTA expression curve). This shows that minimum 4 vaccine antigens will be expressed with 95% probability in breast cancer cell (AG95).
**Fig. 13**
   PEPI representing antigens: breast cancer vaccine-specific CTA antigens with ≥1 PEPI, called as "AP") distribution within the Model Population (n=433) for breast cancer vaccine. **A:** non-cumulative distribution of AP where the average number of APs is: AP50=5.30, meaning that in average almost 6 CTAs will have PEPIs in the Model Population. **B**: cumulative distribution curve of the minimum number of APs in the Model Population (n=433). This shows that at least one vaccine antigen will have PEPIs in 95% of the Model Population (n=433) (AP95=1).
**Fig**. **14**
   PEPI represented expressed antigen (breast cancer vaccine-specific CTA antigens expressed by the tumor, for which ≥1 PEPI is predicted, called as "AGP") distribution within the model population (n=433) calculated with CTA expression rates for breast cancer. **A:** non-cumulative distribution of AGP where the expected value for number expressed CTAs represented by PEPI is AGP50=3.37. AGP50 is a measure of the effectiveness of the disclosed breast cancer vaccine in attacking breast tumor in an unselected patient population. AGP50 = 3.37 means that at least 3 CTAs from the vaccine will probably be expressed by the breast tumor cells and present PEPIs in the Model Population. **B**: cumulative distribution curve of the minimum number of AGPs in the Model Population (n=433) shows that at least 1 of the vaccine CTAs will present PEPIs in 92% of the population and the remaining 8% of the population will likely have no AGP at all (AGP95=0, AGP92=1).
**Fig**. **15**
   CTA Expression Curve calculated by analyzing expression frequency data of tumor specific antigens (CTAs) in human colorectal cancer tissues. (No cell line data were included.)
**Fig**. **16**
   Antigen expression distribution for colorectal cancer based on the calculation of multi-antigen responses from expression frequencies of the selected 8 different CTAs. A: non-cumulative distribution to calculate the expected vale for the number of expressed vaccine antigens in colorectal cancers (AG50). This value shows that probably 5.22 vaccine antigens will be expressed by colorectal tumor cells. B: cumulative distribution curve of the minimum number of expressed antigens (CTA expression curve). This shows that minimum 3 antigens will be expressed with 95% probability in the colorectal cancer cell (AG95).
**Fig. 17**
   PEPI represented antigen (colorectal cancer vaccine-specific CTA antigens for which ≥1 PEPI is predicted. Called as "AP") distribution within the model population (n=433) for colorectal cancer. **A:** non-cumulative distribution of AP where the average number of APs is: AP50=4.73, meaning that in average 5 CTAs will be represented by PEPIs in the model population **B**: cumulative distribution curve of the minimum number of APs in the model population (n=433). This shows that 2 or more antigens will be represented by PEPIs in 95% of the modell population (n=433) (AP95=2).
**Fig**. **18**
   PEPI represented expressed antigen (colorectal cancer vaccine-specific CTA antigens expressed by the tumor, for which ≥1 PEPI is predicted. Called as "AGP") distribution within the model population (n=433) calculated with CTA expression rates for colorectal cancer. **A:** non-cumulative distribution of AGP where the expected value for number expressed CTAs represented by PEPI is AGP50=3.16. AGP50 is a measure of the effectiveness of the disclosed colorectal cancer vaccine in attacking colorectal tumors in an unselected patient population. AGP50 = 3.16 means that at least 3 CTAs from the vaccine will probably be expressed by the colorectal tumor cells and present PEPIs in the Model Population. **B**: cumulative distribution curve of the minimum number of AGPs in the Model Population (n=433) shows that at least 1 of the vaccine CTAs will be expressed and also present PEPIs in 95% of the population (AGP95=1).

### Description of the Sequences

SEQ ID NOs: 1 to 20 set forth the T cell epitopes described in Table 17.
SEQ ID NOs: 21 to 40 set forth the T cell epitopes described in Table 20.
SEQ ID NOs 41 to 80 set forth additional longer peptide sequence each comprising the sequence of one of SEQ ID NOs: 1 to 40.
SEQ ID NOs: 81 to 111 set forth the breast cancer vaccine peptides set forth in Table 18a.
SEQ ID NOs 112 to 142 set forth the colorectal cancer vaccine peptides set forth in Table 21 a.
SEQ ID NOs 143 to 158 set forth breast cancer and/or colorectal cancer associated antigens.
SEQ ID NOs 159 to 171 set forth the additional peptide sequences described in Table 10.

### Detailed Description

### HLA Genotypes

HLAs are encoded by the most polymorphic genes of the human genome. Each person has a maternal and a paternal allele for the three HLA class I molecules (HLA-A*, HLA-B*, HLA-C*) and four HLA class II molecules (HLA-DP*, HLA-DQ*, HLA-DRB1*, HLA-DRB3*/4*/5*). Practically, each person expresses a different combination of 6 HLA class I and 8 HLA class II molecules that present different epitopes from the same protein antigen. The function of HLA molecules is to regulate T cell responses. However up to date it was unknown how the HLAs of a person regulate T cell activation.

The nomenclature used to designate the amino acid sequence of the HLA molecule is as follows: gene name*allele:protein number, which, for instance, can look like: HLA-A*02:25. In this example, "02" refers to the allele. In most instances, alleles are defined by serotypes - meaning that the proteins of a given allele will not react with each other in serological assays. Protein numbers ("25" in the example above) are assigned consecutively as the protein is discovered. A new protein number is assigned for any protein with a different amino acid sequence (e.g. even a one amino acid change in sequence is considered a different protein number). Further information on the nucleic acid sequence of a given locus may be appended to the HLA nomenclature, but such information is not required for the methods described herein.

The HLA class I genotype or HLA class II genotype of an individual may refer to the actual amino acid sequence of each class I or class II HLA of an individual, or may refer to the nomenclature, as described above, that designates, minimally, the allele and protein number of each HLA gene. An HLA genotype may be determined using any suitable method. For example, the sequence may be determined via sequencing the HLA gene loci using methods and protocols known in the art. Alternatively, the HLA set of an individual may be stored in a database and accessed using methods known in the art.

### Peptides

The invention relates to peptides that are derived from CTAs and that are immunogenic for a high proportion of the human population.

As used herein, the term "polypeptide" refers to a full-length protein, a portion of a protein, or a peptide characterized as a string of amino acids. As used herein, the term "peptide" refers to a short polypeptide comprising between 2, or 3, or 4, or 5 or 6 or 7 or 8 or 9 and 20, or 25, or 30, or 35, or 40, or 45, or 50 amino acids.

The terms "fragment", "fragment of a polypeptide" or "fragment of a peptide" as used herein refer to a string of amino acids or an amino acid sequence that may be of reduced length relative to the reference polypeptide and comprising, over the common portion, an amino acid sequence identical to the reference polypeptide. Such a fragment according to the invention may be, where appropriate, included in a larger polypeptide of which it is a constituent. In some cases the fragments referred to herein may be between 2, or 3, or 4, or 5 or 6 or 7 or 8 or 9 and 20, or 25, or 30, or 35, or 40, or 45, or 50 amino acids.

As used herein, the term "epitope" or "T cell epitope" refers to a sequence of contiguous amino acids contained within a protein antigen that possess a binding affinity for (is capable of binding to) one or more HLAs. An epitope is HLA- and antigen-specific (HLA-epitope pairs, predicted with known methods), but not subject specific, since a given epitope is recognised by the specific HLAs of some individuals and not others. An epitope, a T cell epitope, a polypeptide, a fragment of a polypeptide or a composition comprising a polypeptide or a fragment thereof is "immunogenic" for a specific human subject if it is capable of inducing a T cell response (a cytotoxic T cell response or a helper T cell response) in that subject. The terms "T cell response" and "immune response" are used herein interchangeably, and refer to the activation T cells and/or the induction of one or more effector functions following recognition of one or more HLA-epitope binding pairs. Effector functions include cytotoxicity, cytokine production and proliferation. According to the present disclosure, an epitope, a T cell epitope, or a fragment of a polypeptide is immunogenic for a specific subject if it is capable of binding to at least three class I or at least four class II HLA of the subject.

For the purposes of this disclosure we have coined the term "personal epitope", or "PEPI". A "PEPI" is a fragment of a polypeptide consisting of a sequence of contiguous amino acids of the polypeptide that is a T cell epitope capable of binding to one or more HLA class I molecules or one or more HLA class II molecules of a specific human subject. In other words a "PEPI" is a T cell epitope that is recognised by the HLA set of a specific individual. In contrast to an "epitope", which is an HLA restricted peptide, PEPIs are specific to an individual because different individuals have different HLA molecules which each bind to different T cell epitopes.

"PEPI1" as used herein refers to a peptide, or a fragment of a polypeptide, that can bind to one HLA class I of an individual. "PEPI1+" refers to a peptide, or a fragment of a polypeptide, that can bind to one or more HLA class I of an individual.

"PEPI2" refers to a peptide, or a fragment of a polypeptide, that can bind to two HLA class I of an individual. "PEPI2+" refers to a peptide, or a fragment of a polypeptide, that can bind to two or more HLA class I of an individual, i.e. a fragment identified according to a method of the invention.

"PEPI3" refers to a peptide, or a fragment of a polypeptide, that can bind to three HLA class I of an individual. "PEPI3+" refers to a peptide, or a fragment of a polypeptide, that can bind to three or more HLA class I of an individual.

"PEPI4" refers to a peptide, or a fragment of a polypeptide, that can bind to four HLA class I of an individual. "PEPI4+" refers to a peptide, or a fragment of a polypeptide, that can bind to four or more HLA class I of an individual.

"PEPI5" refers to a peptide, or a fragment of a polypeptide, that can bind to five HLA class I of an individual. "PEPI5+" refers to a peptide, or a fragment of a polypeptide, that can bind to five or more HLA class I of an individual.

"PEPI6" refers to a peptide, or a fragment of a polypeptide, that can bind to all 6 HLA class I of an individual.

Generally speaking, epitopes presented by HLA class I molecules are about nine amino acids long and epitopes presented by HLA class II molecules are about fifteen amino acids long. For the purposes of this disclosure, however, an epitope may be more or less than nine (for HLA Class I) or fifteen (for HLA Class II) amino acids long, as long as the epitope is capable of binding HLA. For example, an epitope that is capable of binding to class I HLA may be between 7, or 8 or 9 and 9 or 10 or 11 amino acids long. An epitope that is capable of binding to a class II HLA may be between 13, or 14 or 15 and 15 or 16 or 17 amino acids long.

A given HLA of a subject will only present to T cells a limited number of different peptides produced by the processing of protein antigens in an APC. As used herein, "display" or "present", when used in relation to HLA, references the binding between a peptide (epitope) and an HLA. In this regard, to "display" or "present" a peptide is synonymous with "binding" a peptide.

Using techniques known in the art, it is possible to determine the epitopes that will bind to a known HLA. Any suitable method may be used, provided that the same method is used to determine multiple HLA-epitope binding pairs that are directly compared. For example, biochemical analysis may be used. It is also possible to use lists of epitopes known to be bound by a given HLA. It is also possible to use predictive or modelling software to determine which epitopes may be bound by a given HLA. Examples are provided in Table 1.

**Table 1**

| **EPITOPE PREDICTION TOOLS** | **WEB LINK** |
|---|---|
| BIMAS, NIH | www-bimas.cit.nih.gov/molbio/hla bind/ |
| PPAPROC, Tubingen Univ. | |
| MHCPred, Edward Jenner Inst. of Vaccine Res. | |
| EpiJen, Edward Jenner Inst. of Vaccine Res. | http://www.ddg-pharmfac.net/epijen/EpiJen/EpiJen.htm |
| NetMHC, Center for Biological Sequence Analysis | http://www.cbs.dtu.dk/services/NetMHC/ |
| SVMHC, Tubingen Univ. | http://abi.inf.uni-tuebingen.de/Services/SVMHC/ |
| SYFPEITHI, Biomedical Informatics, Heidelberg | http://www.syfpeithi.de/bin/MHCServer.dll/EpitopePrediction. htm |
| ETK EPITOOLKIT, Tubingen Univ. | http://etk.informatik.uni-tuebingen.de/epipred/ |
| PREDEP, Hebrew Univ. Jerusalem | http://margalit.huji.ac.il/Teppred/mhc-bind/index.html |
| RANKPEP, MIF Bioinformatics | http://bio.dfci.harvard.edu/RANKPEP/ |
| IEDB, Immune Epitope Database | http://tools.immuneepitope.org/main/html/tcell tools.html |

| **EPITOPE DATABASES** | **web link** |
|---|---|
| MHCBN, Institute of Microbial Technology, Chandigarh, INDIA | http://www.imtech.res.in/raghava/mhcbn/ |
| SYFPEITHI, Biomedical Informatics, Heidelberg | http://www.svfpeithi.de/ |
| AntiJen, Edward Jenner Inst. of | http://www.ddg- |
| Vaccine Res. | pharmfac.net/antijen/AntiJen/antijenhomepage.htm |
| EPIMHC database of MHC ligands, MIF Bioinformatics | http://immunax.dfci.harvard.edu/epimhc/ |
| IEDB, Immune Epitope Database | http://www.iedb.org/ |

The peptides of the invention may comprise or consist of one or more fragments of one or more CTAs. CTAs are not typically expressed beyond embryonic development in healthy cells. In healthy adults, CTA expression is limited to male germ cells that do not express HLAs and cannot present antigens to T cells. Therefore, CTAs are considered expressional neoantigens when expressed in cancer cells.

CTAs are a good choice for cancer vaccine targets because their expression is (i) specific for tumor cells, (ii) more frequent in metastases than in primary tumors and (iii) conserved among metastases of the same patient (Gajewski ed. Targeted Therapeutics in Melanoma. Springer New York. 2012).

The peptides of the invention may comprise or consist of one or more fragments of one or more breast cancer associated antigens selected from SPAG9 (SEQ ID NO: 143), AKAP-4 (SEQ ID NO: 144), BORIS (SEQ ID NO: 145), NY-SAR-35 (SEQ ID NO: 146), NY-BR-1 (SEQ ID NO: 147), SURVIVIN (SEQ ID NO: 148), MAGE-A11 (SEQ ID NO: 149), PRAME (SEQ ID NO: 150), MAGE-A9 (SEQ ID NO: 151) or HOM-TES-85 (SEQ ID NO: 152); or one or more colorectal cancer-associated antigens selected from TSP50 (SEQ ID NO: 153), EpCAM (SEQ ID NO: 154), SPAG9 (SEQ ID NO: 143), CAGE1 (SEQ ID NO: 155), FBXO39 (SEQ ID NO: 156), SURVIVIN (SEQ ID NO: 148), MAGE-A8 (SEQ ID NO 157): and MAGE-A6 (SEQ ID NO: 158). In some cases the peptide comprises or consists of one or more amino acid sequences selected from SEQ ID NOs: 41-80, that are optimised for T cell activation / binding to all HLA types across the population.

The inventors have discovered that the presence in a vaccine or immunotherapy composition of at least two T cell epitopes that can bind to at least three HLA of an individual (≥2 PEPI3+) is predictive for a clinical response in a specific subject. In other words, if ≥2 PEPI3+ can be identified within the active ingredient polypeptide(s) of a vaccine or immunotherapy composition, then an individual is a likely clinical responder. The at least two multiple HLA-binding PEPIs of the composition polypeptides may both target a single antigen (e.g a polypeptide vaccine comprising two multiple HLA-binding PEPIs derived from a single tumor associated antigen targeted by the vaccine) or may target different antigens (e.g. a polypeptide vaccine comprising one multiple HLA-binding PEPI derived from one tumor associated antigen and a second multiple HLA-binding PEPI derived from a different tumor associated antigen).

Without wishing to be bound by theory, the inventors believe that one reason for the increased likelihood of deriving clinical benefit from a vaccine or immunotherapy composition comprising at least two multiple-HLA binding PEPIs, is that tumor similarly to HIV consist of heterogeneous cell population expressing different tumor associated antigens and therefore effective tumor therapy similarly to an effective HIV therapy must consist of combination of drug with different targets. In addition, a patient is less likely to develop resistance to the composition through mutation of the target epitope. The likelihood of developing resistance is decreased when more multiple HLA-binding PEPIs are included.

Accordingly in some cases, the peptide of the invention may comprise or consist of two or more fragments of one or more of the CTA discussed above, wherein each fragment comprises or consists of a different target epitope having an amino acid sequence selected from SEQ ID NOs. 1 to 40, SEQ ID NOs. 1 to 20, or SEQ ID NOs. 21 to 40, optionally excluding SEQ ID NOs: 12 or 19, and/or SEQ ID NOs: 21 or 23. A peptide of the invention may comprise or consist of the amino acid sequence of any one of SEQ ID NOs: 81 to 142, which were selected to minimise cytotoxicity.

In other cases, the peptide of the invention may comprise or consist of three, four or five fragments of one or more of the CTA discussed above, wherein each fragment comprises or consists of a different target epitope having an amino acid sequence selected from SEQ ID NOs: 1 to 40.

Currently most vaccines and immunotherapy compositions target only a single polypeptide antigen. However according to the present invention it is in some cases beneficial to target multiple cancer associated antigens. For example, most cancers or tumors are heterogeneous, meaning that different cancer or tumor cells of a subject express different antigens. The anti-cancer immunogenic compositions that are most likely to be effective are those that target multiple antigens expressed by the tumor, and therefore more of the cancer or tumor cells of a subject.

Therefore in some cases a peptide of the invention comprises or consists of fragments of at least two different breast cancer associated antigens, or at least two different colorectal cancer associated antigens as described above. In some cases the peptide comprises or consists of fragments of three, four or five different breast cancer associated antigens, or of three, four or five different colorectal cancer associated antigens, as described above.

In some cases the invention provides a panel of any two or more of the peptides described above. For example the panel may comprise 2, 3, 4, 5, 6, 7, 8, 9, 10, 11, 12, 13, 14, 15, 16, 17, 18, 19, 20, 21, 22, 23, 24, 25 or more such peptides, for example 2, 3, 4, 5, 6, 7, 8, 9, 10, 11, 12, 13, 14, 15, 16, 17, 18, 19, 20, 21, 22, 23, 24, 25 or more peptides, each comprising or consisting of a different amino acid sequences selected from SEQ ID NOs: 81 to 142; or selected from SEQ ID NOs: 81 to 82, 83, 84, 85, 86, 87, 88, 89, 90, 91, 92, 93, 94, 95, 96, 97, 98, 99, 100, 101, 102, 103, 105, 106, 107, 108, 109, 110, or 111; or selected from SEQ ID NOs: 81 to 105; or selected from SEQ ID NOs: 99, 100, 92, 93, 101, 103, 104, 105 and 98; or selected from SEQ ID NOs: 112 to 142; or selected from SEQ ID NOs: 112 to 113, 114, 115, 116, 117, 118, 119, 120, 121, 123, 124, 125, 126, 127, 128, 129, 130, 131, 132, 133, 134, 135, 136, 137, 138, 139, 140, 141 or 142; or selected from SEQ ID NOs: 112 to 134; or selected from SEQ ID NOs: 121, 124, 126, 127, 130, 131, 132, 133 and 134. In some cases the panel comprises or consists of peptides comprising or consisting of the amino acid sequences of SEQ ID NOs: 99, 100, 92, 93, 101, 103, 104, 105 and 98. In some cases the panel comprises or consists of peptides comprising or consisting of the amino acid sequences of SEQ ID NOs: 121, 124, 126, 127, 130, 131, 132, 133 and 134.

### Pharmaceutical Compositions, Methods of Treatment and Modes of Administration

In some aspects the invention relates to a pharmaceutical composition having a peptide or a panel of peptides as described above as active ingredient(s). The pharmaceutical composition may be a vaccine or immunotherapy composition. The term "active ingredient" as used herein refers to a polypeptide that is intended to induce an immune response and may include a polypeptide product of a vaccine or immunotherapy composition that is induced *in vivo* by cells after administration to a subject. For a DNA or RNA vaccine/immunotherapy composition, the polypeptide that is intended to induce immune responses may be induced *in vivo* in the cells of a subject to whom the composition is administered. For a cell-based vaccine or immunotherapy composition, the polypeptide may be processed and/or presented by cells, for example autologous dendritic cells or antigen presenting cells, pulsed with the polypeptide or comprising an expression construct encoding the polypeptide.

The immunogenic or pharmaceutical compositions described herein may comprise, in addition to one or more immunogenic peptides, a pharmaceutically acceptable excipient, carrier, diluent, buffer, stabiliser, preservative, adjuvant or other materials well known to those skilled in the art. Such materials are preferably non-toxic and preferably do not interfere with the pharmaceutical activity of the active ingredient(s). The pharmaceutical carrier or diluent may be, for example, water containing solutions. The precise nature of the carrier or other material may depend on the route of administration, e.g. oral, intravenous, cutaneous or subcutaneous, nasal, intramuscular, intradermal, and intraperitoneal routes.

The pharmaceutical compositions of the invention may comprise one or more "pharmaceutically acceptable carriers". These are typically large, slowly metabolized macromolecules such as proteins, saccharides, polylactic acids, polyglycolic acids, polymeric amino acids, amino acid copolymers, sucrose (Paoletti et al., 2001, Vaccine, 19:2118), trehalose (WO 00/56365), lactose and lipid aggregates (such as oil droplets or liposomes). Such carriers are well known to those of ordinary skill in the art. The pharmaceutical compositions may also contain diluents, such as water, saline, glycerol, etc. Additionally, auxiliary substances, such as wetting or emulsifying agents, pH buffering substances, and the like, may be present. Sterile pyrogen-free, phosphate buffered physiologic saline is a typical carrier (Gennaro, 2000, Remington: The Science and Practice of Pharmacy, 20th edition, ISBN:0683306472).

The pharmaceutical compositions of the disclosure may be lyophilized or in aqueous form, i.e. solutions or suspensions. Liquid formulations of this type allow the compositions to be administered direct from their packaged form, without the need for reconstitution in an aqueous medium, and are thus ideal for injection. The pharmaceutical compositions may be presented in vials, or they may be presented in ready filled syringes. The syringes may be supplied with or without needles. A syringe will include a single dose, whereas a vial may include a single dose or multiple doses.

Liquid formulations of the disclosure are also suitable for reconstituting other medicaments from a lyophilized form. Where a pharmaceutical composition is to be used for such extemporaneous reconstitution, the disclosure provides a kit, which may comprise two vials, or may comprise one ready-filled syringe and one vial, with the contents of the syringe being used to reconstitute the contents of the vial prior to injection.

The pharmaceutical compositions of the disclosure may include an antimicrobial, particularly when packaged in a multiple dose format. Antimicrobials may be used, such as 2-phenoxyethanol or parabens (methyl, ethyl, propyl parabens). Any preservative is preferably present at low levels. Preservative may be added exogenously and/or may be a component of the bulk antigens which are mixed to form the composition (e.g. present as a preservative in pertussis antigens).

The pharmaceutical compositions of the disclosure may comprise detergent e.g. Tween (polysorbate), DMSO (dimethyl sulfoxide), DMF (dimethylformamide). Detergents are generally present at low levels, e.g. <0.01%, but may also be used at higher levels, e.g. 0.01 - 50%.

The pharmaceutical compositions of the disclosure may include sodium salts (e.g. sodium chloride) and free phosphate ions in solution (e.g. by the use of a phosphate buffer).

In certain embodiments, the pharmaceutical composition may be encapsulated in a suitable vehicle either to deliver the peptides into antigen presenting cells or to increase the stability. As will be appreciated by a skilled artisan, a variety of vehicles are suitable for delivering a pharmaceutical composition of the invention. Non-limiting examples of suitable structured fluid delivery systems may include nanoparticles, liposomes, microemulsions, micelles, dendrimers and other phospholipid-containing systems. Methods of incorporating pharmaceutical compositions into delivery vehicles are known in the art.

In order to increase the immunogenicity of the composition, the pharmacological compositions may comprise one or more adjuvants and/or cytokines.

Suitable adjuvants include an aluminum salt such as aluminum hydroxide or aluminum phosphate, but may also be a salt of calcium, iron or zinc, or may be an insoluble suspension of acylated tyrosine, or acylated sugars, or may be cationically or anionically derivatised saccharides, polyphosphazenes, biodegradable microspheres, monophosphoryl lipid A (MPL), lipid A derivatives (e.g. of reduced toxicity), 3-O-deacylated MPL [3D-MPL], quil A, Saponin, QS21, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators suitable for use as adjuvants in the disclosure include cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte, macrophage colony stimulating factor (GM-CSF) may also be used as adjuvants.

In some embodiments, the compositions comprise an adjuvant selected from the group consisting of Montanide ISA-51 (Seppic, Inc., Fairfield, N.J., United States of America), QS-21 (Aquila Biopharmaceuticals, Inc., Lexington, Mass., United States of America), tetanus helper peptides, GM-CSF, cyclophosamide, bacillus Calmette-Guerin (BCG), corynbacterium parvum, levamisole, azimezone, isoprinisone, dinitrochlorobenezene (DNCB), keyhole limpet hemocyanins (KLH), Freunds adjuvant (complete and incomplete), mineral gels, aluminum hydroxide (Alum), lysolecithin, pluronic polyols, polyanions, peptides, oil emulsions, dinitrophenol, diphtheria toxin (DT).

By way of example, the cytokine may be selected from the group consisting of a transforming growth factor (TGF) such as but not limited to TGF-α and TGF-β; insulin-like growth factor-I and/or insulin-like growth factor-II; erythropoietin (EPO); an osteoinductive factor; an interferon such as but not limited to interferon-.α, -β, and -γ; a colony stimulating factor (CSF) such as but not limited to macrophage-CSF (M-CSF); granulocyte-macrophage-CSF (GM-CSF); and granulocyte-CSF (G-CSF). In some embodiments, the cytokine is selected from the group consisting of nerve growth factors such as NGF-β; platelet-growth factor; a transforming growth factor (TGF) such as but not limited to TGF-α. and TGF-β; insulin-like growth factor-I and insulin-like growth factor-II; erythropoietin (EPO); an osteoinductive factor; an interferon (IFN) such as but not limited to IFN-α, IFN-β, and IFN-γ; a colony stimulating factor (CSF) such as macrophage-CSF (M-CSF); granulocyte-macrophage-CSF (GM-CSF); and granulocyte-CSF (G-CSF); an interleukin (Il) such as but not limited to IL-1, IL-1.alpha., IL-2, IL-3, IL-4, IL-5, IL-6, IL-7, IL-8, IL-9, IL-10, IL-11, IL-12; IL-13, IL-14, IL-15, IL-16, IL-17, IL-18; LIF; kit-ligand or FLT-3; angiostatin; thrombospondin; endostatin; a tumor necrosis factor (TNF); and LT.

It is expected that an adjuvant or cytokine can be added in an amount of about 0.01 mg to about 10 mg per dose, preferably in an amount of about 0.2 mg to about 5 mg per dose. Alternatively, the adjuvant or cytokine may be at a concentration of about 0.01 to 50%, preferably at a concentration of about 2% to 30%.

In certain aspects, the pharmaceutical compositions of the disclosure are prepared by physically mixing the adjuvant and/or cytokine with the peptides of the invention under appropriate sterile conditions in accordance with known techniques to produce the final product.

Examples of suitable compositions of the invented polypeptide fragments and methods of administration are provided in Esseku and Adeyeye (2011) and Van den Mooter G. (2006). Vaccine and immunotherapy composition preparation is generally described in Vaccine Design ("The subunit and adjuvant approach" (eds Powell M. F. & Newman M. J. (1995) Plenum Press New York). Encapsulation within liposomes, which is also envisaged, is described by Fullerton, US Patent 4,235,877.

Polynucleotide or oligonucleotide components may be naked nucleotide sequences or be in combination with cationic lipids, polymers or targeting systems. They may be delivered by any available technique. For example, the polynucleotide or oligonucleotide may be introduced by needle injection, preferably intradermally, subcutaneously or intramuscularly. Alternatively, the polynucleotide or oligonucleotide may be delivered directly across the skin using a delivery device such as particle-mediated gene delivery. The polynucleotide or oligonucleotide may be administered topically to the skin, or to mucosal surfaces for example by intranasal, oral, or intrarectal administration.

Uptake of polynucleotide or oligonucleotide constructs may be enhanced by several known transfection techniques, for example those including the use of transfection agents. Examples of these agents include cationic agents, for example, calcium phosphate and DEAE-Dextran and lipofectants, for example, lipofectam and transfectam. The dosage of the polynucleotide or oligonucleotide to be administered can be altered.

Administration is typically in a "prophylactically effective amount" or a "therapeutically effective amount" (as the case may be, although prophylaxis may be considered therapy), this being sufficient to result in a clinical response or to show clinical benefit to the individual, e.g. an effective amount to prevent or delay onset of the disease or condition, to ameliorate one or more symptoms, to induce or prolong remission, or to delay relapse or recurrence.

The dose may be determined according to various parameters, especially according to the substance used; the age, weight and condition of the individual to be treated; the route of administration; and the required regimen. The amount of antigen in each dose is selected as an amount which induces an immune response. A physician will be able to determine the required route of administration and dosage for any particular individual. The dose may be provided as a single dose or may be provided as multiple doses, for example taken at regular intervals, for example 2, 3 or 4 doses administered hourly. Typically peptides, polynucleotides or oligonucleotides are typically administered in the range of 1 pg to 1 mg, more typically 1 pg to 10 µg for particle mediated delivery and 1 µg to 1 mg, more typically 1-100 µg, more typically 5-50 µg for other routes. Generally, it is expected that each dose will comprise 0.01-3 mg of antigen. An optimal amount for a particular vaccine can be ascertained by studies involving observation of immune responses in subjects.

Examples of the techniques and protocols mentioned above can be found in Remington's Pharmaceutical Sciences, 20th Edition, 2000, pub. Lippincott, Williams & Wilkins.

In some cases in accordance with the invention, more than one peptide or composition of peptides is administered. Two or more pharmaceutical compositions may be administered together/simultaneously and/or at different times or sequentially. Thus, the invention includes sets of pharmaceutical compositions and uses thereof. The use of combination of different peptides, optionally targeting different antigens, is important to overcome the challenges of genetic heterogeneity of tumors and HLA heterogeneity of individuals. The use of peptides of the invention in combination expands the group of individuals who can experience clinical benefit from vaccination. Multiple pharmaceutical compositions of peptides of the invention, manufactured for use in one regimen, may define a drug product.

Routes of administration include but are not limited to intranasal, oral, subcutaneous, intradermal, and intramuscular. The subcutaneous administration is particularly preferred. Subcutaneous administration may for example be by injection into the abdomen, lateral and anterior aspects of upper arm or thigh, scapular area of back, or upper ventrodorsal gluteal area.

The skilled artisan will recognize that compositions of the invention may also be administered in one, or more doses, as well as, by other routes of administration. For example, such other routes include, intracutaneously, intravenously, intravascularly, intraarterially, intraperitnoeally, intrathecally, intratracheally, intracardially, intralobally, intramedullarly, intrapulmonarily, and intravaginally. Depending on the desired duration of the treatment, the compositions according to the invention may be administered once or several times, also intermittently, for instance on a monthly basis for several months or years and in different dosages.

Solid dosage forms for oral administration include capsules, tablets, caplets, pills, powders, pellets, and granules. In such solid dosage forms, the active ingredient is ordinarily combined with one or more pharmaceutically acceptable excipients, examples of which are detailed above. Oral preparations may also be administered as aqueous suspensions, elixirs, or syrups. For these, the active ingredient may be combined with various sweetening or flavoring agents, coloring agents, and, if so desired, emulsifying and/or suspending agents, as well as diluents such as water, ethanol, glycerin, and combinations thereof.

One or more compositions of the invention may be administered alone or in combination with other pharmacological compositions or treatments, for example chemotherapy and/or immunotherapy and/or vaccine. The other therapeutic compositions or treatments may for example be one or more of those discussed herein, and may be administered either simultaneously or sequentially with the composition or treatment of the invention.

As used herein, "immunotherapy" is the prevention or treatment of a disease or condition by inducing or enhancing an immune response in an individual. In certain embodiments, immunotherapy refers to a therapy that comprises the administration of one or more drugs to an individual to elicit T cell responses. In a specific embodiment, immunotherapy refers to a therapy that comprises the administration or expression of polypeptides that contain one or more PEPIs to an individual to elicit a T cell response to recognize and kill cells that display the one or more PEPIs on their cell surface in conjunction with a class I HLAs. In another specific embodiment, immunotherapy comprises the administration of one or more PEPIs to an individual to elicit a cytotoxic T cell response against cells that display tumor associated antigens (TAAs) or cancer testis antigens (CTAs) comprising the one or more PEPIs on their cell surface. In another embodiment, immunotherapy refers to a therapy that comprises the administration or expression of polypeptides that contain one or more PEPIs presented by class II HLAs to an individual to elicit a T helper response to provide co-stimulation to cytotoxic T cells that recognize and kill diseased cells that display the one or more PEPIs on their cell surface in conjunction with a class I HLAs. In still another specific embodiment, immunotherapy refers to a therapy that comprises administration of one or more drugs to an individual that re-activate existing T cells to kill target cells. The theory is that the cytotoxic T cell response will eliminate the cells displaying the one or more PEPIs, thereby improving the clinical condition of the individual. In some instances, immunotherapy may be used to treat tumors. In other instances, immunotherapy may be used to treat intracellular pathogen-based diseases or disorders.

### Selection of peptides and patients

Specific polypeptide antigens induce immune responses in only a fraction of human subjects. The peptides of the present invention are specifically selected to induce immune responses in a high proportion of the population, but they will not be effective in all individuals due to HLA genotype heterogeneity. Accordingly some aspects of the invention relate to a method of identifying a human subject who will likely have a cytotoxic T cell response to administration of a pharmaceutical composition comprising a peptide of the invention (likely responders), or of predicting the likelihood that a subject will have a cytotoxic T cell response.

The inventors have discovered that T cell epitope presentation by multiple HLAs of an individual is generally needed to trigger a T cell response. The best predictor of a cytotoxic T cell response to a given polypeptide, as determined by the inventors, is the presence of at least one T cell epitope that is presented by three or more HLA class I of an individual (≥1 PEPI3+). Accordingly the presence within the active ingredient peptides of a pharmaceutical composition of one or more T cell epitopes that is capable of binding to at least three HLA of a subject is predictive for the subject having a cytotoxic T cell response to administration of the pharmaceutical composition. The subject is a likely immune responder.

In some cases the T cell epitope that is capable of binding to at least three HLA class I of the subject has the amino acid sequence of any one of SEQ ID NOs: 1 to 40. In other cases the T cell epitope may have a different amino acid sequence within the one or more peptides of the pharmaceutical composition.

The inventors have further discovered that the presence in a vaccine or immunotherapy composition of at least two epitopes that can bind to at least three HLA of an individual is predictive for a clinical response. In other words, if an individual has a total of ≥2 PEPI3+ within the active ingredient polypeptide(s) of a vaccine or immunotherapy composition, and these PEPI3+s are derived from antigen sequences that are in fact expressed in the individual (for example, target tumor cells of the individual express the target tumor-associated antigens), then the individual is a likely clinical responder (i.e. a clinically relevant immune responder).

Accordingly some aspects of the invention relate to a method of identifying a subject who will likely have a clinical response to a method of treatment according to the invention, or of predicting the likelihood that a subject will have a clinical response.

A "clinical response" or "clinical benefit" as used herein may be the prevention or a delay in the onset of a disease or condition, the amelioration of one or more symptoms, the induction or prolonging of remission, or the delay of a relapse or recurrence or deterioration, or any other improvement or stabilisation in the disease status of a subject. Where appropriate, a "clinical response" may correlate to "disease control" or an "objective response" as defined by the Response Evaluation Criteria In Solid Tumors (RECIST) guidelines.

In some embodiments the method comprises determining that one or more cancer-associated antigens selected from SPAG9, AKAP-4, BORIS, NY-SAR-35, NY-BR-1, SURVIVIN, MAGE-A11, PRAME, MAGE-A9, HOM-TES-85, TSP50, EpCAM, CAGE1, FBXO39, MAGE-A8 and MAGE-A6 is expressed by a cancer type. For example expression of the cancer associated antigen may be detected in a sample obtained from the subject, for example a tumor biopsy, using methods that are well known in the art.

The inventors have discovered that it is not sufficient that a vaccine or immunotherapy composition targets an antigen that is expressed by cancer or tumor cells of a patient, nor that the target sequences of that antigen can bind to HLA class I of the patient (HLA restricted epitopes). The composition is likely effective only in patients that both express the target antigen and have three or more HLA class I that bind to a single T cell epitope of the target antigen. Moreover, as described above, at least two epitopes that binds to at least 3 HLAs of the patient are generally needed to induce a clinically relevant immune response.

Therefore the method further comprises determining that the active ingredient peptide(s) of the pharmaceutical composition comprise two or more different amino acid sequences each of which is a) a fragment of a cancer-associated antigen expressed by cancer cells of the subject, determined as described above; and b) a T cell epitope capable of binding to at least three HLA class I of the subject.

In some cases the T cell epitope that is capable of binding to at least three HLA class I of the subject has the amino acid sequence of any one of SEQ ID NOs: 1 to 40. In other cases the T cell epitope may have a different amino acid sequence within the one or more peptides of the pharmaceutical composition.

In some cases the likelihood that a subject will have a clinical response to a peptide vaccine or immunotherapy composition, such as those described herein, can be determined without knowing whether the target antigens are expressed in cancer or tumor cells of the subject and/or without determining the HLA class I genotype of the subject. Known antigen expression frequencies in the disease (e.g. MAGE-A3 in a tumor type like breast or colorectal cancer) and/or known frequencies for HLA class I and class II genotype of subjects in the target population (e.g ethnic population, general population, diseased population) may be used instead. Moreover by combining peptides that target the most frequently presented PEPIs across the population (BestEPIs) in multiple frequently expressed target antigens in the disease, as identified and described herein, it is possible to design a cancer vaccine regime that is effective for a high proportion of patients. However, using the companion diagnostic methods described herein to pre-select patients who are most likely to have a clinical response will increase clinical response rates amongst treated patients.

The results of a prediction as set out above may be used to inform a physician's decisions concerning treatment of the subject. Accordingly, in some cases the method further comprises selecting the pharmaceutical composition for use in a method of treatment of the subject. The method may further comprise administering the pharmaceutical composition to the subject.

Also provided herein is method of identifying a human subject who will likely not have a clinical response to a method of treatment described herein. The method may further comprise selecting a different treatment for the specific human subject.

### Examples

### Example 1 - HLA-epitope binding prediction process and validation

Predicted binding between particular HLA and epitopes (9mer peptides) was based on the Immune Epitope Database tool for epitope prediction (www.iedb.org).

Our HLA I-epitope binding prediction process was validated by comparison with well-characterised HLA I-epitope pairs determined by laboratory experiments. A dataset was compiled of HLA I-epitope pairs reported in peer reviewed publications or public immunological databases.

We determined the rate of agreement with the experimentally determined dataset (Table 2). The binding HLA I-epitope pairs of the dataset were correctly predicted with a 93% probability. Coincidentally the non-binding HLA I-epitope pairs were also correctly predicted with a 93% probability.

**Table 2. Analytical specificity and sensitivity of the HLA-epitope binding prediction process.**

| ***HLA*-*epitope pairs*** | ***True epitopes (n=327)** (Binder match)* | ***False epitopes (n=100)** (Non-binder match)* |
|---|---|---|
| ***HIV*** | 91 % (32) | 82% (14) |
| ***Viral*** | 100% (35) | 100% (11) |
| ***Tumor*** | 90% (172) | 94% (32) |
| ***Other (fungi*, *bacteria*, *etc*.*)*** | 100% (65) | 95% (36) |
| ***All*** | ***93% (304)*** | ***93*% *(93)*** |

Next we determined the accuracy of the prediction of multiple HLA binding epitopes (Table 3). Based on the analytical specificity and sensitivity using the 93% probability for both true positive and true negative prediction and 7% (=100% - 93%) probability for false positive and false negative prediction, the probability of the existence of a multiple HLA binding epitope in a person can be calculated. The probability of multiple HLA binding to an epitope (7) shows the relationship between the number of HLAs binding an epitope and the expected minimum number of real binding. Per PEPI definition three is the expected minimum number of HLA to bind an epitope (in **bold** in Table 3).

**Table 3. Accuracy of multiple HLA binding epitopes predictions.**

| **Expected minimum number of real HLA binding** | **Predicted number of HLAs binding to an epitope** | | | | | | |
|---|---|---|---|---|---|---|---|
| | **0** | **1** | **2** | **3** | **4** | **5** | **6** |
| **1** | 35% | 95% | 100% | 100% | 100% | 100% | 100% |
| **2** | 6% | 29% | 90% | 99% | 100% | 100% | 100% |
| **3** | **1%** | **4%** | **22%** | **84%** | **98%** | **100%** | **100%** |
| **4** | 0% | 0% | 2% | 16% | 78% | 96% | 99% |
| **5** | 0% | 0% | 0% | 1% | 10% | 71% | 94% |
| **6** | 0% | 0% | 0% | 0% | 0% | 5% | 65% |

The validated HLA-epitope binding prediction process was used to determine all HLA-epitope binding pairs described in the Examples below.

### Example 2 - Epitope presentation by multiple HLA predicts cytotoxic T lymphocyte (CTL) response

This study investigates whether the presentation of one or more epitopes of a polypeptide antigen by one or more HLA I of an individual is predictive for a CTL response.

The study was carried out by retrospective analysis of six clinical trials, conducted on 71 cancer and 9 HIV-infected patients (Table 4)¹⁻⁷. Patients from these studies were treated with an HPV vaccine, three different NY-ESO-1 specific cancer vaccines, one HIV-1 vaccines and a CTLA-4 specific monoclonal antibody (Ipilimumab) that was shown to reactivate CTLs against NY-ESO-1 antigen in melanoma patients. All of these clinical trials measured antigen specific CD8+ CTL responses (immunogenicity) in the study subjects after vaccination. In some cases, correlation between CTL responses and clinical responses were reported.

No patient was excluded from the retroactive study for any reason other than data availability. The 157 patient datasets (Table 4) were randomized with a standard random number generator to create two independent cohorts for training and evaluation studies. In some cases the cohorts contained multiple datasets from the same patient, resulting in a training cohort of 76 datasets from 48 patients and a test/validation cohort of 81 datasets from 51 patients.

**Table 4. Summary of patient datasets**

| **Clinical trial** | **Immunotherapy** | **Target Antigen** | **Disease** | # **Patients*** | **# Data sets** (#antigen x #patient) | **Immunoassay performed in the clinical trials**** | **HLA genotyping method** | **Ref** |
|---|---|---|---|---|---|---|---|---|
| 1 | VGX-3100 | HPV16-E6 HPV16-E7 HPV18-E6 HPV18-E7 HPV16/18 | Cervical cancer | 17/18 | 5 x 17 | IFN-γ ELISPOT | High Resolution SBT | 1 |
| 2 | HIVIS vaccine | HIV-1 Gag HIV-1 RT | AIDS | 9/12 | 2 x 9 | IFN-γ ELISPOT | Low-Medium Resolution SSO | 2 |
| | | | Breast-and | | | | | |
| | | | ovarian | | | In vitro and Ex vivo IFN-γ ELISPOT | High Resolution SBT | |
| 3 | rNY-ESO-1 | NY-ESO-1 | cancers, | 18/18 | 1x18 | | | 3 |
| | | | melanoma | | | | | 4 |
| | | | and | | | | | |
| | | | sarcoma | | | | | |
| 4 | Ipilimumab | NY-ESO-1 | Metastatic melanoma | 19/20 | 1 x 19 | ICS after T-cell stimulation | Low to medium resolution typing, SSP typing, of genomic DNA, high resolution sequencing | 5 |
| 5 | NY-ESO-1f | NY-ESO-1 (91-110) | Esophageal-, non-small-cell lung-and gastric cancer | 10/10 | 1 x 10 | ICS after T-cell stimulation | probing SSO probing and SSP of genomic DNA | 6 |
| 6 | NY-ESO-1 overlapping peptides | NY-ESO-1 (79-173) | Esophageal-and lung cancer, malignant melanoma | 7/9 | 1 x 7 | ICS after T-cell stimulation | SSO probing and SSP of genomic DNA | 7 |
| **Total** | **6** | **7** | | **80** | **157** | **N/A** | | |
| | *Number of patients used in the retrospective analysis from the original number of patient of the clinical trials. | | | | | | | |
| | **Immunoassays are based on T cell stimulation with antigen-specific peptide pools and quantify the released cytokines by different techniques. | | | | | | | |
| | CT: Clinical trial; SBT: Sequence Based Typing; SSO: Sequence-Specific Oligonucleotide; ICS: Intracellular cytokine staining; SSP: Sequence-specific priming | | | | | | | |

We compared the reported CTL responses of the training dataset with the HLA I restriction profile of epitopes (9mers) of the vaccine antigens. The antigen sequences and the HLA I genotype of each patient were obtained from publicly available protein sequence databases or peer reviewed publications and the HLA I-epitope binding prediction process was blinded to patients' clinical CTL response data. The number of epitopes from each antigen predicted to bind to at least 1 (PEPI1+), or at least 2 (PEPI2+), or at least 3 (PEPI3+), or at least 4 (PEPI4+), or at least 5 (PEPI5+), or all 6 (PEPI6) HLA class I molecules of each patient was determined and the number of HLA bound were used as classifiers for the reported CTL responses. The true positive rate (sensitivity) and true negative rate (specificity) were determined from the training dataset for each classifier (number of HLA bound) separately.

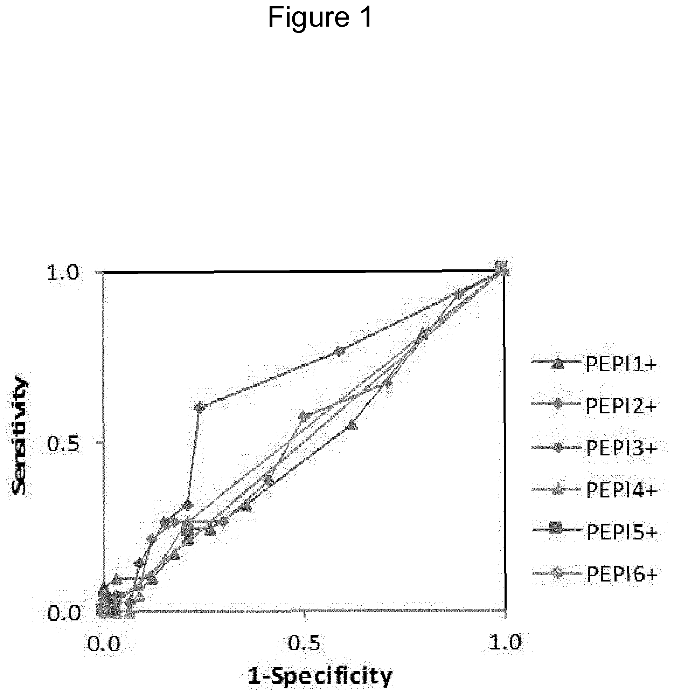

ROC analysis was performed for each classifier. In a ROC curve, the true positive rate (Sensitivity) is plotted in function of the false positive rate (1-Specificity) for different cut-off points (FIG. 1). Each point on the ROC curve represents a sensitivity/specificity pair corresponding to a particular decision threshold (epitope (PEPI) count). The area under the ROC curve (AUC) is a measure of how well the classifier can distinguish between two diagnostic groups (CTL responder or non-responder).

The analysis unexpectedly revealed that predicted epitope presentation by multiple class I HLAs of a subject (PEPI2+, PEPI3+, PEPI4+, PEPI5+, or PEPI6), was in every case a better predictor of CTL response than epitope presentation by merely one or more HLA class I (PEPI1+, AUC = 0.48, Table 5).

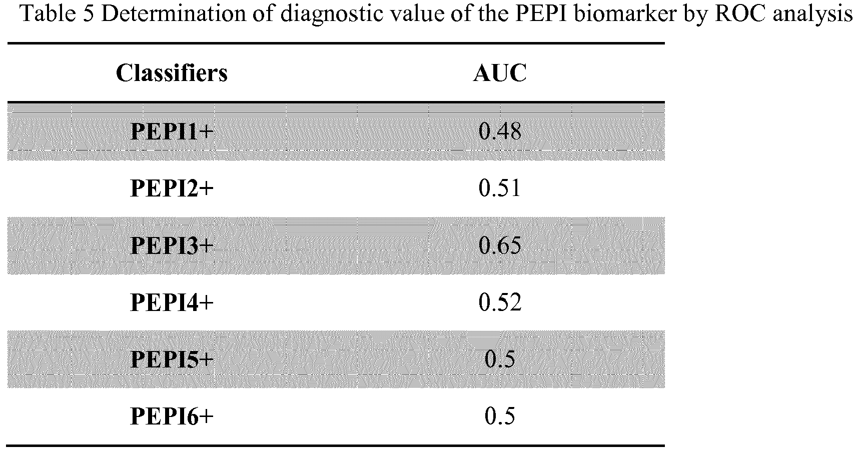

The CTL response of an individual was best predicted by considering the epitopes of an antigen that could be presented by at least 3 HLA class I of an individual (PEPI3+, AUC = 0.65, Table 5). The threshold count of PEPI3+ (number of antigen-specific epitopes presented by 3 or more HLA of an individual) that best predicted a positive CTL response was 1 (Table 6). In other words, at least one antigen-derived epitope is presented by at least 3 HLA class I of a subject (≥1 PEPI3+), then the antigen can trigger at least one CTL clone, and the subject is a likely CTL responder. Using the ≥1 PEPI3+ threshold to predict likely CTL responders ("≧1 PEPI3+ Test") provided 76% diagnostic sensitivity (Table 6).

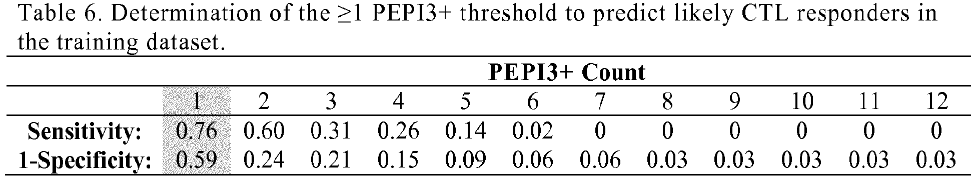

### Example 3 - Validation of the ≧1 PEPI3+ Test

The test cohort of 81 datasets from 51 patients was used to validate the ≥1 PEPI3+ threshold to predict an antigen-specific CTL response. For each dataset in the test cohort it was determined whether the ≥1 PEPI3+ threshold was met (at least one antigen-derived epitope presented by at least three class I HLA of the individual). This was compared with the experimentally determined CTL responses reported from the clinical trials (Table 7).

**Table 7. Diagnostic performance characteristics of the ≥1 PEPI3+ Test (n=81).**

| **Performance characteristic** | | **Description** | **Result** |
|---|---|---|---|
| **Positive predictive value (PPV)** | 100%[A/(A+B)] | The likelihood that an individual that meets the >1 PEPI3+ threshold has antigen-specific CTL responses after treatment with immunotherapy. | ***84%*** |
| **Sensitivity** | 100%[A/(A+C)] | The proportion of subjects with antigen-specific CTL responses after treatment with immunotherapy who meet the ≥1 PEPI3+ threshold. | ***75%*** |
| **Specificity** | 100%[D/(B+D)] | The proportion of subjects without antigen-specific CTL responses after treatment with immunotherapy who do not meet the ≥1 PEPI3+ threshold. | ***55%*** |
| **Negative predictive value (NPV)** | 100%[D/(C +D)] | The likelihood that an individual who does not meet the ≥1 PEPI3+ threshold does not have antigen-specific CTL responses after treatment with immunotherapy. | ***42%*** |
| **Overall percent agreement** (**OPA**) | 100%[(A + D)/N] | The percentage of predictions based on the >1 PEPI3+ threshold that match the experimentally determined result, whether positive or negative. | ***70%*** |
| **Fisher's exact (p)** | | | ***0.01*** |

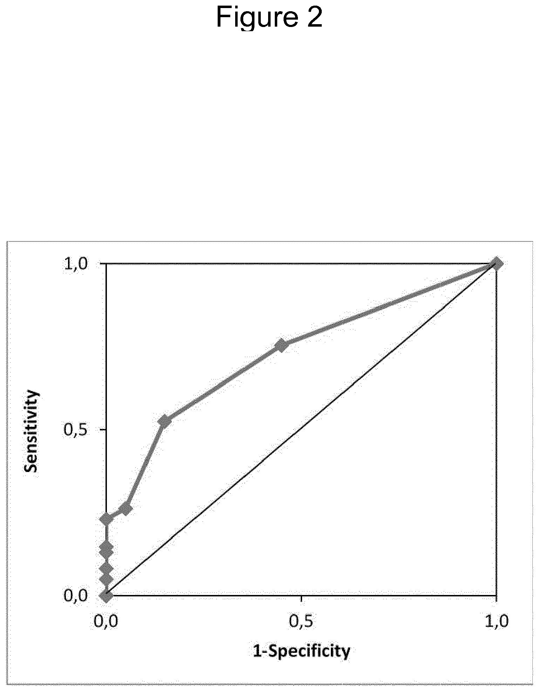

ROC analysis determined the diagnostic accuracy, using the PEPI3+ count as cut-off values (Fig. 2). The AUC value = 0.73. For ROC analysis an AUC of 0.7 to 0.8 is generally considered as fair diagnostic.

A PEPI3+ count of at least 1 (≥1 PEPI3+) best predicted a CTL response in the test dataset (Table 8). This result confirmed the threshold determined during the training (Table 5).

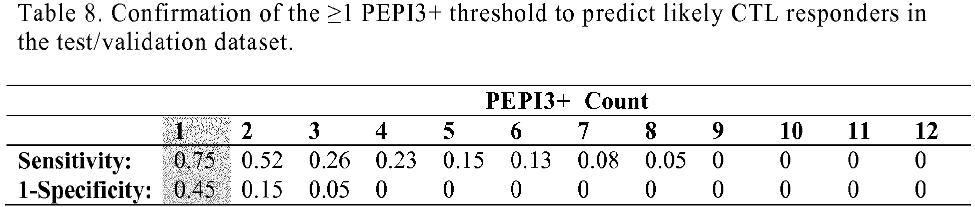

### Example 4 - The ≥1 PEPI3+ Test predicts CD8+ CTL reactivities

This study compares the ≥1 PEPI3+ Test with the prior art method for predicting a specific human subject's CTL response to peptide antigens.

We determined from DNA specimens the HLA genotypes of 28 cervical cancer and VIN-3 patients that received the HPV-16 synthetic long peptide vaccine (LPV) in two different clinical trials ^{8 9 10}. The LPV consists of long peptides covering the HPV-16 viral oncoproteins E6 and E7. The amino acid sequence of the LPV was obtained from these publications. The publications also report the T cell responses of each vaccinated patient to pools of overlapping peptides of the vaccine.

We identified for each patient epitopes (9mers) of the LPV that are presented by at least three patient class I HLA (PEPI3+s) and determined their distribution among the peptide pools. Peptides that comprised at least one PEPI3+ (≥1 PEPI3+) were predicted to induce a CTL response. Peptides that comprised no PEPI3+ were predicted not to induce a CTL response.

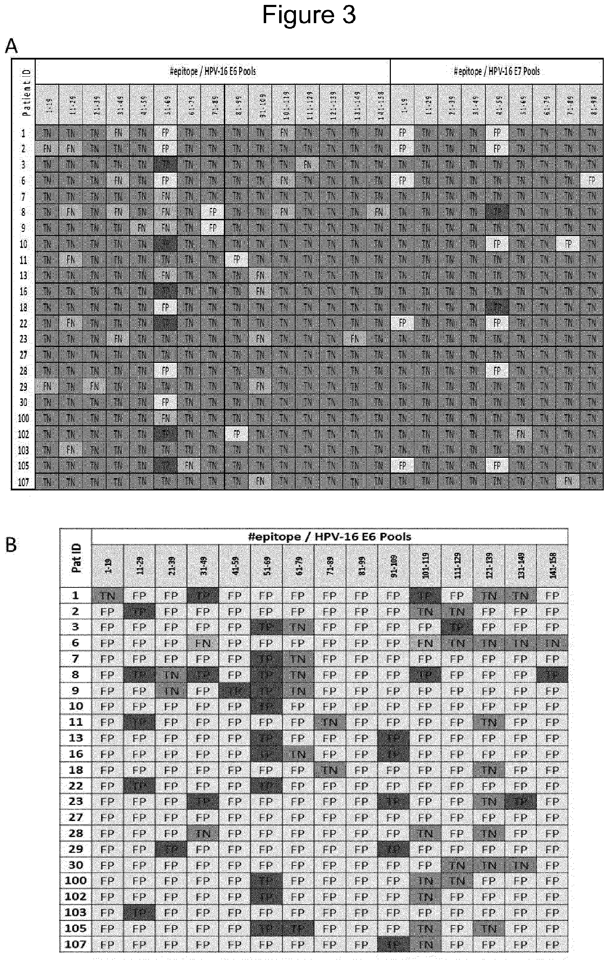

The ≥1 PEPI3+ Test correctly predicted 489 out of 512 negative CTL responses and 8 out of 40 positive CTL responses measured after vaccination (Fig. 3A). Overall, the agreement between the ≥1 PEPI3+ Test and experimentally determined CD8+ T cell reactivity was 90% (p<0.001).

We also determined for each patient the distribution among the peptide pools of epitopes that are presented by at least one patient class I HLA (≥1 PEPI1+, HLA restricted epitope prediction, prior art method). ≥1 PEPI1+ correctly predicted 116 out of 512 negative CTL responses and 37 out of 40 positive CTL responses measured after vaccination (FIG. 3B). Overall, the agreement between the HLA restricted epitope prediction (≥1 PEPI1+) and CD8+ T cell reactivity was 28% (not significant).

### Example 5 - Prediction of HLA classII restricted CD4+ helper T cell epitopes

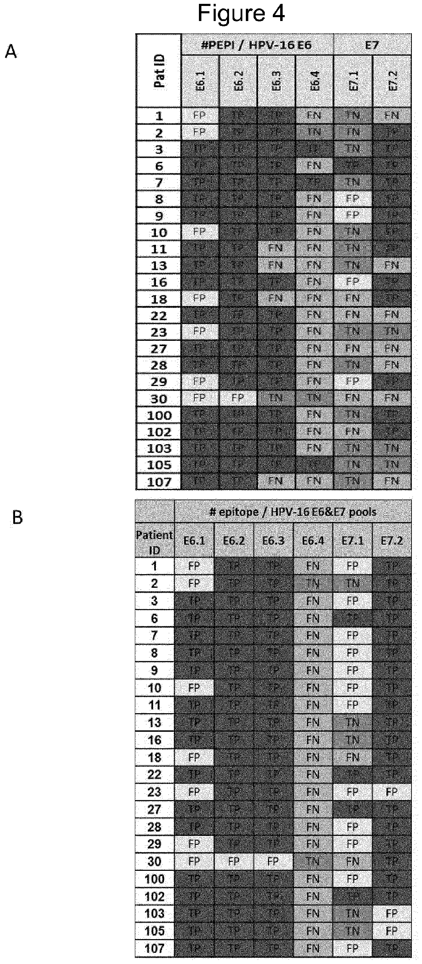

The 28 cervical cancer and VIN-3 patients that received the HPV-16 synthetic long peptide vaccine (LPV) in two different clinical trials (as detailed in Example 4) were investigated for CD4+ T helper responses following LPV vaccination (FIG. 4). The sensitivity of the prediction of HLA class II restricted epitopes was 78%, since the State of Art tool predicted 84 positive responses (positive CD4+ T cell reactivity to a peptide pool for a person's DP alleles) out of 107 (sensitivity = 78%). The specificity was 22% since it could rule out 7 negative responses out of 31. Overall, the agreement between HLA-restricted class II epitope prediction and CD4+ T cell reactivity was 66%, which was statistically not significant.

### Example 6 - The ≥1 PEPI3+ Test predicts T cell responses to full length LPV polypeptides

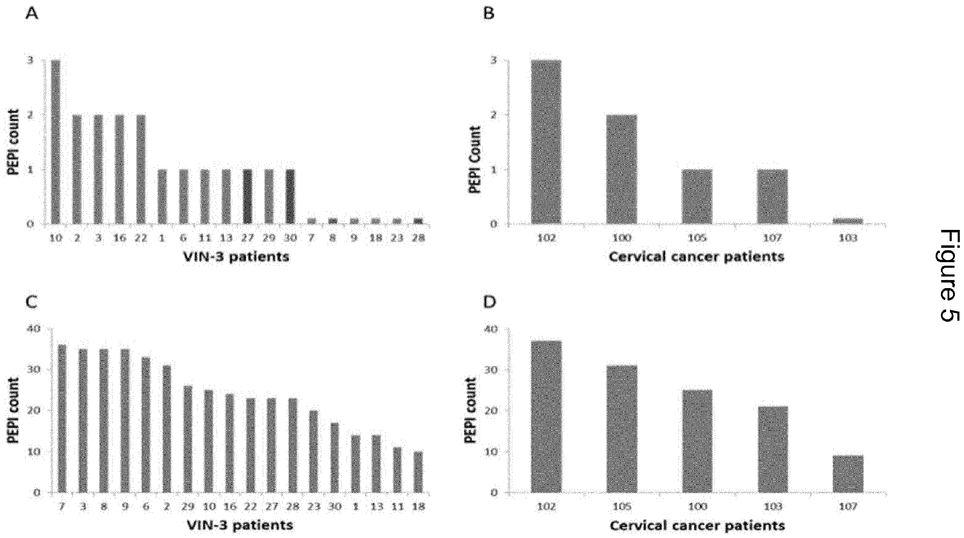

Using the same reported studies as Examples 4 and 5, we used the ≥1 PEPI3+ Test to predict patient CD8+ and CD4+ T cell responses to the full length E6 and E7 polypeptide antigens of the LPV vaccine and compared our predictions to the experimentally determined responses where reported. The Test correctly predicted the CD8+ T cell reactivity (PEPI3+) of 11 out of 15 VIN-3 patients with positive CD8+ T cell reactivity test results (sensitivity 73%, PPV 85%) and of 2 out of 5 cervical cancer patients (sensitivity 40%, PPV 100%). The CD4+ T cell reactivities (PEPI4+) were correctly predicted 100% both of VIN-3 and cervical cancer patients (Figure 5).

We also observed that the class I and class II HLA restricted PEPI3+ count correlated with the reported clinical benefit to LPV vaccinated patients. Patients with higher PEPI3+ counts had either complete or partial response already after 3 months. However the study was too small to establish the responder threshold.

### Example 7 - Case Study

pGX3001 is an HPV16 based DNA vaccine containing full length E6 and E7 antigens with a linker in between. pGX3002 is an HPV18 based DNA vaccine containing full length E6 and E7 antigens with a linker in between. A Phase II clinical trial investigated the T cell responses of 17 HPV-infected patients with cervical cancer who were vaccinated with both pGX3001 and pGX3002 (VGX-3100 vaccination)¹.

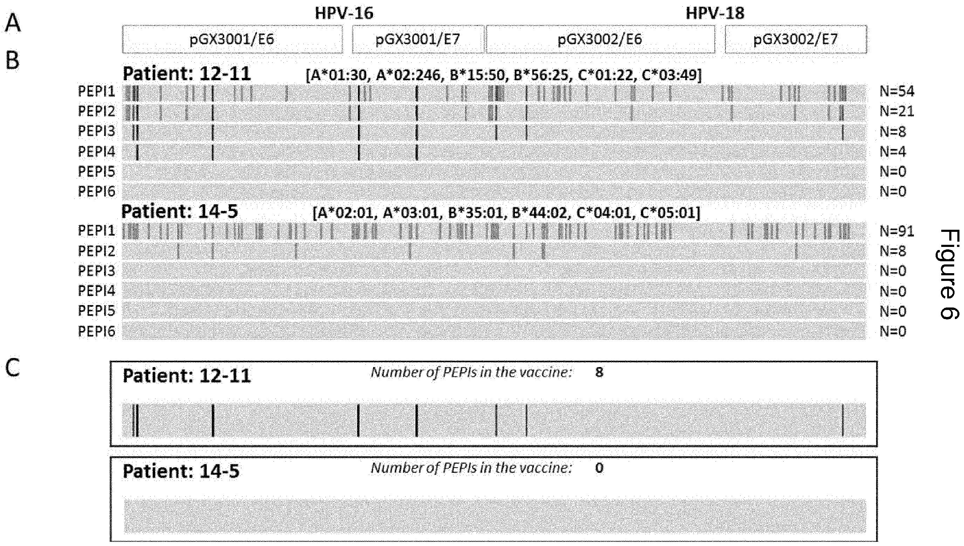

Fig. 5-6 shows for two illustrative patients (patient 12-11 and patient 14-5) the position of each epitope (9mer) presented by at least 1 (PEPI1+), at least 2 (PEPI2+), at least 3 (PEPI3+), at least 4 (PEPI4+), at least 5 (PEPI5+), or all 6 (PEPI6) class I HLA of these patients within the full length sequence of the two HPV-16 and two HPV-18 antigens.

Patient 12-11 had an overall PEPI1+ count of 54 for the combined vaccines (54 epitopes presented by one or more class I HLA). Patient 14-5 had a PEPI1+ count of 91. Therefore patient 14-5 has a higher PEPI1+ count than patient 12-11 with respect to the four HPV antigens. The PEPI1+s represent the distinct vaccine antigen specific HLA restricted epitope sets of patients 12-11 and 14-5. Only 27 PEPI1+s were common between these two patients.

For the PEPI3+ counts (number of epitopes presented by three or more patient class I HLA), the results for patients 12-11 and 14-5 were reversed. Patient 12-11 had a PEPI3+ count of 8, including at least one PEPI3+ in each of the four HPV16/18 antigens. Patient 14-5 had a PEPI3+ count of 0.

The reported immune responses of these two patients matched the PEPI3+ counts, not the PEPI1+ counts. Patient 12-11 developed immune responses to each of the four antigens post-vaccination as measured by ELISpot, whilst patient 14-5 did not develop immune responses to any of the four antigens of the vaccines. A similar pattern was observed when we compared the PEPI1+ and PEPI3+ sets of all 17 patients in the trial. There was no correlation between the PEPI1+ count and the experimentally determined T cell responses reported from the clinical trial. However, we found good agreements between the T cell immunity predicted by the ≥1 PEPI3+ Test and the reported T cell immunity. The ≥1 PEPI3+ Test predicted the immune responders to HPV DNA vaccine.

Moreover, the diversity of the patient's PEPI3+ set resembled the diversity of T cell responses generally found in cancer vaccine trials. Patients 12-3 and 12-6, similar to patient 14-5, did not have PEPI3+s predicting that the HPV vaccine could not trigger T cell immunity. All other patients had at least one PEPI3 predicting the likelihood that the HPV vaccine can trigger T cell immunity. 11 patients had multiple PEPI3+ predicting that the HPV vaccine likely triggers polyclonal T cell responses. Patients 15-2 and 15-3 could mount high magnitude T cell immunity to E6 of both HPV, but poor immunity to E7. Other patients 15-1 and 12-11 had the same magnitude response to E7 of HPV 18 and HPV16, respectively.

### Example 8 - Design of a Model Population for conducting in silico trials and identifying candidate precision vaccine targets for large population

We compiled an *in silico* human trial cohort of 433 subjects with complete 4-digit HLA class I genotype (2 x HLA-A*xx:xx; 2 x HLA-B*xx:xx; 2 x HLA-C*xx:xx) and demographic information. This Model Population has subjects with mixed ethnicity having a total of 152 different HLA alleles that are representative for >85% of presently known allele G-groups.

We also established a database of a "Big Population" containing 7,189 subjects characterized with 4-digit HLA genotype and demographic information¹¹. The Big Population has 328 different HLA class I alleles. The HLA allele distribution of the Model Population significantly correlated with the Big Population (Table 9) (Pearson p<.001). Consequently the 433 patient Model Population is representative for a 16 times larger population.

**Table 9. Statistical analysis of HLA distributions in "Model Population" vs. "Big Population".**

| We concluded that the Model Population is representative for 85% of the human race as given by HLA diversity as well as HLA frequency. | | | | |
|---|---|---|---|---|
| **Group name 1** | **Group name 2** | **Pearson R value** | **Correlation** | **P Value** |
| 433 Model Population | 7,189 Big Population | 0.89 | Strong | P<0.001 |

### Example 9 In silico trials based on the identification of multiple HLA binding epitopes predict the reported T cell response rates of clinical trials

The objective of this study was to determine whether a model population, such as the one described in Example 8, may be used to predict CTL reactivity rates of vaccines, i.e. used in an *in silico* efficacy trials.

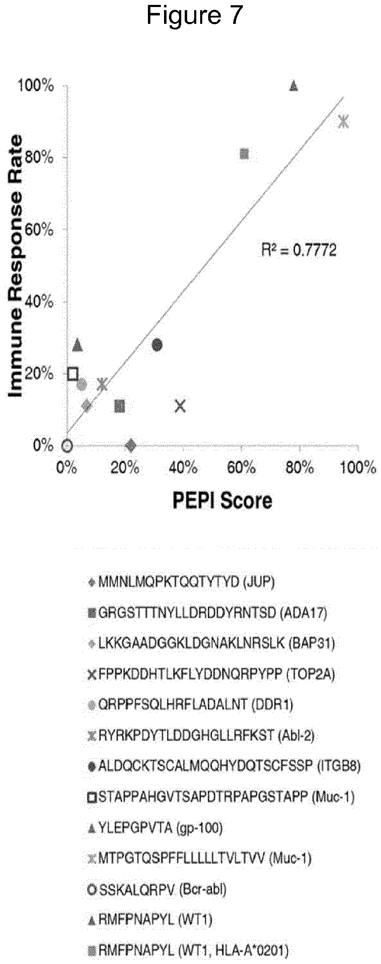

We identified from peer reviewed publications 12 peptide vaccines derived from cancer antigens that induced T cell responses in a subpopulation of subjects (Table 10). These peptides have been investigated in clinical trials enrolling a total of 172 patients (4 ethnicities). T cell responses induced by the vaccine peptides have been determined from blood specimens and reported. We calculated the immune response rate as the percentage of study subjects with positive T cell responses measured in the clinical trials (FIG. 7).

**Table 10. Clinical trials conducted with peptide vaccines.**

| *Peptide vaccines* | *Source antigen* | *Peptide length* | *T cell assay* | *Pop. (n) Ethnicity* | | *Ref.* |
|---|---|---|---|---|---|---|
| MMNLMQPKTQQTYTYD | JUP | 16mer | Multimer | 18 | Canadian | ¹² |
| GRGSTTTNYLLDRDDYRNTSD | ADA17 | 21mer | staining | | | |
| LKKGAADGGKLDGNAKLNRSLK | BAP31 | 22mer | | | | |
| FPPKDDHTLKFLYDDNQRPYPP | | 22mer | | | | |
| RYRKPDYTLDDGHGLLRFKST | | 21mer | | | | |
| QRPPFSQLHRFLADALNT | | 18mer | | | | |
| ALDQCKTSCALMQQHYDQTSCFSSP | | 25mer | | | | |
| STAPPAHGVTSAPDTRPAPGSTAPP | MUC-1 | 25mer | Proliferation | 80 | Canadian | ¹³ |
| YLEPGPVTA | gp100 | 9mer | Tetramer | 18 | US | ¹⁴ |
| MTPGTQSPFFLLLLLTVLTVV | | 21mer | Cytotoxicity | 10 | Israeli | ¹⁵ |
| SSKALQRPV | Bcr-Abl9mer | 9mer | ELISPOT | 4 | US | ¹⁶ |
| RMFPNAPYL | WT-1 | 9mer | Multimer staining | 24 | US | ¹⁷ |
| RMFPNAPYL (HLA-A*0201) | WT-1 | 9mer | Cytokine staining | 18 | CEU | ¹⁸ |

The 12 peptides were investigated with the ≥1 PEPI3+ Test in each of the 433 subjects of the Model Population described in Example 8. The "≧1 PEPI3+ Score" for each peptide was calculated as the proportion of subjects in the Model Population having at least one vaccine derived epitope that could bind to at least three subject-specific HLA class I (≥1 PEPI3+). If the corresponding clinical trial stratified patients for HLA allele selected population, the Model Population was also filtered for subjects with the respective allele(s) (Example: WT1, HLA-A*0201).

The experimentally determined response rates reported from the trials were compared with the ≥1 PEPI3+ Scores. The Overall Percentage of Agreements (OPA) were calculated on the paired data (Table 11). We also found a linear correlation between ≥1 PEPI3+ Score and response rate (R² = 0.77) (FIG. 7). This result shows that the identification of peptides predicted

**Table 11. Comparison of ≥1 PEPI3+ Scores and CTL response rates of 12 peptide vaccines. to bind to multiple HLAs of an individual is useful to predict in silico the outcome of clinical trials.**

| ***Peptide vaccine*** | ***Source antigen*** | ***Response rate (Clinical Trials)*** | ≧***1 PEPI3***+ ***Score**** ***(Model Population)*** | ***OPA*** |
|---|---|---|---|---|
| MMNLMQPKTQQTYTYD | JUP | 0% | 22% | NA |
| GRGSTTTNYLLDRDDYRNTSD | ADA17 | 11% | 18% | 61% |
| LKKGAADGGKLDGNAKLNRSLK | BAP31 | 11% | 7% | 64% |
| FPPKDDHTLKFLYDDNQRPYPP | TOP2A | 11% | 39% | 28% |
| RYRKPDYTLDDGHGLLRFKST | Abl-2 | 17% | 12% | 71% |
| QRPPFSQLHRFLADALNT | DDR1 | 17% | 5% | 29% |
| ALDQCKTSCALMQQHYDQTSCFSSP | ITGB8 | 28% | 31% | 90% |
| STAPPAHGVTSAPDTRPAPGSTAPP | MUC-1 | 20% | 2% | 10% |
| YLEPGPVTA | gp100 | 28% | 4% | 14% |
| MTPGTQSPFFLLLLLTVLTVV | MUC-1 | 90% | 95% | 95% |
| SSKALQRPV | Bcr-Abl | 0% | 0% | 100% |
| RMFPNAPYL | WT-1 | 100% | 78% | 78% |
| RMFPNAPYL (HLA-A*0201) | WT-1 | 81% | 61% | 75% |

| | | | | |
|---|---|---|---|---|
| * *% subjects in the Model Population with* ≧*1 vaccine deprived PEPI3* + | | | | |

### Example 10. In silico trials based on the identification of multiple HLA binding epitopes predict the reported T cell response rates of clinical trials II

We identified a further 19 clinical trials with published immune response rates (IRR) conducted with peptide or DNA based vaccines (Table 12). These trials involved 604 patients (9 ethnicities) and covered 38 vaccines derived from tumor and viral antigens. Vaccine antigen specific CTL responses were measured in each study patient and the response rate in the clinical study populations was calculated and reported.

| Table 12. Response rates published in clinical trials. | | | | | |
|---|---|---|---|---|---|
| **Immunotherapy** | **Type** | **CTL assay** | **Pop. (n)** | **Race/ Ethnicity** | **Ref.** |
| StimuVax | peptide | Proliferation | 80 | Canadian | 13 |
| gp100 vaccine | DNA | Tetramer | 18 | US | 14 |
| IMA901 phase I | peptide | ELISPOT | 64 | CEU | |
| IMA901 phase II | peptide | Multimer staining | 27 | CEU | 19 |
| ICT107 | peptide | ICC | 15 | US | ²0 |
| ProstVac | DNA | ELISPOT | 32 | CEU87%, Afr. Am.12%, Hisp.1% | ²1 |
| Synchrotope TA2M | DNA | Tetramer | 26 | US | ²2 |
| MELITAC 12.1 | peptide | ELISPOT | 167 | US | ²3 |
| WT1 vaccine | peptide | Tetramer | 22 | Japanese | ²4 |
| Ipilimumab (NY-ESO-1) | checkpoi nt inhibitor ** | ICC | 19 | US | 5 |
| VGX-3100 | DNA | ELISPOT | 17 | US | 1 |
| HIVIS-1 | DNA | ELISPOT | 12 | CEU98%, Asian1%, Hisp.1% | 2 |
| ImMucin | peptide | Cytotoxicity | 10 | Israeli | 15 |
| NY-ESO-1 OLP | peptide | IFN-gamma | 7 | Japanese | 7 |
| GVX301 | peptide | Proliferation | 14 | CEU | ²5 |
| WT1 vaccine | peptide | ELISPOT | 12 | US | ²6 |
| WT1 vaccine | peptide | ICC | 18 | CEU | 18 |
| DPX-0907* | peptide | Multimer staining | 18 | Canadian | 12 |
| Melanoma peptide vaccine | peptide | ELISPOT | 26 | White | ²7 |

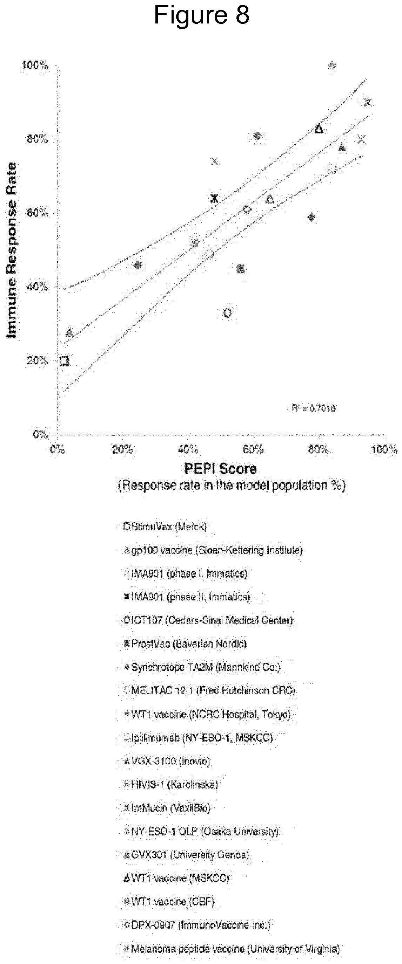

Each vaccine peptide of the 19 clinical trials was investigated with the ≥1 PEPI3+ Test in each subject of the Model Population. The ≥1 PEPI3+ Score for each peptide was calculated as the proportion of subjects in the Model Population having at least one vaccine derived PEPI3+. The experimentally determined response rates reported from the trials were compared with the PEPI Scores, as in Example 9 (Table 13). We found a linear correlation between the response rate and ≥1 PEPI3+ Score (R² = 0.70) (FIG. 8). This result confirms that the identification of peptides predicted to bind to multiple HLAs of an individual can predict T cell responses of subjects, and *in silico* trials can predict the outcome of clinical trials.

**Table 13. Linear correlation between PEPI Score and response rate (R² = 0.7).**

| **Immunotherapy** | **Clinical Trial Response Rate** | **≥1 PEPI3+ Score*** | **OPA** |
|---|---|---|---|
| StimuVax (failed to show efficacy in Phase III) | 20% | 2% | 10% |
| gp100 vaccine | 28% | 4% | 14% |
| IMA901 phase I | 74% | 48% | 65% |
| IMA901 phase II | 64% | 48% | 75% |
| ICT107 | 33% | 52% | 63% |
| ProstVac | 45% | 56% | 80% |
| Synchrotope TA2M | 46% | 24% | 52% |
| MELITAC 12.1 | 49% | 47% | 96% |
| WT1 vaccine | 59% | 78% | 76% |
| Ipilimumab (NY-ESO-1*) | 72% | 84% | 86% |
| VGX-3100 | 78% | 87% | 90% |
| HIVIS-1 | 80% | 93% | 86% |
| ImMucin | 90% | 95% | 95% |
| NY-ESO-1 OLP | 100% | 84% | 84% |
| GVX301 | 64% | 65% | 98% |
| WT1 vaccine | 83% | 80% | 96% |
| WT1 vaccine | 81% | 61% | 75% |
| DPX-0907 | 61% | 58% | 95% |
| Melanoma peptide vaccine | 52% | 42% | 81% |

| | | | |
|---|---|---|---|
| * *% subjects in the Model Population with* ≧*1 vaccine derived PEPI3+* | | | |

### Example 11 - In silico trial based on the identification of multiple HLA binding epitopes in a multi-peptide vaccine predict the reported clinical trial immune response rate

IMA901 is a therapeutic vaccine for renal cell cancer (RCC) comprising 9 peptides derived from tumor-associated peptides (TUMAPs) that are naturally presented in human cancer tissue ¹⁹. A total of 96 HLA-A*02+ subjects with advanced RCC were treated with IMA901 in two independent clinical studies (phase I and phase II)¹⁹. Each of the 9 peptides of IMA901 have been identified in the prior art as HLA-A2-restricted epitopes. Based on currently accepted standards, they are all strong candidate peptides to boost T cell responses against renal cancer in the trial subjects, because their presence has been detected in renal cancer patients, and because the trial patients were specifically selected to have at least one HLA molecule capable of presenting each of the peptides.

We determined for each subject in the Model population how many of the nine peptides

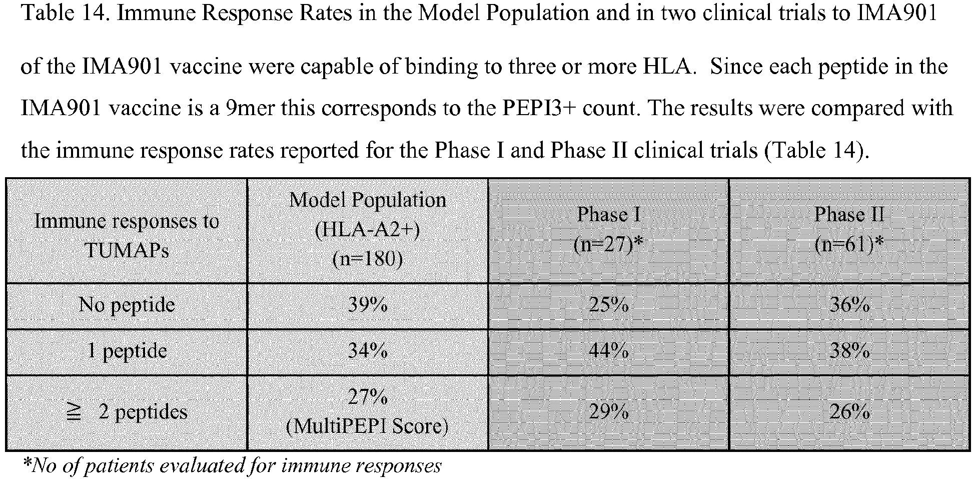

The phase I and phase II study results show the variability of the immune responses to the same vaccine in different trial cohorts. Overall, however, there was a good agreement between response rates predicted by the ≥2 PEPI3+ Test and the reported clinical response rates.

In a retrospective analysis, the clinical investigators of the trials discussed above found that subjects who responded to multiple peptides of the IMA901 vaccine were significantly (p = 0.019) more likely to experience disease control (stable disease, partial response) than subjects who responded only to one peptide or had no response. 6 of 8 subjects (75%) who responded to multiple peptides experienced clinical benefit in the trial, in contrast to 14% and 33% of 0 and 1 peptide responders, respectively. The randomized phase II trial confirmed that immune responses to multiple TUMAPs were associated with a longer overall survival.

The reported disease control rate (DCR) of the phase I and phase II clinical trials were 43% and 22% respectively. The tumors of the vaccinated patients were reasonably assumed to present the target TUMAPs. The reported disease control rates approximate to the percentage of subjects in our Model Population predicted by the ≥1 PEPI3+ Test to mount an immune response to at least two of the nine IMA901 9mer peptides (27%), also suggesting that this is the threshold for identifying likely clinical responders to the IMA901 vaccine. The Disease Control Rate of the clinical trials was predicted with 75% probability by the identification of ≥1 PEPI3+ in at least two of the 9mer peptide antigens of the IMA901 vaccine (corresponds for this vaccine of nine 9mer peptides to ≥2 PEPI3+ in total). In contrast, a single PEPI3+ in the 9 peptide vaccine was not predictive of clinical benefit.

### Example 12 - In silico trial based on the identification of vaccine-derived multiple HLA binding epitopes predict reported experimental clinical response rates

We demonstrate here a correlation between the ≥2 PEPI3+ Score of immunotherapy vaccines determined in our Model Population and the reported Disease Control Rate (DCR, proportion of patients with complete responses and partial responses and stable disease) determined in clinical trials.

In peer reviewed scientific journals we found 17 clinical trials, conducted with peptide- and DNA-based cancer immunotherapy vaccines that have published Disease Control Rates (DCRs) or objective response rate (ORR) (Table 15). These trials involved 594 patients (5 ethnicities) and covered 29 tumor and viral antigens. DCRs were determined according to the Response Evaluation Criteria in Solid Tumors (RECIST), which is the current standard for clinical trials, in which clinical responses are based on changes in maximum cross-sectional dimensions^{42, 43, 44.} In case there was no available DCR data, objective response rate (ORR) data was used, which is also defined according to the RECIST guidelines.

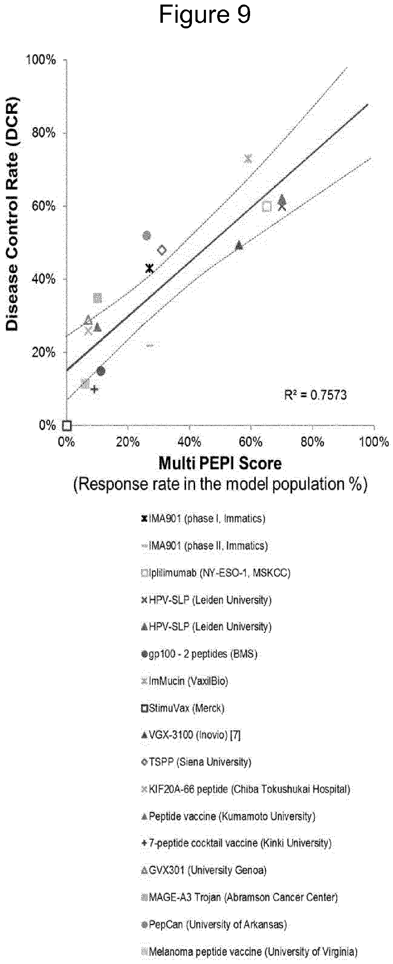

Table 16 compares the ≥2 PEPI3+ Score for each vaccine in the Model Population and the published DCR or ORR. We found a remarkable correlation between the predicted and measured DCR providing further evidence that not only the immunogenicity but also the potency of cancer vaccines depends on the multiple HLA sequences of individuals (R² = 0.76) (FIG. 9).

**Table 15.Clinical trials selected for Disease Control Rate (DCR) prediction.**

| **Immuno-therapy** | **Antigen** | **Sponsor** | **Disease** | **Pop. (n)** | **Study pop./ Ethnicity** | **HLA restriction** | **Adm. form** | **Dose (mg)** | **Dosing schedule** | **Assessmen t time (weeks)** | **Ref.** |
|---|---|---|---|---|---|---|---|---|---|---|---|
| **IMA901 phase I** | 9 TAAs | Immatics | Renal cell cancer | 28 | CEU | A02 | i.d. | 0.4 | 8x in 10 wks | 12 | 19 |
| **IMA901 phase II** | 9 TAAs | Immatics | Renal cell cancer | 68 | CEU | A02 | i.d | 0.4 | 7x in 5 wks then 10x 3 wks | 24 | 19 |
| **Ipilimumab** | NY-ESO-1 | MSKCC | Melanoma | 19 | US | no | i.v. | 0.3 3 10 | 4 x every 3 wks | 24 | 5 |
| **HPV-SLP*** | HPV-16 E6, | Leiden University | VIN | 20 | CEU | no | s.c. | 0.3 | 3 x every 3 wks | 12 | 9 |
| **HPV-SLP*** | E7 | Leiden University | HPV-related cervical cancer | 5 | CEU | no | s.c. | 0.3 | 3 x every 3 wks | 12 (OR) | 10 |
| **gp100** - 2 **peptides*** | gp100 | BMS | Melanoma | 136 | US | A*0201 | s.c. | 1 | 4 x every 3 wks | 12 | ²8 |
| **Immucin** | Muc-1 | VaxilBio | Myeloma | 15 | Israeli | no | s.c. | 0.1 | 6 x every 2 wks | 12** | 29 |
| **StimuVax** | Muc-1 | Merck | NSCLC | 80 | Canadian | no | s.c. | 1 | 8x wkly then every 6 wks | 12 | 13, 30 |
| **VGX-3100** | HPV-16&18 | Inovio | HPV-related cervical cancer | 125 | US | no | i.m. | 6 | 0, 4, 12 wks | 36 | ³1 |
| **TSPP peptide vaccine** | Thymidylate synthase | Siena University | CRC, NSCLC, Gallbladder carc., Breast-, Gastric cancer | 21 | CEU | no | s.c. | 0.1 0.2 0.3 | 3 x 3 wks | 12 | 32 |
| **KIF20A-66 peptide vaccine*** | KIF20A | Chiba Tokushukai Hospital | Metastatic pancreatic cancer | 29 | Japanese | A*2402 | s.c. | 1 3 | 2 cycles 1, 8, 15, 22 days then every 2 wks | 12 (OR) | 33 |
| **Peptide vaccine*** | 3 TAAs | Kumamoto University | HNSCC | 37 | Japanese | A*2402 | s.c. | 1 | 8 x wkly then every 4 wks | 12 | ³4 |
| **7-peptide cocktail vaccine*** | 7 TAAs | Kinki University | Metastatic colorectal cancer | 30 | Japanese | A*2402 | s.c. | 1 | Cycles: 5 x wkly then 1 wk rest | 10 (OR) | ³5 |
| **GVX301*** | hTERT | University Genoa | Prostate and renal cancer | 14 | Japanese | A02 | i.d. | 0.5 | 1, 3, 5, 7, 14, 21, 35, 63 days | 12 | 25 |
| **MAGE**-**A3 Trojan*** | MAGE-A3 | Abramson Cancer Center | Multiple myeloma | 26 | US | no | s.c. | 0.3 | 14, 42, 90, 120, 150 days | 24 | ³6 |
| **PepCan** | HPV-16 E6 | University of Arkansas | CIN2/3 | 23 | US | no | i.m. | 0.05 0.1 0.25 0.5 | 4x3wks | 24 | ³7 |
| **Melanoma peptide vaccine*** | Tyrosinase, gp100 | University of Virginia | Melanoma | 26 | US | A1, A2 or A3 | s.c. | 0.1 | 6 cycles: 0, 7, 14, 28, 35, 42 days | 6 | 27 |

| | | | | | | | | | | | |
|---|---|---|---|---|---|---|---|---|---|---|---|
| *Montanide ISA51 VG as adjuvant **Disease response was assessed according to the International Myeloma Working Group response criteria ⁴⁵ | | | | | | | | | | | |

**Table 16. The Disease Control Rates (DCRs) and MultiPEPI Scores (predicted DCR) in 17 clinical trials.**

| **Immunotherapy** | **DCR** | **MultiPEPI Score (Predicted DCR)** | **Overall Percentage of Agreement** |
|---|---|---|---|
| **IMA901 phase I** | 43% | 27% | ***61%*** |
| **IMA901 phase II** | 22% | 27% | ***81%*** |
| **Ipilimumab** | 60% | 65% | ***92%*** |
| **HPV-SLP** | 60% | 70% | ***86%*** |
| **HPV-SLP** | 62% | 70% | ***89%*** |
| **gp100 - 2 peptides** | 15% | 11% | ***73%*** |
| **Immucin** | 73% | 59% | ***81%*** |
| **StimuVax** | 0% | 0% | ***100%*** |
| **VGX-3100** | 50% | 56% | ***89%*** |
| **TSPP peptide vaccine** | 48% | 31% | ***65%*** |
| **KIF20A-66 peptide vaccine** | 26% | 7% | ***27%*** |
| **Peptide vaccine** | 27% | 10% | ***37%*** |
| **7-peptide cocktail vaccine** | 10% | 9% | ***90%*** |
| **GVX301** | 29% | 7% | ***24%*** |
| **MAGE-A3 Trojan** | 35% | 10% | ***29%*** |
| **PepCan** | 52% | 26% | ***50%*** |
| **Melanoma peptide vaccine** | 12% | 6% | ***50%*** |

### Example 14. Breast cancer vaccine design for large population and composition

We used the PEPI3+ Test described above to design peptides for use in breast cancer vaccines that are effective in a large percentage of patients, taking into account the heterogeneities of both tumour antigens and patients' HLAs.

Breast cancer CTAs were identified and ranked based on the overall expression frequencies of antigens found in breast cancer tumor samples as reported in peer reviewed publications (data sources: Gudmundsdotter et al. Amplified antigen-specific immune responses in HIV-1 infected individuals in a double blind DNA immunization and therapy interruption trial. Vaccine. 2011; 29(33):5558-66; Bioley et al. HLA class I - associated immunodominance affects CTL responsiveness to an ESO recombinant protein tumor antigen vaccine. Clin Cancer Res. 2009; 15(1):299-306; Valmori et al. Vaccination with NY-ESO-1 protein and CpG in Montanide induces integrated antibody/Th1 responses and CD8 T cells through cross-priming. Proceedings of the National Academy of Sciences of the United States of America. 2007; 104(21):8947-52; Kakimi et al. A phase I study of vaccination with NY-ESO-1f peptide mixed with Picibanil OK-432 and Montanide ISA-51 in patients with cancers expressing the NY-ESO-1 antigen.Int J Cancer. 2011;129(12):2836-46; Wada et al. Vaccination with NY-ESO-1 overlapping peptides mixed with Picibanil OK-432 and montanide ISA-51 in patients with cancers expressing the NY-ESO-1 antigen. J Immunother. 2014;37(2):84-92.).

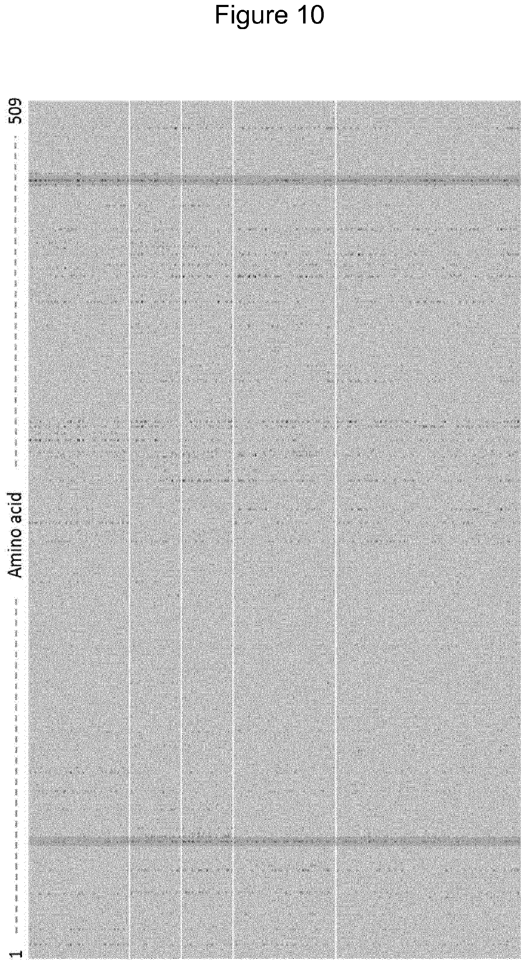

For select CTAs we used the PEPI3+ Test and the Model Population described in Example 8 to identify the 9mer epitopes (PEPI3+s) that are most frequently presented by at least 3HLAs of the individuals in the Model Population. We refer to these epitopes herein as "bestEPIs". An illustrative example of the "PEPI3+ hotspot" analysis and bestEPI identification is shown in FIG. 10 for the PRAME antigen.

We multiplied the reported expression frequency for each CTA by the frequency of the PEPI3+ hotspots in the Model Population to identify the T cell epitopes (9mers) that will induce an immune response against breast cancer antigens in the highest proportion of individuals (Table 17, SEQ ID NOs: 1 to 20).

**Table 17 BestEPI list for selecting breast cancer peptide vaccine composition. Ntotal: number of samples analyzed for the expression of the certain antigen; N+: number of individuals expressing the certain antigen; N%: expression frequency of the certain antigen; B%: bestEPI frequency, ie. the percentage of induviduals having the bestEPI within the model population; N%*B%: expression frequency multiplied by the bestEPI frequency.**

| SEQ ID NO. | Antigen Information | | | | | BestEPIs | | | |
|---|---|---|---|---|---|---|---|---|---|
| | Gene | length | Ntotal | N+ | N% | SEQ | Position | B% | N%*B% |
| 1 | AKAP-4 | 854 | 91 | 77 | 85% | YLMNRPQNL | 167 | 52% | 44% |
| 2 | AKAP-4 | 854 | 91 | 77 | 85% | MMAYSDTTM | 1 | 49% | 41% |
| 3 | BORIS | 663 | 58 | 41 | 71% | FTSSRMSSF | 264 | 57% | 40% |
| 4 | AKAP-4 | 854 | 91 | 77 | 85% | YALGFQHAL | 121 | 46% | 39% |
| 5 | SPAG9 | 1321 | 100 | 88 | 88% | KMSSLLPTM | 964 | 43% | 38% |
| 6 | SPAG9 | 1321 | 100 | 88 | 88% | FTVCNSHVL | 785 | 36% | 31% |
| 7 | BORIS | 663 | 58 | 41 | 71% | MAFVTSGEL | 320 | 44% | 31% |
| 8 | PRAME | 509 | 100 | 55 | 55% | YLHARLREL | 462 | 52% | 28% |
| 9 | SPAG9 | 1321 | 100 | 88 | 88% | VMSERVSGL | 19 | 28% | 25% |
| 10 | BORIS | 663 | 58 | 41 | 71% | FTQSGTMKI | 407 | 35% | 25% |
| 11 | NY-SAR-35 | 255 | 29 | 14 | 48% | FSSSGTTSF | 163 | 45% | 22% |
| 12 | MAGE-A9 | 315 | 142 | 63 | 44% | FMFQEALKL | 102 | 49% | 22% |
| 13 | NY-SAR-35 | 255 | 29 | 14 | 48% | FVLANGHIL | 97 | 42% | 21% |
| 14 | PRAME | 509 | 100 | 55 | 55% | KAMVQAWPF | 70 | 37% | 20% |
| 15 | NY-BR-1 | 1341 | 131 | 61 | 47% | YSCDSRSLF | 424 | 39% | 18% |
| 16 | Survivin | 142 | 167 | 118 | 71% | RAIEQLAAM | 133 | 26% | 18% |
| 17 | MAGE-A11 | 429 | 135 | 79 | 59% | AMDAIFGSL | 184 | 23% | 14% |
| 18 | HOM-TES-85 | 313 | 100 | 47 | 47% | MASFRKLTL | 1 | 29% | 13% |
| 19 | MAGE-A9 | 315 | 142 | 63 | 44% | SSISVYYTL | 67 | 30% | 13% |
| 20 | NY-BR-1 | 1341 | 131 | 61 | 47% | SAFEPATEM | 584 | 27% | 12% |

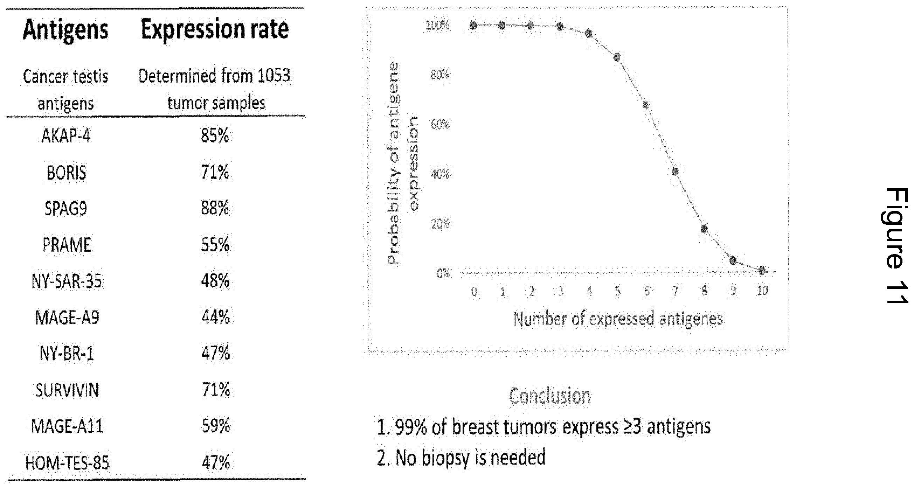

Then we designed 31 30mer peptides (SEQ ID NOs: 81 to 111, Table 18a). The 30mers may each consist of two optimized 15mer fragments (SEQ ID NOs: 41 to 60), generally from different frequent CTAs, arranged end to end, each fragment comprising one of the 9mers (BestEPIs) from Table 17 (SEQ ID NOs: 1 to 20). Nine of these 30mer peptides were selected for a panel of peptides, referred to as PolyPEPI915 (Table 18b). Expression frequencies for the 10 CTAs targeted by PolyPEPI915, singly and in combination, are shown in FIG. 11.

**Table 18a - 30mer breast cancer vaccine peptides**

| SEQID | TREOSID | Source Antigen | Peptide (30mer) |
|---|---|---|---|
| 81 | BCV900-2-1 | AKAP4 | LQKYALGFQHALSPSMMAYSDTTMMSDDID |
| 82 | BCV900-2-2 | BORIS/AKAP4 | VCMFTSSRMSSFNRHVNIDYLMNRPQNLRL |
| 83 | BCV900-2-3 | BORIS | NMAFVTSGELVRHRRHTRFTQSGTMKIHIL |
| 84 | BCV900-2-4 | SPAG9 | LDSFTVCNSHVLCIAKLGFSFVRITALMVS |
| 85 | BCV900-2-5 | SPAG9/NY-SAR-35 | AQKMSSLLPTMWLGAMMQMFGLGAISLILV |
| 86 | BCV900-2-6 | PRAME | LERLAYLHARLRELLQTLKAMVQAWPFTCL |
| 87 | BCV900-2-7 | NY-SAR-35 | SSYFVLANGHILPNSLRHKCCFSSSGTTSF |
| 88 | BCV900-2-8 | Survivin/MAGE-A9 | TAKKVRRAIEQLAAMQLEFMFQEALKLKVA |
| 89 | BCV900-2-9 | MAGE-A11/NY-BR-1 | TSHSYVLVTSLNLSYYSCDSRSLFESSAKI |
| 90 | BCV900-3-1 | SPAG9/BORIS | LDSFTVCNSHVLCIAVCMFTSSRMSSFNRH |
| 91 | BCV900-3-2 | NY-SAR-35/PRAME | LRHKCCFSSSGTTSFQTLKAMVQAWPFTCL |
| 92 | BCV900-3-3 | NY-BR-1/SURVIVIN | YSCDSRSLFESSAKITAKKVRRAIEQLAAM |
| 93 | BCV900-3-4 | AKAP-4/BORIS | MMAYSDTTMMSDDIDHTRFTQSGTMKIHIL |
| 94 | BCV900-3-5 | SPAG9/AKAP-4 | AQKMSSLLPTMWLGALQKYALGFQHALSPS |
| 95 | BCV900-3-6 | MAGE-A11/BORIS | TSHSYVLVTSLNLSYNMAFVTSGELVRHRR |
| 96 | BCV900-3-7 | NY-SAR-35/AKAP-4 | MMQMFGLGAISLILVVNIDYLMNRPQNLRL |
| 97 | BCV900-3-8 | NY-SAR-35/SPAG-9 | SSYFVLANGHILPNSKLGFSFVRITALMVS |
| 98 | BCV900-3-9 | PRAME/MAGE-A9 | LERLAYLHARLRELLQLEFMFQEALKLKVA |
| 99 | BCV900-4-1 | SPAG9/AKAP4 | GNILDSFTVCNSHVLLQKYALGFQHALSPS |
| 100 | BCV900-4-2 | BORIS/NY-SAR-35 | NMAFVTSGELVRHRRFSSSGTTSFKCFAPF |
| 101 | BCV900-4-5 | SPAG9/BORIS | AQKMSSLLPTMWLGAMFTSSRMSSFNRHMK |
| 102 | BCV900-4-6 | MAGE-A11/PRAME | TSHSYVLVTSLNLSYHSQTLKAMVQAWPFT |
| 103 | BCV900-5-6 | HomTes85/MageA11 | MASFRKLTLSEKVPPSPTAMDAIFGSLSDE |
| 104 | BCV900-5-7 | AKAP4/PRAME | DQVNIDYLMNRPQNLRHSQTLKAMVQAWPF |
| 105 | BCV900-5-8 | NYSAR/SPAG9 | CSGSSYFVLANGHILSGAVMSERVSGLAGS |
| 106 | BCV900-S-2 | AKAP-4/MAGE-A9 | DLSFYVNRLSSLVIQSSISVYYTLWSQFDE |
| 107 | BCV900-S-4 | SPAG9/NY-ESO-1 | SGAVMSERVSGLAGSSRLLEFYLAMPFATP |
| 108 | BCV900-S-6 | HOM-TES-85/MAGE-A11 | MASFRKLTLSEKVPPESFSPTAMDAIFGSL |
| 109 | BCV900-S-7 | NY-ESO-1/NY-BR-1 | FYLAMPFATPMEAELKPSAFEPATEMQKSV |
| 110 | BCV900-T-27 | MAGE-A11/PRAME | AMDAIFGSLSDEGSGHSQTLKAMVQAWPFT |
| 111 | BCV900-T-28 | NY-SAR-35/SPAG9 | FVLANGHILPNSENAGTGKLGFSFVRITAL |

**Table 18b - Selected Breast Cancer Vaccine peptides for PolyPEPI915 panel/composition**

| SEQID | TREOSID | Source Antigen | Peptide (30mer) | HLAI* (CD8) | HLAII** (CD4) |
|---|---|---|---|---|---|
| 99 | BCV900-4-1 | SPAG9/AKAP4 | GNILDSFTVCNSHVLLQKYALGFQHALSPS | 53% | 75% |
| 100 | BCV900-4-2 | BORIS/NY-SAR-35 | NMAFVTSGELVRHRRFSSSGTTSFKCFAPF | 65% | 46% |
| 92 | BCV900-3-3 | NY-BR-1/SURVIVIN | YSCDSRSLFESSAKITAKKVRRAIEQLAAM | 55% | 11% |
| 93 | BCV900-3-4 | AKAP-4/BORIS | MMAYSDTTMMSDDIDHTRFTQSGTMKIHIL | 72% | 45% |
| 101 | BCV900-4-5 | SPAG9/BORIS | AQKMSSLLPTMWLGAMFTSSRMSSFNRHMK | 72% | 50% |
| 103 | BCV900-5-6 | HomTes85/MageA11 | MASFRKLTLSEKVPPSPTAMDAIFGSLSDE | 45% | 16% |
| 104 | BCV900-5-7 | AKAP4/PRAME | DQVNIDYLMNRPQNLRHSQTLKAMVQAWPF | 64% | 33% |
| 105 | BCV900-5-8 | NYSAR/SPAG9 | CSGSSYFVLANGHILSGAVMSERVSGLAGS | 46% | 48% |
| 98 | BCV900-3-9 | PRAME/MAGE-A9 | LERLAYLHARLRELLQLEFMFQEALKLKVA | 73% | 100% |
| | | | PolyPEPI915 (9 peptide together) | 96% | 100% |

| | | | | | |
|---|---|---|---|---|---|
| * Percentage of individuals having CD8+ T cell specific PEPI3+ within the Model Population (n=433). **Percentage of individuals having CD4+ T cell specific PEPI4+ within the Model Population (n=433). | | | | | |

### Characterization of PolyPEPI915

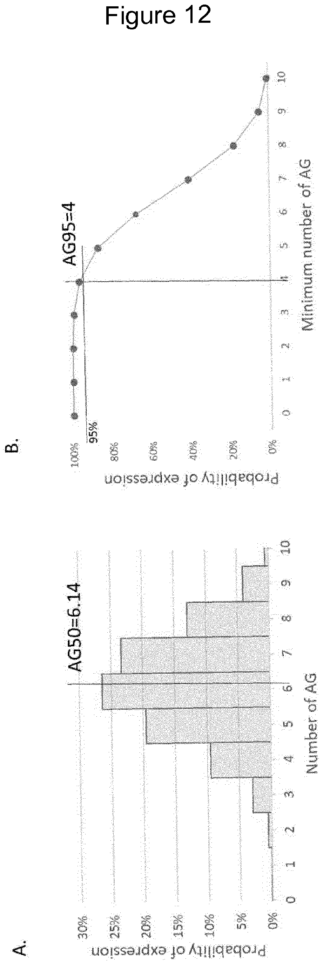

Tumor heterogeneity can be addressed by including peptide sequences that target multiple CTAs in a vaccine or immunotherapy regime. The PolyPEPI915 composition targets 10 different CTAs. Based on the antigen expression rates for these 10 CTAs, we modelled the predicted average number of expressed antigens (AG50) and the minimum number of expressed antigens with 95% likelihood (AG95) in the cancer cells. 95% of individuals expressed minimum 4 of the 10 target antigens (AG95=4) as shown by the antigen expression curve in **FIG. 12**.

The AG values described above characterize a vaccine independently from the target patient population. They can be used to predict the likelihood that a specific cancer (e.g. breast cancer) expresses antigens targeted by a specific vaccine or immunotherapy composition. AG values are based on known tumor heterogeneity, but do not take HLA heterogeneity into account.

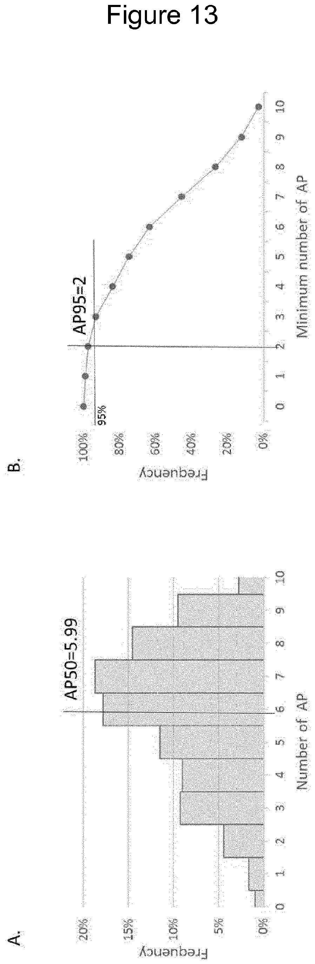

HLA heterogeneity of a certain population can be characterised from the viewpoint of a immunotherapy or vaccine composition by the number of antigens representing PEPI3+. These are the vaccine-specific CTA antigens for which ≥1 PEPI3+ is predicted, referred to herein as the "AP". The average number of antigens with PEPI3+ (AP50) shows how the vaccine can induce immune response against the antigens targeted by the composition (breast cancer vaccine specific immune response). The PolyPEPI915 composition can induce immune response against an average of 5.3 vaccine antigens (AP50=5.30) and 95% of the Model Population can induce immune response against at least one vaccine antigen (AP95=1)(FIG. 13).

Vaccines can be further characterized by AGP values that refers to "antigens with PEPIs". This parameter is the combination of the previous two parameters: (1) AG is depending on the antigen expression frequencies in the specific tumor type but not on the HLA genotype of individuals in the population, and (2) AP is depending on the HLA genotype of individuals in a population without taking account the expression frequencies of the antigen. The AGP is depending on both, the expression frequencies of vaccine antigens in the disease and the HLA genotype of individuals in a population.

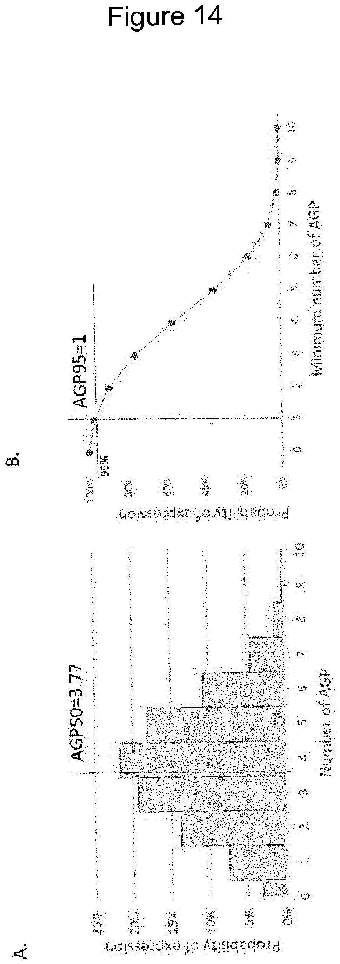

Combining the data of AG of breast cancer and AP in the Model Population we determined the AGP value of PolyPEPI915 that represents the probability distribution of vaccine antigens that are induce immune responses against antigens expressed in breast tumors. For PolyPEPI915, the APG50 value in the Model Population is 3.37. The AGP92=1, means that 92% of the subjects in the Model Population induce immune responses against at least one expressed vaccine antigen (FIG. 14).

### Example 15 - Patient selection using companion diagnostic for breast cancer vaccine

The likelihood that a specific patient will have an immune response or a clinical response to treatment with one or more cancer vaccine peptides, for example as described above, can be determined based on (i) the identification of PEPI3+ within the vaccine peptide(s) (9mer epitopes capable of binding at least three HLA of the patient); and/or (ii) a determination of target antigen expression in cancer cells of the patient, for example as measured in a tumour biopsy. Ideally both parameters are determined and the optimal combination of vaccine peptides is selected for use in treatment of the patient. However, PEPI3+ analysis alone may be used if a determination of the expressed tumour antigens, for example by biopsy, is not possible, not advised, or unreliable due to biopsy error (i.e. biopsy tissue samples taken from a small portion of the tumor or metastasised tumors do not represent the complete repertoire of CTAs expressed in the patient).

### Example 16 - Comparison of PolyPEPI915 with competing breast cancer vaccines

We used the *in silico* clinical trial model described in above to predict the immune response rates of competing breast cancer vaccines that investigated in clinical trials (Table 19). The immune response rate of these products were between 3% and 91%.

The single peptide vaccines were immunogenic in 3% - 23% of individuals. In comparison, peptides having an amino acid sequence selected from SEQ ID NOs: 81-111 were immunogenic in from 44% to 73% of individuals in the same cohorts. This result represents substantial improvement in immunogenicity of each peptide in PolyPEPI915.

Competing combination peptide products immune response rates were between 10 - 62%. The invented PolyPEPI915 combination product were 96% in the Model Population and 93% in a breast cancer patient population representing improvement in immunogenicity.

**Table 19. Predicted immune response rates of competing breast cancer vaccines**

| | | | Predicted immune response rates* | |
|---|---|---|---|---|
| Breast Cancer Vaccines | Sponsors | Target antigens | 433 normal donors | 90 patients |
| | | | (Model Population) | with breast cancer |
| DPX0907 Multipeptide | ImmunoVaccine Tech. | 7 | 58% | 62% |
| Multipeptide vaccine | University of Virginia | 5 | 22% | 31% |
| Ad-sig-hMUC-1/ecdCD40L | Singapore CRI | 1 | 91% | 80% |
| NY-ESO-1 IDC-G305 | Immune Design Corp. | 1 | 84% | 84% |
| 6 HER2 peptide pulsed DC | University Pennsylvania | 1 | 29% | 36% |
| HER-2 B Cell peptide | Ohio State University | 1 | 18% | 23% |
| HER-2/neu ID protein | University Washington | 1 | 10% | 11% |
| NeuVax peptide | Galena Biopharma | 1 | 6% | 3% |
| StimuVax®(L-BLP25) peptide | EMD Serono | 1 | 6% | 8% |
| PolyPEPI915 | Treos Bio | 10 | 96% | 93% |

| | | | | |
|---|---|---|---|---|
| *Proportion of subjects with ≧ 1 PEPI3+ | | | | |

Another improvement of using the PolyPEPI915 vaccine is the lower chance of tumor escape. Each 30mer peptide in PolyPEPI915 targets 2 tumor antigens. CTLs against more tumor antigens are more effective against heterologous tumor cells that CTLs against a single tumor antigen.

Another improvement is that PolyPEPI915 vaccine that individuals who likely respond to vaccination can be identified based on HLA sequences and optionally antigen expression in their tumor using the methods described here. Pharmaceutical compositions with PolyPEPI vaccines will not be administered to individuals who's HLA cannot present any PEPI3 from the vaccines. During clinical trials correlation will be made between the number of AGP in the PolyPEPI915 regimen and the duration of individual's responses. A vaccine combination with > 1 AGP is most likely required to destroy heterologous tumor cells.
Pharmaceutical compositions with PolyPEPI vaccines will not be administered to individuals who's HLA cannot present any PEPI3 from the vaccines.

### Example 17 Colorectal cancer vaccine design and composition

We show another example for colorectal vaccine composition using the same design method demonstrated above. We used the PEPI3+ Test described above to design peptides for use in colorectal cancer vaccines that are effective in a large percentage of patients, taking into account the heterogeneities of both tumour antigens and patient HLAs.

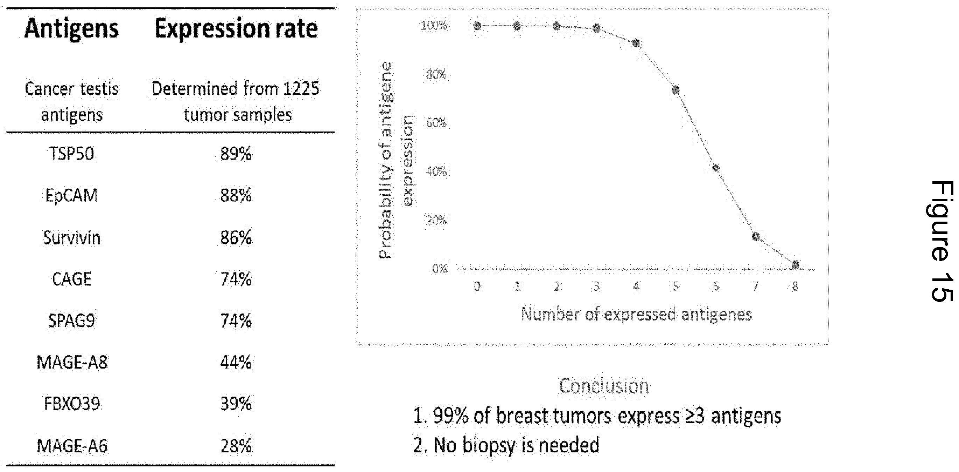

Colorectal cancer CTAs were identified and ranked based on the overall expression frequencies of antigens found in breast cancer tumor samples as reported in peer reviewed publications (FIG. 15) (Gudmundsdotter et al. Amplified antigen-specific immune responses in HIV-1 infected individuals in a double blind DNA immunization and therapy interruption trial. Vaccine. 2011; 29(33):5558-66; Bioley et al. HLA class I - associated immunodominance affects CTL responsiveness to an ESO recombinant protein tumor antigen vaccine. Clin Cancer Res. 2009; 15(1):299-306; Valmori et al. Vaccination with NY-ESO-1 protein and CpG in Montanide induces integrated antibody/Th1 responses and CD8 T cells through cross-priming. Proceedings of the National Academy of Sciences of the United States of America. 2007; 104(21):8947-52; Kakimi et al. A phase I study of vaccination with NY-ESO-1f peptide mixed with Picibanil OK-432 and Montanide ISA-51 in patients with cancers expressing the NY-ESO-1 antigen.Int J Cancer. 2011;129(12):2836-46; Wada et al. Vaccination with NY-ESO-1 overlapping peptides mixed with Picibanil OK-432 and montanide ISA-51 in patients with cancers expressing the NY-ESO-1 antigen. J Immunother. 2014;37(2):84-92).

For the selection of the most frequently expressed colorectal cancer CTAs we used the PEPI3+ Test and the Model Population described in Example 8 to identify the "bestEPIs".

We multiplied the reported expression frequency for each CTA by the frequency of the PEPI3+ hotspots in the Model Population to identify the T cell epitopes (9mers) that will induce an immune response against colorectal cancer antigens in the highest proportion of individuals (Table 20, SEQ ID NOs: 21 to 40).

**Table 20 BestEPI list for selecting colorectal cancer peptide vaccine composition. Ntotal: number of biopsy samples (tumor specific antigen expression in human colorectal cancer tissues) analyzed for the expression of the certain antigen; N+: number of individuals expressing the certain antigen; N%: expression frequency of the certain antigen; B%: bestEPI frequency, ie. the percentage of induviduals having the bestEPI within the model population; N%*B%: expression frequency multiplied by the bestEPI frequency.**

| SEQ ID NO. | Antigen Information | | | | | BestEPIs | | | |
|---|---|---|---|---|---|---|---|---|---|
| | Gene | LEN | Ntotal | N+ | N% | SEQ | POS | B% | N%*B% |
| 21 | TSP50 | 385 | 95 | 85 | 89% | FSYEQDPTL | 106 | 51% | 45.7% |
| 22 | EpCAM | 314 | 309 | 273 | 88% | RTYWIIIEL | 140 | 51% | 45.1% |
| 23 | TSP50 | 385 | 95 | 85 | 89% | TTMETQFPV | 85 | 36% | 32.6% |
| 24 | Spag9 | 1321 | 78 | 58 | 74% | FSFVRITAL | 1143 | 44% | 32.6% |
| 25 | Spag9 | 1321 | 78 | 58 | 74% | KMSSLLPTM | 964 | 43% | 32.1% |
| 26 | CAGE1 | 777 | 47 | 35 | 74% | KMHSLLALM | 616 | 42% | 31.5% |
| 27 | FBXO39 | 442 | 57 | 22 | 39% | FMNPYNAVL | 96 | 78% | 30.1% |
| 28 | CAGE1 | 777 | 47 | 35 | 74% | KSMTMMPAL | 760 | 37% | 27.3% |
| 29 | EpCAM | 314 | 309 | 273 | 88% | YVDEKAPEF | 251 | 28% | 24.7% |
| 30 | FBXO39 | 442 | 57 | 22 | 39% | KTMSTFHNL | 218 | 58% | 22.2% |
| 31 | Survivin | 142 | 309 | 267 | 86% | RAIEQLAAM | 133 | 26% | 22.2% |
| 32 | Spag9 | 1321 | 78 | 58 | 74% | VMSERVSGL | 19 | 28% | 21.0% |
| 33 | TSP50 | 385 | 95 | 85 | 89% | YRAQRFWSW | 192 | 20% | 17.8% |
| 34 | FBXO39 | 442 | 57 | 22 | 39% | FFFERIMKY | 287 | 46% | 17.6% |
| 35 | Survivin | 142 | 309 | 267 | 86% | STFKNWPFL | 20 | 15% | 13.0% |
| 36 | Mage-A8 | 318 | 80 | 35 | 44% | AIWEALSVM | 223 | 20% | 8.7% |
| 37 | Mage-A8 | 318 | 80 | 35 | 44% | KVAELVRFL | 115 | 18% | 7.7% |
| 38 | Mage-A6 | 314 | 250 | 69 | 28% | FVQENYLEY | 250 | 27% | 7.5% |
| 39 | Mage-A8 | 318 | 80 | 35 | 44% | RALAETSYV | 279 | 16% | 7.1% |
| 40 | Mage-A6 | 314 | 250 | 69 | 28% | YIFATCLGL | 176 | 25% | 6.9% |

Then we designed 31 30mer peptides (SEQ ID NOs: 112 to 142, Table 21a). The 30mers may each consist of two optimized 15mer fragments (SEQ ID NOs: 61 to 80), generally from different frequent CTAs, where the 15mer fragments are arranged end to end, each fragment comprising one of the 9mers (BestEPIs) from Table 20 (SEQ ID NOs: 21 to 40). Nine of these 30mer peptides were selected for a panel of peptide vaccines, referred to as PolyPEPI1015 (Table 21b). Expression frequencies for the 8 CTAs targeted by PolyPEPI1015, singly and in combination, are shown in FIG. 15.

**Table 21a - 30mer colorectal cancer vaccine peptides**

| SEQID | TREOSID | Source Antigen | Peptide (30mer) |
|---|---|---|---|
| 112 | CCV1000-1-1 | TSP50 | VCSMEGTWYLVGLVSYRSCGFSYEQDPTLR |
| 113 | CCV1000-1-2 | EpCAM/TSP50 | VRTYWIIIELKHKARLPSTTMETQFPVSEG |
| 114 | CCV1000-1-4 | Survivin | TAKKVRRAIEQLAAMMGAPTLPPAWQPFLK |
| 115 | CCV1000-1-5 | CAGE1 | LASKMHSLLALMVGLPKSMTMMPALFKENR |
| 116 | CCV1000-1-6 | Spag9 | KLGFSFVRITALMVSLDSFTVCNSHVLCIA |
| 117 | CCV1000-1-7 | FBXO39 | KFMNPYNAVLTKKFQFKKTMSTFHNLVSLN |
| 118 | CCV1000-1-8 | Spag9/FBXO39 | AQKMSSLLPTMWLGAKVNFFFERIMKYERL |
| 119 | CCV1000-1-9 | Survivin/Mage-A8 | KDHRISTFKNWPFLEPEEAIWEALSVMGLY |
| 120 | CCV1000-2-1 | TSP50 | YRSCGFSYEQDPTLRVCSMEGTWYLVGLVS |
| 121 | CCV1000-2-2 | EpCAM/Survivin | VRTYWIIIELKHKARTAKKVRRAIEQLAAM |
| 122 | CCV1000-2-4 | TSP50/Spag9 | LPSTTMETQFPVSEGKLGFSFVRITALMVS |
| 123 | CCV1000-2-5 | Survivin/Mage-A8 | MGAPTLPPAWQPFLKPEEAIWEALSVMGLY |
| 124 | CCV1000-2-6 | CAGE1/Survivin | LASKMHSLLALMVGLKDHRISTFKNWPFLE |
| 125 | CCV1000-2-7 | CAGE1/Spag9 | PKSMTMMPALFKENRLDSFTVCNSHVLCIA |
| 126 | CCV1000-2-8 | FBXO39 | KFMNPYNAVLTKKFQKVNFFFERIMKYERL |
| 127 | CCV1000-2-9 | Spag9/FBXO39 | AQKMSSLLPTMWLGAFKKTMSTFHNLVSLN |
| 128 | CCV1000-3-1 | TSP50 | GFSYEQDPTLRDPEAVCSMEGTWYLVGLVS |
| 129 | CCV1000-3-7 | CAGE1/Spag9 | PKSMTMMPALFKENRGNILDSFTVCNSHVL |
| 130 | CCV1000-5-1 | TSP50 | PSTTMETQFPVSEGKSRYRAQRFWSWVGQA |
| 131 | CCV1000-5-3 | EpCAM /Mage-A8 | YVDEKAPEFSMQGLKDEKVAELVRFLLRKY |
| 132 | CCV1000-5-4 | TSP50/Spag9 | RSCGFSYEQDPTLRDGTGKLGFSFVRITAL |
| 133 | CCV1000-5-5 | Mage-A8/Mage-A6 | SRAPEEAIWEALSVMQYFVQENYLEYRQVP |
| 134 | CCV1000-5-7 | CAGE1/Spag9 | PKSMTMMPALFKENRSGAVMSERVSGLAGS |
| 135 | CCV1000-S-1 | SPAG9/FBXO39 | SGAVMSERVSGLAGSRNSIRSSFISSLSFF |
| 136 | CCV1000-S-2 | CAGE11/MAGE-A8 | NIENYSTNALIQPVDEKVAELVRFLLRKYQ |
| 137 | CCV1000-S-3 | CAGE11/MAGE-A6 | RQFETVCKFHWVEAFKLLTQYFVQENYLEY |
| 138 | CCV1000-S-5 | MAGE-A8/MAGE-A3 | EFLWGPRALAETSYVKLLTQHFVQENYLEY |
| 139 | CCV1000-S-6 | MAGE-A8/EpCAM | ASSSSTLIMGTLEEVQTLIYYVDEKAPEFS |
| 140 | CCV1000-S-7 | TSP50/MAGE-A3 | SRTLLLALPLPLSLLIGHLYIFATCLGLSY |
| 141 | CCV1000-S-9 | LEMD1/MAGE-A6 | FIIVVFVYLTVENKSIGHVYIFATCLGLSY |
| 142 | CCV1000-S-17 | EPCAM | LLAAATATFAAAQEEQTLIYYVDEKAPEFS |

**Table 21b - Selected Colorectal Cancer Vaccine peptides for PolyPEPI1015 composition**

| SEQID | TREOSID | Source Antigen | Peptide (30mer) | HLAI* (CD8) | HLAII** (CD4) |
|---|---|---|---|---|---|
| 130 | CCV1000-5-1 | TSP50 | PSTTMETQFPVSEGKSRYRAQRFWSWVGQA | 53% | 53% |
| 121 | CCV1000-2-2 | EpCAM/Survivin | VRTYWIIIELKHKARTAKKVRRAIEQLAAM | 57% | 98% |
| 131 | CCV1000-5-3 | EpCAM /Mage-A8 | YVDEKAPEFSMQGLKDEKVAELVRFLLRKY | 43% | 72% |
| 132 | CCV1000-5-4 | TSP50/Spag9 | RSCGFSYEQDPTLRDGTGKLGFSFVRITAL | 67% | 82% |
| 133 | CCV1000-5-5 | Mage-A8/Mage-A6 | SRAPEEAIWEALSVMQYFVQENYLEYRQVP | 45% | 76% |
| 124 | CCV1000-2-6 | CAGE1/Survivin | LASKMHSLLALMVGLKDHRISTFKNWPFLE | 58% | 95% |
| 134 | CCV1000-5-7 | CAGE1/Spag9 | PKSMTMMPALFKENRSGAVMSERVSGLAGS | 57% | 57% |
| 126 | CCV1000-2-8 | FBXO39 | KFMNPYNAVLTKKFQKVNFFFERIMKYERL | 90% | 98% |
| 127 | CCV1000-2-9 | Spag9/FBXO39 | AQKMSSLLPTMWLGAFKKTMSTFHNLVSLN | 67% | 66% |
| | | | PolyPEPI1015 (9 peptide together) | 100% | 99% |

| | | | | | |
|---|---|---|---|---|---|
| * Percentage of individuals having CD8+ T cell specific PEPI3+ within the Model Population (n=433). **Percentage of individuals having CD4+ T cell specific PEPI4+ within the Model Population (n=433). | | | | | |

### Characterization of PolyPEPI1015 colorectal cancer vaccine

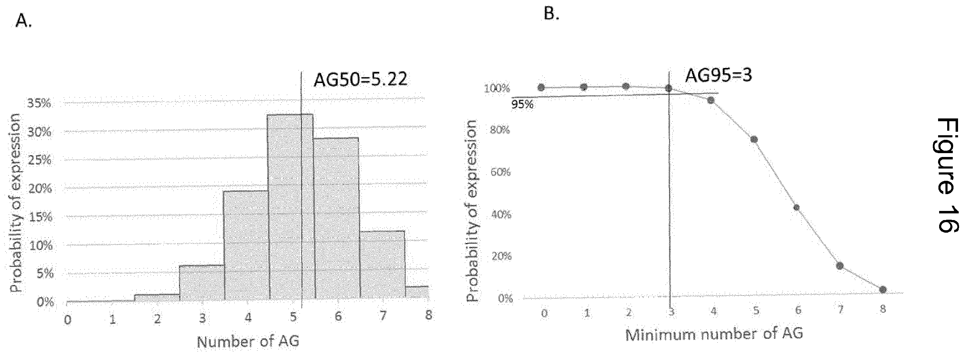

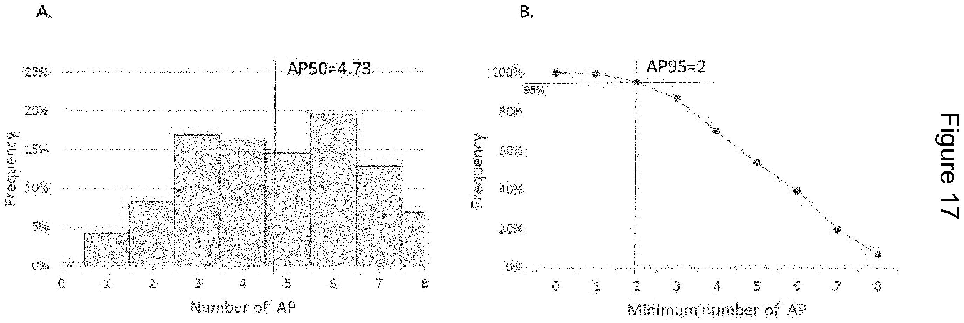

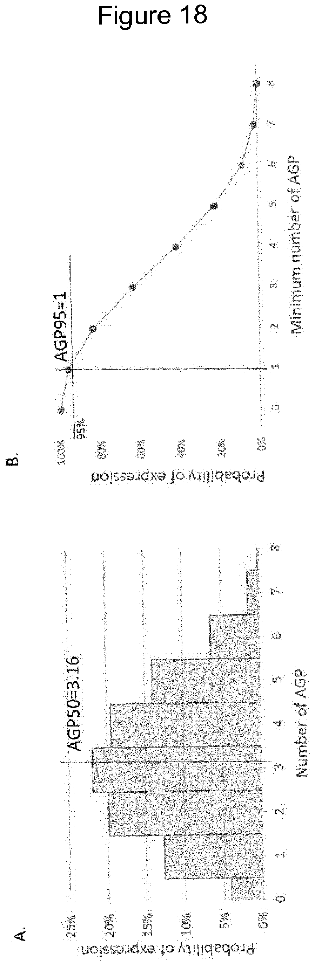

Tumor heterogeneity: The PolyPEPI1015 composition targets 8 different CTAs (Fig 15). Based on the antigen expression rates for these 8 CTAs, AG50 = 5.22 and AG95 = 3 **FIG. 16**. Patient heterogeneity: the AP50=4.73 and AP95 = 2 (AP95=2) (FIG. 17). Both tumor and patient heterogeneity: AGP50 = 3.16 and AGP95 = 1 (Model Population) (FIG. 18).

### Example 18 - Comparison of colorectal cancer vaccine peptides with competing colorectal cancer vaccines

We used the *in silico* clinical trial model described above to determine T cell responder rate of state of art and currently developed CRC peptide vaccines and compared to and compared to that of polyPEPI1015 (Table 22). Our PEPI3+ test demonstrate that competing vaccines can induce immune responses against one tumor antigen in a fraction of subjects (2% - 77%). However, the multi-antigen (multi-PEPI) response determination for the 2 competitor multi-antigen vaccines resulted in no or 2% responders. *% of responders are the ratio of subjects from the Model population with 1≥PEPI3+ for HLAI (CD8+ T cell responses) in case of 1, or for 2, 3, 4 or 5 antigens of the vaccine compositions. Since multi-PEPI responses correlate with clinical responses induced by tumor vaccines, it is unlikely that any of the competing vaccines will demonstrate clinical benefit in 98% of patients. In contrast, we predicted multi-PEPI responses in 95% of subjects suggesting the likelihood for clinical benefit in the majority of patients.

**Table 22 Predicted immune response rates of polyPEPI1015 and competing colorectal cancer vaccines**

| *Colorectal Cancer Vaccines* | *Sponsor* | % of CD8+ T cell responders in 433 subjects* | | | | | |
|---|---|---|---|---|---|---|---|
| | | Vaccine antigens (Ags) | % responders against multiple Ags | | | | |
| | | | 1 Ag | 2 Ags | 3 Ags | 4Ags | 5 Ags |
| Stimuvax^{®}(L-BLP25) Peptide Vaccine | Johannes Gutenberg University Mainz | 1 | 6% | - | - | - | - |
| WT1 Multipeptide Vaccine | Shinshu University, Japan | 1 | 79% | - | - | - | - |
| Multiepitope Peptide Cocktail Vaccine | Kinki University | 7 | 5% | 2% | 0% | 0% | 0% |
| p53 Synthetic Long Peptide Vaccine | Leiden University Medical Center | 1 | 77% | - | - | - | - |
| HER-2 B Cell Peptide Vaccine | Ohio State University Comprehensive Cancer Center | 1 | 18% | - | - | - | - |
| NY-ESO-1 peptide pulsed dendritic cell vaccine | Jonsson Comprehensive Cancer Center | 1 | 0% | - | - | - | - |
| OCV-C02 | Otsuka Pharmaceutical Co., Ltd. | 2 | 2% | 0% | - | - | - |
| **PolyPEPI1015** | **Treos Bio** | **8** | **100%** | **95%** | **87%** | **70%** | **54%** |

### Additional Sequences

### SEQ ID NO: 41

### DQVNIDYLMNRPQNL

**42**
   MMAYSDTTMMSDDID
**43**
   MFTSSRMSSFNRHMK
**44**
   LQKYALGFQHALSPS
**45**
   AQKMSSLLPTMWLGA
**46**
   GNILDSFTVCNSHVL
**47**
   NMAFVTSGELVRHRR
**48**
   LERLAYLHARLRELL
**49**
   SGAVMSERVSGLAGS
**50**
   HTRFTQSGTMKIHIL
**51**
   FSSSGTTSFKCFAPF
**52**
   QLEFMFQEALKLKVA
**53**
   CSGSSYFVLANGHIL
**54**
   RHSQTLKAMVQAWPF
**55**
   YSCDSRSLFESSAKI
**56**
   TAKKVRRAIEQLAAM
**57**
   SPTAMDAIFGSLSDE
**58**
   MASFRKLTLSEKVPP
**59**
   SSISVYYTLWSQFDE
**60**
   PGKPSAFEPATEMQK
**61**
   RSCGFSYEQDPTLRD
**62**
   VRTYWIIIELKHKAR
**63**
   PSTTMETQFPVSEGK
**64**
   GTGKLGFSFVRITAL
**65**
   AQKMSSLLPTMWLGA
**66**
   LASKMHSLLALMVGL
**67**
   KFMNPYNAVLTKKFQ
**68**
   PKSMTMMPALFKENR
**69**
   YVDEKAPEFSMQGLK
**70**
   FKKTMSTFHNLVSLN
**71**
   TAKKVRRAIEQLAAM
**72**
   SGAVMSERVSGLAGS
**73**
   SRYRAQRFWSWVGQA
**74**
   KVNFFFERIMKYERL
**75**
   KDHRISTFKNWPFLE
**76**
   SRAPEEAIWEALSVM
**77**
   DEKVAELVRFLLRKY
**78**
   QYFVQENYLEYRQVP
**79**
   EFLWGPRALAETSYV
**80**
   IGHVYIFATCLGLSY
**143**
   SPAG9, UniProtID:060271, Len:1321 
MELEDGVVYQEEPGGSGAVMSERVSGLAGSIYREFERLIGRYDEEVVKELMPLVVAVLEN
LDSVFAQDQEHQVELELLRDDNEQLITQYEREKALRKHAEEKFIEFEDSQEQEKKDLQTR
VESLESQTRQLELKAKNYADQISRLEEREAELKKEYNALHQRHTEMIHNYMEHLERTKLH
QLSGSDQLESTAHSRIRKERPISLGIFPLPAGDGLLTPDAQKGGETPGSEQWKFQELSQP
RSHTSLKVSNSPEPQKAVEQEDELSDVSQGGSKATTPASTANSDVATIPTDTPLKEENEG
FVKVTDAPNKSEISKHIEVQVAQETRNVSTGSAENEEKSEVQAIIESTPELDMDKDLSGY
KGSSTPTKGIENKAFDRNTESLFEELSSAGSGLIGDVDEGADLLGMGREVENLILENTQL
LETKNALNIVKNDLIAKVDELTCEKDVLQGELEAVKQAKLKLEEKNRELEEELRKARAEA
EDARQKAKDDDDSDIPTAQRKRFTRVEMARVLMERNQYKERLMELQEAVRWTEMIRASRE
NPAMQEKKRSSIWQFFSRLFSSSSNTTKKPEPPVNLKYNAPTSHVTPSVKKRSSTLSQLP
GDKSKAFDFLSEETEASLASRREQKREQYRQVKAHVQKEDGRVQAFGWSLPQKYKQVTNG
QGENKMKNLPVPVYLRPLDEKDTSMKLWCAVGVNLSGGKTRDGGSVVGASVFYKDVAGLD
 
TEGSKQRSASQSSLDKLDQELKEQQKELKNQEELSSLVWICTSTHSATKVLIIDAVQPGN
ILDSFTVCNSHVLCIASVPGARETDYPAGEDLSESGQVDKASLCGSMTSNSSAETDSLLG
GITVVGCSAEGVTGAATSPSTNGASPVMDKPPEMEAENSEVDENVPTAEEATEATEGNAG
SAEDTVDISQTGVYTEHVFTDPLGVQIPEDLSPVYQSSNDSDAYKDQISVLPNEQDLVRE
EAQKMSSLLPTMWLGAQNGCLYVHSSVAQWRKCLHSIKLKDSILSIVHVKGIVLVALADG
TLAIFHRGVDGQWDLSNYHLLDLGRPHHSIRCMTVVHDKVWCGYRNKIYVVQPKAMKIEK
SFDAHPRKESQVRQLAWVGDGVWVSIRLDSTLRLYHAHTYQHLQDVDIEPYVSKMLGTGK
LGFSFVRITALMVSCNRLWVGTGNGVIISIPLTETNKTSGVPGNRPGSVIRVYGDENSDK
VTPGTFIPYCSMAHAQLCFHGHRDAVKFFVAVPGQVISPQSSSSGTDLTGDKAGPSAQEP
GSQTPLKSMLVISGGEGYIDFRMGDEGGESELLGEDLPLEPSVTKAERSHLIVWQVMYGN
E
**144**
   AKAP-4, UniProtID:Q5JQC9, Len:854 
MMAYSDTTMMSDDIDWLRSHRGVCKVDLYNPEGQQDQDRKVICFVDVSTLNVEDKDYKDA
ASSSSEGNLNLGSLEEKEIIVIKDTEKKDQSKTEGSVCLFKQAPSDPVSVLNWLLSDLQK
YALGFQHALSPSTSTCKHKVGDTEGEYHRASSENCYSVYADQVNIDYLMNRPQNLRLEMT
AAKNTNNNQSPSAPPAKPPSTQRAVISPDGECSIDDLSFYVNRLSSLVIQMAHKEIKEKL
EGKSKCLHHSICPSPGNKERISPRTPASKIASEMAYEAVELTAAEMRGTGEESREGGQKS
FLYSELSNKSKSGDKQMSQRESKEFADSISKGLMVYANQVASDMMVSLMKTLKVHSSGKP
IPASVVLKRVLLRHTKEIVSDLIDSCMKNLHNITGVLMTDSDFVSAVKRNLFNQWKQNAT
DIMEAMLKRLVSALIGEEKETKSQSLSYASLKAGSHDPKCRNQSLEFSTMKAEMKERDKG
KMKSDPCKSLTSAEKVGEHILKEGLTIWNQKQGNSCKVATKACSNKDEKGEKINASTDSL
AKDLIVSALKLIQYHLTQQTKGKDTCEEDCPGSTMGYMAQSTQYEKCGGGQSAKALSVKQ
LESHRAPGPSTCQKENQHLDSQKMDMSNIVLMLIQKLLNENPFKCEDPCEGENKCSEPRA
SKAASMSNRSDKAEEQCQEHQELDCTSGMKQANGQFIDKLVESVMKLCLIMAKYSNDGAA
LAELEEQAASANKPNFRGTRCIHSGAMPQNYQDSLGHEVIVNNQCSTNSLQKQLQAVLQW
IAASQFNVPMLYFMGDKDGQLEKLPQVSAKAAEKGYSVGGLLQEVMKFAKERQPDEAVGK
VARKQLLDWLLANL
**145**
   BORIS, UniProtID:Q8NI51, Len:663 
MAATEISVLSEQFTKIKELELMPEKGLKEEEKDGVCREKDHRSPSELEAERTSGAFQDSV
LEEEVELVLAPSEESEKYILTLQTVHFTSEAVELQDMSLLSIQQQEGVQVVVQQPGPGLL
WLEEGPRQSLQQCVAISIQQELYSPQEMEVLQFHALEENVMVASEDSKLAVSLAETTGLI
KLEEEQEKNQLLAERTKEQLFFVETMSGDERSDEIVLTVSNSNVEEQEDQPTAGQADAEK
AKSTKNQRKTKGAKGTFHCDVCMFTSSRMSSFNRHMKTHTSEKPHLCHLCLKTFRTVTLL
RNHVNTHTGTRPYKCNDCNMAFVTSGELVRHRRYKHTHEKPFKCSMCKYASVEASKLKRH
VRSHTGERPFQCCQCSYASRDTYKLKRHMRTHSGEKPYECHICHTRFTQSGTMKIHILQK
HGENVPKYQCPHCATIIARKSDLRVHMRNLHAYSAAELKCRYCSAVFHERYALIQHQKTH
KNEKRFKCKHCSYACKQERHMTAHIRTHTGEKPFTCLSCNKCFRQKQLLNAHFRKYHDAN
 
FIPTVYKCSKCGKGFSRWINLHRHSEKCGSGEAKSAASGKGRRTRKRKQTILKEATKGQK
EAAKGWKEAANGDEAAAEEASTTKGEQFPGEMFPVACRETTARVKEEVDEGVTCEMLLNT
MDK
**146**
   NY-SAR-35, UniProtID:Q8N0W7, Len:255 
MSSHRRKAKGRNRRSHRAMRVAHLELATYELAATESNPESSHPGYEAAMADRPQPGWRES
LKMRVSKPFGMLMLSIWILLFVCYYLSYYLCSGSSYFVLANGHILPNSENAHGQSLEEDS
ALEALLNFFFPTTCNLRENQVAKPCNELQDLSESECLRHKCCFSSSGTTSFKCFAPFRDV
PKQMMQMFGLGAISLILVCLPIYCRSLFWRSEPADDLQRQDNRVVTGLKKQRRKRKRKSE
MLQKAARGREEHGDE
**147**
   NY-BR-1, UniProtID:Q9BXX3, Len:1341 
MTKRKKTINLNIQDAQKRTALHWACVNGHEEVVTFLVDRKCQLDVLDGEHRTPLMKALQC
HQEACANILIDSGADINLVDVYGNTALHYAVYSEILSVVAKLLSHGAVIEVHNKASLTPL
LLSITKRSEQIVEFLLIKNANANAVNKYKCTALMLAVCHGSSEIVGMLLQQNVDVFAADI
CGVTAEHYAVTCGFHHIHEQIMEYIRKLSKNHQNTNPEGTSAGTPDEAAPLAERTPDTAE
SLVEKTPDEAAPLVERTPDTAESLVEKTPDEAASLVEGTSDKIQCLEKATSGKFEQSAEE
TPREITSPAKETSEKFTWPAKGRPRKIAWEKKEDTPREIMSPAKETSEKFTWAAKGRPRK
IAWEKKETPVKTGCVARVTSNKTKVLEKGRSKMIACPTKESSTKASANDQRFPSESKQEE
DEEYSCDSRSLFESSAKIQVCIPESIYQKVMEINREVEEPPKKPSAFKPAIEMQNSVPNK
AFELKNEQTLRADPMFPPESKQKDYEENSWDSESLCETVSQKDVCLPKATHQKEIDKING
KLEESPNKDGLLKATCGMKVSIPTKALELKDMQTFKAEPPGKPSAFEPATEMQKSVPNKA
LELKNEQTLRADEILPSESKQKDYEENSWDTESLCETVSQKDVCLPKAAHQKEIDKINGK
LEGSPVKDGLLKANCGMKVSIPTKALELMDMQTFKAEPPEKPSAFEPAIEMQKSVPNKAL
ELKNEQTLRADEILPSESKQKDYEESSWDSESLCETVSQKDVCLPKATHQKEIDKINGKL
EESPDNDGFLKAPCRMKVSIPTKALELMDMQTFKAEPPEKPSAFEPAIEMQKSVPNKALE
LKNEQTLRADQMFPSESKQKKVEENSWDSESLRETVSQKDVCVPKATHQKEMDKISGKLE
DSTSLSKILDTVHSCERARELQKDHCEQRTGKMEQMKKKFCVLKKKLSEAKEIKSQLENQ
KVKWEQELCSVRLTLNQEEEKRRNADILNEKIREELGRIEEQHRKELEVKQQLEQALRIQ
DIELKSVESNLNQVSHTHENENYLLHENCMLKKEIAMLKLEIATLKHQYQEKENKYFEDI
KILKEKNAELQMTLKLKEESLTKRASQYSGQLKVLIAENTMLTSKLKEKQDKEILEAEIE
SHHPRLASAVQDHDQIVTSRKSQEPAFHIAGDACLQRKMNVDVSSTIYNNEVLHQPLSEA
QRKSKSLKINLNYAGDALRENTLVSEHAQRDQRETQCQMKEAEHMYQNEQDNVNKHTEQQ
ESLDQKLFQLQSKNMWLQQQLVHAHKKADNKSKITIDIHFLERKMQHHLLKEKNEEIFNY
NNHLKNRIYQYEKEKAETENS
**148**
   SURVIVIN, UniProtID:O15392, Len:142 
MGAPTLPPAWQPFLKDHRISTFKNWPFLEGCACTPERMAEAGFIHCPTENEPDLAQCFFC
FKELEGWEPDDDPIEEHKKHSSGCAFLSVKKQFEELTLGEFLKLDRERAKNKIAKETNNK
KKEFEETAKKVRRAIEQLAAMD
**149**
   MAGE-A11, UniProtID:P43364, Len:429 
METQFRRGGLGCSPASIKRKKKREDSGDFGLQVSTMFSEDDFQSTERAPYGPQLQWSQDL
PRVQVFREQANLEDRSPRRTQRITGGEQVLWGPITQIFPTVRPADLTRVIMPLEQRSQHC
KPEEGLQAQEEDLGLVGAQALQAEEQEAAFFSSTLNVGTLEELPAAESPSPPQSPQEESF
SPTAMDAIFGSLSDEGSGSQEKEGPSTSPDLIDPESFSQDILHDKIIDLVHLLLRKYRVK
GLITKAEMLGSVIKNYEDYFPEIFREASVCMQLLFGIDVKEVDPTSHSYVLVTSLNLSYD
GIQCNEQSMPKSGLLIIVLGVIFMEGNCIPEEVMWEVLSIMGVYAGREHFLFGEPKRLLT
QNWVQEKYLVYRQVPGTDPACYEFLWGPRAHAETSKMKVLEYIANANGRDPTSYPSLYED
ALREEGEGV
**150**
   PRAME, UniProtID:P78395, Len:509 
MERRRLWGSIQSRYISMSVWTSPRRLVELAGQSLLKDEALAIAALELLPRELFPPLFMAA
FDGRHSQTLKAMVQAWPFTCLPLGVLMKGQHLHLETFKAVLDGLDVLLAQEVRPRRWKLQ
VLDLRKNSHQDFWTVWSGNRASLYSFPEPEAAQPMTKKRKVDGLSTEAEQPFIPVEVLVD
LFLKEGACDELFSYLIEKVKRKKNVLRLCCKKLKIFAMPMQDIKMILKMVQLDSIEDLEV
TCTWKLPTLAKFSPYLGQMINLRRLLLSHIHASSYISPEKEEQYIAQFTSQFLSLQCLQA
LYVDSLFFLRGRLDQLLRHVMNPLETLSITNCRLSEGDVMHLSQSPSVSQLSVLSLSGVM
LTDVSPEPLQALLERASATLQDLVFDECGITDDQLLALLPSLSHCSQLTTLSFYGNSISI
SALQSLLQHLIGLSNLTHVLYPVPLESYEDIHGTLHLERLAYLHARLRELLCELGRPSMV
WLSANPCPHCGDRTFYDPEPILCPCFMPN
**151**
   MAGE-A9, UniProtID:P43362 , Len:315 
MSLEQRSPHCKPDEDLEAQGEDLGLMGAQEPTGEEEETTSSSDSKEEEVSAAGSSSPPQS
PQGGASSSISVYYTLWSQFDEGSSSQEEEEPSSSVDPAQLEFMFQEALKLKVAELVHFLL
HKYRVKEPVTKAEMLESVIKNYKRYFPVIFGKASEFMQVIFGTDVKEVDPAGHSYILVTA
LGLSCDSMLGDGHSMPKAALLIIVLGVILTKDNCAPEEVIWEALSVMGVYVGKEHMFYGE
PRKLLTQDWVQENYLEYRQVPGSDPAHYEFLWGSKAHAETSYEKVINYLVMLNAREPICY
PSLYEEVLGEEQEGV
**152**
   HOM-TES-85, UniProtID:Q9P127, Len:313 
MASFRKLTLSEKVPPNHPSRKKVNFLDMSLDDIIIYKELEGTNAEEEKNKRQNHSKKESP
SRQQSKAHRHRHRRGYSRCRSNSEEGNHDKKPSQKPSGFKSGQHPLNGQPLIEQEKCSDN
YEAQAEKNQGQSEGNQHQSEGNPDKSEESQGQPEENHHSERSRNHLERSLSQSDRSQGQL
 
KRHHPQYERSHGQYKRSHGQSERSHGHSERSHGHSERSHGHSERSHGHSKRSRSQGDLVD
TQSDLIATQRDLIATQKDLIATQRDLIATQRDLIVTQRDLVATERDLINQSGRSHGQSER
HQRYSTGKNTITT
**153**
   TSP50, UniProtID:Q9UI38, Len:385 
MGRWCQTVARGQRPRTSAPSRAGALLLLLLLLRSAGCWGAGEAPGALSTADPADQSVQCV
PKATCPSSRPRLLWQTPTTQTLPSTTMETQFPVSEGKVDPYRSCGFSYEQDPTLRDPEAV
ARRWPWMVSVRANGTHICAGTIIASQWVLTVAHCLIWRDVIYSVRVGSPWIDQMTQTASD
VPVLQVIMHSRYRAQRFWSWVGQANDIGLLKLKQELKYSNYVRPICLPGTDYVLKDHSRC
TVTGWGLSKADGMWPQFRTIQEKEVIILNNKECDNFYHNFTKIPTLVQIIKSQMMCAEDT
HREKFCYELTGEPLVCSMEGTWYLVGLVSWGAGCQKSEAPPIYLQVSSYQHWIWDCLNGQ
ALALPAPSRTLLLALPLPLSLLAAL
**154**
   EpCAM, UniProtID:P16422, Len:314 
MAPPQVLAFGLLLAAATATFAAAQEECVCENYKLAVNCFVNNNRQCQCTSVGAQNTVICS
KLAAKCLVMKAEMNGSKLGRRAKPEGALQNNDGLYDPDCDESGLFKAKQCNGTSMCWCVN
TAGVRRTDKDTEITCSERVRTYWIIIELKHKAREKPYDSKSLRTALQKEITTRYQLDPKF
ITSILYENNVITIDLVQNSSQKTQNDVDIADVAYYFEKDVKGESLFHSKKMDLTVNGEQL
DLDPGQTLIYYVDEKAPEFSMQGLKAGVIAVIVVVVIAVVAGIVVLVISRKKRMAKYEKA
EIKEMGEMHRELNA
**155**
   CAGE1, UniProtID:Q8CT20, Len:777 
MNKDYQKFWSSPSDPVHFEVDTSHEKVESMSESDTMNVSNLSQGVMLSHSPICMETTGTT
CDLPQNEIKNFERENEYESTLCEDAYGTLDNLLNDNNIENYSTNALIQPVDTISISSLRQ
FETVCKFHWVEAFDDEMTEKPEFQSQVYNYAKDNNIKQDSFKEENPMETSVSANTDQLGN
EYFRQPPPRSPPLIHCSGEMLKFTEKSLAKSIAKESALNPSQPPSFLCKTAVPSKEIQNY
GEIPEMSVSYEKEVTAEGVERPEIVSTWSSAGISWRSEACRENCEMPDWEQSAESLQPVQ
EDMALNEVLQKLKHTNRKQEVRIQELQCSNLYLEKRVKELQMKITKQQVFIDVINKLKEN
VEELIEDKYKIILEKNDTKKTLQNLEEVLANTQKHLQESRNDKEMLQLQFKKIKANYVCL
QERYMTEMQQKNKSVSQYLEMDKTLSKKEEEVERLQQLKKELEKATASALDLLKREKEAQ
EQEFLSLQEEFQKLEKENLEERQKLKSRLEKLLTQVRNLQFMSENERTKNIKLQQQINEV
KNENAKLKQQVARSEEQNYVPKFETAQLKDQLEEVLKSDITKDTKTTHSNLLPDCSPCEE
RLNPADIKRASQLASKMHSLLALMVGLLTCQDIINSDAEHFKESEKVSDIMLQKLKSLHL
KKKTLDKEVIDCDSDEAKSIRDVPTLLGAKLDKYHSLNEELDFLVTSYEEIIECADQRLA
ISHSQIAHLEERNKHLEDLIRKPREKARKPRSKSLENHPKSMTMMPALFKENRNDLD
**156**
   FBXO39, UniProtID:Q8N4B4, Len:442 
MDEESELIQPQDQSCWAFLPDLCLCRVFWWLGDRDSRAALVCRKWNQMMYSAELWRYRT
ITFSGRPSRVHASEVESAVWYVKKFGRYLEHLEVKFMNPYNAVLTKKFQVTMRGLLSCLS
KSNNRLKSLSIQYLELDRLVWRNSIRSSFISSLSFFLKKMGKRLDYLNLKGARLTVEQGC
QILDSLSYMRNENVISELNIEDYFSHHLAVYNSPQFKKTMSTFHNLVSLNLNYNCISDEL
LENLCENASTLRTINIKCHVHDPHGQVIWGMSWAKLARQATNLKVNFFFERIMKYERLAR
ILLQEIPIRSISLRSCYFSDPDCSMRPTLIDLLPTFRHTLQKLTCEFNNNHESLDEELHL
LIISCRKLFYFKIWAFLDVSFVERILKSQKERQCALRVFKARIYTNRYETNEEDKTLQEI
YRKYRKLIESELSYFVIVYSVM
157
   MAGE-A8, UniProtID:P43361, Len:318 
MLLGQKSQRYKAEEGLQAQGEAPGLMDVQIPTAEEQKAASSSSTLIMGTLEEVTDSGSPS
PPQSPEGASSSLTVTDSTLWSQSDEGSSSNEEEGPSTSPDPAHLESLFREALDEKVAELV
RFLLRKYQIKEPVTKAEMLESVIKNYKNHFPDIFSKASECMQVIFGIDVKEVDPAGHSYI
LVTCLGLSYDGLLGDDQSTPKTGLLIIVLGMILMEGSRAPEEAIWEALSVMGLYDGREHS
VYWKLRKLLTQEWVQENYLEYRQAPGSDPVRYEFLWGPRALAETSYVKVLEHVVRVNARV
RISYPSLHEEALGEEKGV
**158**
   MAGE-A6, UniProtID:P43360, Len:314

MPLEQRSQHCKPEEGLEARGEALGLVGAQAPATEEQEAASSSSTLVEVTLGEVPAAESPD
PPQSPQGASSLPTTMNYPLWSQSYEDSSNQEEEGPSTFPDLESEFQAALSRKVAKLVHFL
LLKYRAREPVTKAEMLGSVVGNWQYFFPVIFSKASDSLQLVFGIELMEVDPIGHVYIFAT
CLGLSYDGLLGDNQIMPKTGFLIIILAIIAKEGDCAPEEKIWEELSVLEVFEGREDSIFG
DPKKLLTQYFVQENYLEYRQVPGSDPACYEFLWGPRALIETSYVKVLHHMVKISGGPRIS
YPLLHEWALREGEE

### References

¹ Bagarazzi et al. Immunotherapy against HPV16/18 generates potent TH1 and cytotoxic cellular immune responses. Science Translational Medicine. 2012; 4(155):155ra138.
² Gudmundsdotter et al. Amplified antigen-specific immune responses in HIV-1 infected individuals in a double blind DNA immunization and therapy interruption trial. Vaccine. 2011; 29(33):5558-66.
³ Bioley et al. HLA class I - associated immunodominance affects CTL responsiveness to an ESO recombinant protein tumor antigen vaccine. Clin Cancer Res. 2009; 15(1):299-306.
⁴ Valmori et al. Vaccination with NY-ESO-1 protein and CpG in Montanide induces integrated antibody/Th1 responses and CD8 T cells through cross-priming. Proceedings of the National Academy of Sciences of the United States of America. 2007; 104(21):8947-52.
⁵ Yuan et al. Integrated NY-ESO-1 antibody and CD8+ T-cell responses correlate with clinical benefit in advanced melanoma patients treated with ipilimumab.Proc Natl Acad Sci USA. 2011;108(40):16723-16728.
⁶ Kakimi et al. A phase I study of vaccination with NY-ESO-1f peptide mixed with Picibanil OK-432 and Montanide ISA-51 in patients with cancers expressing the NY-ESO-1 antigen.Int J Cancer. 2011;129(12):2836-46.
⁷ Wada et al. Vaccination with NY-ESO-1 overlapping peptides mixed with Picibanil OK-432 and montanide ISA-51 in patients with cancers expressing the NY-ESO-1 antigen. J Immunother. 2014;37(2):84-92.
⁸ Welters et al. Induction of tumor-specific CD4+ and CD8+ T-cell immunity in cervical cancer patients by a human papillomavirus type 16 E6 and E7 long peptides vaccine. Clin. Cancer Res. 2008; 14(1):178-87.
⁹ Kenter et al. Vaccination against HPV-16 oncoproteins for vulvar intraepithelial neoplasia. N Engl J Med. 2009; 361(19):1838-47.
¹⁰ Welters et al. Success or failure of vaccination for HPV16-positive vulvar lesions correlates with kinetics and phenotype of induced T-cell responses. PNAS. 2010; 107(26):11895-9.
¹¹ http://www.ncbi.nlm.nih.gov/projects/gv/mhc/main.fcgi?cmd=initThe MHC database, NCBI (Accessed Mar 7, 2016).
¹² Karkada et al. Therapeutic vaccines and cancer: focus on DPX-0907. Biologics. 2014;8:27-38.
¹³ Butts et al. Randomized phase IIB trial of BLP25 liposome vaccine in stage IIIB and IV non-small-cell lung cancer. J Clin Oncol. 2005;23(27):6674-81.
¹⁴ Yuan et al.Safety and immunogenicity of a human and mouse gp100 DNA vaccine in a phase I trial of patients with melanoma.Cancer Immun. 2009;9:5.
¹⁵ Kovjazin et al. ImMucin: a novel therapeutic vaccine with promiscuous MHC binding for the treatment of MUC1-expressing tumors. Vaccine. 2011;29(29-30):4676-86.
¹⁶Cathcart et al. Amultivalent bcr-abl fusion peptide vaccination trial in patients with chronic myeloid leukemia.Blood. 2004;103:1037-1042.
¹⁷ Chapuis et al. Transferred WT1-reactive CD8+ T cells can mediate antileukemic activity and persist in post-transplant patients. Sci Transl Med. 2013;5(174):174ra27.
¹⁸ Keilholz et al. A clinical and immunologic phase 2 trial of Wilms tumor gene product 1 (WT1) peptide vaccination in patients with AML and MDS. Blood; 2009; 113(26):6541-8.
¹⁹ Walter et al. Multipeptide immune response to cancer vaccine IMA901 after single-dose cyclophosphamide associates with longer patient survival. Nat Med. 2012;18(8):1254-61.
²⁰ Phuphanich et al. Phase I trial of a multi-epitope-pulsed dendritic cell vaccine for patients with newly diagnosed glioblastoma. Cancer Immunol Immunother. 2013;62(1):125-35.
²¹ Kantoff et al. Overall survival analysis of a phase II randomized controlled trial of a Poxviral-based PSA-targeted immunotherapy in metastatic castration-resistant prostate cancer. J Clin Oncol. 2010;28(7):1099-105.
²² Tagawa et al. Phase I study of intranodal delivery of a plasmid DNA vaccine for patients with Stage IV melanoma. Cancer. 2003;98(1):144-54.
²³ Slingluff et al. Randomized multicenter trial of the effects of melanoma-associated helper peptides and cyclophosphamide on the immunogenicity of a multipeptide melanoma vaccine.J Clin Oncol. 2011;29(21):2924-32.
²⁴ Kaida et al. Phase 1 trial of Wilms tumor 1 (WT1) peptide vaccine and gemcitabine combination therapy in patients with advanced pancreatic or biliary tract cancer. J Immunother. 2011;34(1):92-9.
²⁵ Fenoglio et al. A multi-peptide, dual-adjuvant telomerase vaccine (GX301) is highly immunogenic in patients with prostate and renal cancer. Cancer Immunol Immunother; 2013; 62:1041-1052.
²⁶ Krug et al. WT1 peptide vaccinations induce CD4 and CD8 T cell immune responses in patients with mesothelioma and non-small cell lung cancer. Cancer Immunol Immunother; 2010; 59(10):1467-79.
²⁷ Slingluff et al. Clinical and immunologic results of a randomized phase II trial of vaccination using four melanoma peptides either administered in granulocyte-macrophage colony-stimulating factor in adjuvant or pulsed on dendritic cells. J Clin Oncol; 2003; 21(21):4016-26.
²⁸ Hodi et al. Improved survival with ipilimumab in patients with metastatic melanoma. N Engl J Med; 2010;363(8):711-23.
²⁹ Carmon et al. Phase I/II study exploring ImMucin, a pan-major histocompatibility complex, anti-MUC1 signal peptide vaccine, in multiple myeloma patients. Br J Hematol. 2014; 169(1):44-56.
³⁰ http://www.merckgroup.com/en/media/extNewsDetail.html?newsId=EB4A46A2AC4A52E7C 1257AD9001F3186&newsType=1(Accessed Mar 28, 2016)
³¹ Trimble et al. Safety, efficacy, and immunogenicity of VGX-3100, a therapeutic synthetic DNA vaccine targeting human papillomavirus 16 and 18 E6 and E7 proteins for cervical intraepithelial neoplasia 2/3: a randomised, double-blind, placebo-controlled phase 2b trial. Lancet. 2015;386(10008):2078-88.
³² Cusi et al. Phase I trial of thymidylate synthase poly epitope peptide (TSPP) vaccine in advanced cancer patients. Cancer Immunol Immunother; 2015; 64:1159-1173.
³³ Asahara et al. Phase I/II clinical trial using HLA-A24-restricted peptide vaccine derived from KIF20A for patients with advanced pancreatic cancer. J Transl Med; 2013;11:291.
³⁴ Yoshitake et al. Phase II clinical trial of multiple peptide vaccination for advanced head and neck cancer patients revealed induction of immune responses and improved OS. Clin Cancer Res; 2014;21 (2):312-21.
³⁵ Okuno et al. Clinical Trial of a 7-Peptide Cocktail Vaccine with Oral Chemotherapy for Patients with Metastatic Colorectal Cancer. Anticancer Res; 2014; 34: 3045-305.
³⁶ Rapoport et al. Combination Immunotherapy after ASCT for Multiple Myeloma Using MAGE-A3/Poly-ICLC Immunizations Followed by Adoptive Transfer of Vaccine-Primed and Costimulated Autologous T Cells. Clin Cancer Res; 2014; 20(5): 1355-1365.
³⁷ Greenfield et al. A phase I dose-escalation clinical trial of a peptidebased human papillomavirus therapeutic vaccine with Candida skin test reagent as a novel vaccine adjuvant for treating women with biopsy-proven cervical intraepithelial neoplasia 2/3. Oncoimmunol; 2015; 4:10, e1031439.
³⁸ Snyder et al. Genetic basis for clinical response to CTLA-4 blockade in melanoma. N Engl J Med. 2014; 371(23):2189-99.
³⁹ Van Allen et al. Genomic correlates of response to CTLA-4 blockade in metastatic melanoma. Science; 2015; 350:6257.
⁴⁰ Li et al. Thrombocytopenia caused by the development of antibodies to thrombopoietin. Blood; 2001; 98:3241-3248
⁴¹ Takedatsu et al. Determination of Thrombopoietin-Derived Peptides Recognized by Both Cellular and Humoral Immunities in Healthy Donors and Patients with Thrombocytopenia. 2005; 23(7): 975-982
⁴² Eisenhauer et al. New response evaluation criteria in solid tumors: revised RECIST guideline (version 1.1). Eur J Cancer; 2009; 45(2):228-47.
⁴³ Therasse et al. New guidelines to evaluate the response to treatment in solid tumors: European Organization for Research and Treatment of Cancer, National Cancer Institute of the United States, National Cancer Institute of Canada. J Natl Cancer Inst; 2000; 92:205-216.
⁴⁴ Tsuchida & Therasse. Response evaluation criteria in solid tumors (RECIST): New guidelines. Med Pediatr Oncol. 2001; 37:1-3.
⁴⁵ Durie et al. International uniform response criteria for multiple myeloma. Leukemia; 2006;20:1467-1473.

## Claims

1. A peptide that
(a) is a fragment of a breast cancer associated antigen selected from SPAG9, AKAP-4, BORIS, NY-SAR-35, NY-BR-1, SURVIVIN, MAGE-A11, PRAME, MAGE-A9, and HOM-TES-85, wherein the fragment comprises the amino acid sequence of any one of SEQ ID NOs: 1 to 20; or
(b) consists of two or more fragments of one or more breast cancer associated antigens selected from SPAG9, AKAP-4, BORIS, NY-SAR-35, NY-BR-1, SURVIVIN, MAGE-A11, PRAME, MAGE-A9 and HOM-TES-8 wherein the fragments overlap or are arranged end to end in the peptide and wherein each fragment comprises a different amino acid sequence selected from SEQ ID NOs: 1 to 20; or
(c) is a fragment of a colorectal cancer-associated antigen selected from TSP50, EpCAM, SPAG9, CAGE1, FBXO39, SURVIVIN, MAGE-A8 and MAGE-A6, wherein the fragment comprises the amino acid sequence of any one of SEQ ID NOs: 21 to 40; or
(d) consists of two or more fragments of one or more colorectal cancer associated antigens selected from TSP50, EpCAM, SPAG9, CAGE1, FBXO39, SURVIVIN, MAGE-A8 and MAGE-A6, wherein the fragments overlap or are arranged end to end in the peptide and wherein each fragment comprises a different amino acid sequence selected from SEQ ID NOs: 21 to 40.

2. A peptide according to claim 1, wherein the peptide consists of fragments of
(a) at least two different breast cancer-associated antigens selected from SPAG9, AKAP-4, BORIS, NY-SAR-35, NY-BR-1, SURVIVIN, MAGE-A11, PRAME, MAGE-A9 and HOM-TES-8, wherein each fragment comprises a different amino acid sequence selected from SEQ ID NOs: 1 to 20; or
(b) at least two different colorectal cancer-associated antigens selected from TSP50, EpCAM, SPAG9, CAGE1, FBXO39, SURVIVIN, MAGE-A8 and MAGE-A6, wherein each fragment comprises a different amino acid sequence selected from SEQ ID NOs: 21 to 40.

3. A peptide according to claim 1 or claim 2 that comprises one or more amino acid sequences selected from SEQ ID NOs: 41-80.

4. A peptide according to any one of claims 1 to 3 comprising or consisting of the amino acid sequence of any one of SEQ ID NOs: 81-142.

5. A panel of two or more peptides according to any one of claims 1 to 4, wherein
(a) each peptide comprises a different amino acid sequence selected from SEQ ID NOs: 1 to 20; or
(b) each peptide comprises a different amino acid sequence selected from SEQ ID NOs: 21 to 40.

6. A panel of peptides according to claim 5 comprising nine peptides having the amino acid sequences of SEQ ID NOs: 92, 93, 98, 99, 100, 101, 103, 104, and 105.

7. A panel of peptides according to claim 5 comprising nine peptides having the amino acid sequences of SEQ ID NOs: 121, 124, 126, 127, 130, 131, 132, 133 and 134.

8. A pharmaceutical composition having a peptide according to any one of claims 1 to 4 or a panel of peptides according to any one of claims 5 to 7 as active ingredients.

9. A method of inducing a cytotoxic T cell response in a subject, the method comprising administering to the subject a pharmaceutical composition according to claim 8.

10. The method of claim 9 that is a method of treating cancer, optionally breast cancer or colorectal cancer.

11. A method of identifying a human subject who will likely have a cytotoxic T cell response to administration of a pharmaceutical composition according to claim 8, the method comprising
(i) determining the HLA class I genotype of a human subject;
(ii) determining that the active ingredient peptide(s) of the pharmaceutical composition comprise a sequence that is a T cell epitope capable of binding to at least three HLA class I of the subject; and
(iii) identifying the subject as likely to have a cytotoxic T cell response to administration of the pharmaceutical composition.

12. A method of identifying a subject who will likely have a clinical response to a method of treatment according to claim 10, the method comprising
(i) determining the HLA class I genotype of the subject;
(ii) determining that one or more cancer-associated antigens selected from SPAG9, AKAP-4, BORIS, NY-SAR-35, NY-BR-1, SURVIVIN, MAGE-A11, PRAME, MAGE-A9, HOM-TES-85, TSP50, EpCAM, CAGE1, FBXO39, MAGE-A8 and MAGE-A6 is expressed by cancer cells of the subject;
(iii) determining that the active ingredient peptide(s) of the pharmaceutical composition comprise two or more different amino acid sequences each of which is
(a) a fragment of a cancer-associated antigen expressed by cancer cells of the subject as determined in step (ii); and
(b) a T cell epitope capable of binding to at least three HLA class I of the subject; and
(iv) identifying the subject as likely to have a clinical response to the method of treatment.

13. The method of claim 12 further comprising selecting administration of the pharmaceutical composition as a method of treatment for the subject, and optionally further treating the subject by administering the pharmaceutical composition.

14. A method of treatment according to claim 10, wherein the subject has been identified as likely to have a clinical response to the treatment by the method according to claim 12.

15. A method of identifying a human subject who will likely not have a clinical response to a method of treatment according to claim 10, the method comprising
(i) determining the HLA class I genotype of a human subject;
(ii) determining that the active ingredient peptide(s) of the pharmaceutical composition do not comprise two or more different amino acid sequences each of which is a T cell epitope capable of binding to at least three HLA class I of the subject; and
(iii) identifying the subject as likely not to have a clinical response to the method of treatment.


SEQUENCE LISTING

<110>  Epitope Kft

<120>  VACCINE

<130>  N409651

<140>  tbc
<141>  2017-03-03

<160>  171

<170>  PatentIn version 3.5

<210>  1
<211>  9
<212>  PRT
<213>  Artificial Sequence

<220>
<223>  BestEPI AKAP-4

<400>  1

Tyr Leu Met Asn Arg Pro Gln Asn Leu
1               5


<210>  2
<211>  9
<212>  PRT
<213>  Artificial Sequence

<220>
<223>  BestEPI AKAP-4

<400>  2

Met Met Ala Tyr Ser Asp Thr Thr Met
1               5


<210>  3
<211>  9
<212>  PRT
<213>  Artificial Sequence

<220>
<223>  BestEPI BORIS

<400>  3

Phe Thr Ser Ser Arg Met Ser Ser Phe
1               5


<210>  4
<211>  9
<212>  PRT
<213>  Artificial Sequence

<220>

<223>  BestEPI AKAP-4

<400>  4

Tyr Ala Leu Gly Phe Gln His Ala Leu
1               5

<210>  5
<211>  9
<212>  PRT
<213>  Artificial Sequence

<220>
<223>  BestEPI SPAG9

<400>  5

Lys Met Ser Ser Leu Leu Pro Thr Met
1               5

<210>  6
<211>  9
<212>  PRT
<213>  Artificial Sequence

<220>
<223>  BestEPI SPAG9

<400>  6

Phe Thr Val Cys Asn Ser His Val Leu
1               5

<210>  7
<211>  9
<212>  PRT
<213>  Artificial Sequence

<220>
<223>  BestEPI BORIS

<400>  7

Met Ala Phe Val Thr Ser Gly Glu Leu
1               5

<210>  8
<211>  9
<212>  PRT
<213>  Artificial Sequence

<220>
<223>  BestEPI PRAME

<400>  8

Tyr Leu His Ala Arg Leu Arg Glu Leu
1               5

<210>  9
<211>  9
<212>  PRT
<213>  Artificial Sequence

<220>
<223>  BestEPI SPAG9

<400>  9

Val Met Ser Glu Arg Val Ser Gly Leu
1               5


<210>  10
<211>  9
<212>  PRT
<213>  Artificial Sequence

<220>
<223>  BestEPI BORIS

<400>  10

Phe Thr Gln Ser Gly Thr Met Lys Ile
1               5


<210>  11
<211>  9
<212>  PRT
<213>  Artificial Sequence

<220>
<223>  BestEPI NY-SAR-35

<400>  11

Phe Ser Ser Ser Gly Thr Thr Ser Phe
1               5


<210>  12
<211>  9
<212>  PRT
<213>  Artificial Sequence

<220>
<223>  BestEPI MAGE-A9

<400>  12

Phe Met Phe Gln Glu Ala Leu Lys Leu
1               5


<210>  13
<211>  9
<212>  PRT
<213>  Artificial Sequence

<220>

<223>  BestEPI NY-SAR-35

<400>  13

Phe Val Leu Ala Asn Gly His Ile Leu
1               5

<210>  14
<211>  9
<212>  PRT
<213>  Artificial Sequence

<220>
<223>  BestEPI PRAME

<400>  14

Lys Ala Met Val Gln Ala Trp Pro Phe
1               5

<210>  15
<211>  9
<212>  PRT
<213>  Artificial Sequence

<220>
<223>  BestEPI NY-BR-1

<400>  15

Tyr Ser Cys Asp Ser Arg Ser Leu Phe
1               5

<210>  16
<211>  9
<212>  PRT
<213>  Artificial Sequence

<220>
<223>  BestEPI Survivin

<400>  16

Arg Ala Ile Glu Gln Leu Ala Ala Met
1               5

<210>  17
<211>  9
<212>  PRT
<213>  Artificial Sequence

<220>
<223>  BestEPI MAGE-A11

<400>  17

Ala Met Asp Ala Ile Phe Gly Ser Leu
1               5

<210>  18
<211>  9
<212>  PRT
<213>  Artificial Sequence

<220>
<223>  BestEPI HOM-TES-85

<400>  18

Met Ala Ser Phe Arg Lys Leu Thr Leu
1               5


<210>  19
<211>  9
<212>  PRT
<213>  Artificial Sequence

<220>
<223>  BestEPI MAGE-A9

<400>  19

Ser Ser Ile Ser Val Tyr Tyr Thr Leu
1               5


<210>  20
<211>  9
<212>  PRT
<213>  Artificial Sequence

<220>
<223>  BestEPI NY-BR-1

<400>  20

Ser Ala Phe Glu Pro Ala Thr Glu Met
1               5


<210>  21
<211>  9
<212>  PRT
<213>  Artificial Sequence

<220>
<223>  BestEPI TSP50

<400>  21

Phe Ser Tyr Glu Gln Asp Pro Thr Leu
1               5


<210>  22
<211>  9
<212>  PRT
<213>  Artificial Sequence

<220>

<223>  BestEPI EpCAM

<400>  22

Arg Thr Tyr Trp Ile Ile Ile Glu Leu
1               5


<210>  23
<211>  9
<212>  PRT
<213>  Artificial Sequence

<220>
<223>  BestEPI TSP50

<400>  23

Thr Thr Met Glu Thr Gln Phe Pro Val
1               5


<210>  24
<211>  9
<212>  PRT
<213>  Artificial Sequence

<220>
<223>  BestEPI Spag9

<400>  24

Phe Ser Phe Val Arg Ile Thr Ala Leu
1               5


<210>  25
<211>  9
<212>  PRT
<213>  Artificial Sequence

<220>
<223>  BestEPI Spag9

<400>  25

Lys Met Ser Ser Leu Leu Pro Thr Met
1               5


<210>  26
<211>  9
<212>  PRT
<213>  Artificial Sequence

<220>
<223>  BestEPI CAGE1

<400>  26

Lys Met His Ser Leu Leu Ala Leu Met
1               5

<210>  27
<211>  9
<212>  PRT
<213>  Artificial Sequence

<220>
<223>  BestEPI FBXO39

<400>  27

Phe Met Asn Pro Tyr Asn Ala Val Leu
1               5


<210>  28
<211>  9
<212>  PRT
<213>  Artificial Sequence

<220>
<223>  BestEPI CAGE1

<400>  28

Lys Ser Met Thr Met Met Pro Ala Leu
1               5


<210>  29
<211>  9
<212>  PRT
<213>  Artificial Sequence

<220>
<223>  BestEPI EpCAM

<400>  29

Tyr Val Asp Glu Lys Ala Pro Glu Phe
1               5


<210>  30
<211>  9
<212>  PRT
<213>  Artificial Sequence

<220>
<223>  BestEPI FBXO39

<400>  30

Lys Thr Met Ser Thr Phe His Asn Leu
1               5


<210>  31
<211>  9
<212>  PRT
<213>  Artificial Sequence

<220>

<223>  BestEPI Survivin

<400>  31

Arg Ala Ile Glu Gln Leu Ala Ala Met
1               5


<210>  32
<211>  9
<212>  PRT
<213>  Artificial Sequence

<220>
<223>  BestEPI Spag9

<400>  32

Val Met Ser Glu Arg Val Ser Gly Leu
1               5


<210>  33
<211>  9
<212>  PRT
<213>  Artificial Sequence

<220>
<223>  BestEPI TSP50

<400>  33

Tyr Arg Ala Gln Arg Phe Trp Ser Trp
1               5


<210>  34
<211>  9
<212>  PRT
<213>  Artificial Sequence

<220>
<223>  BestEPI FBXO39

<400>  34

Phe Phe Phe Glu Arg Ile Met Lys Tyr
1               5


<210>  35
<211>  9
<212>  PRT
<213>  Artificial Sequence

<220>
<223>  BestEPI Survivin

<400>  35

Ser Thr Phe Lys Asn Trp Pro Phe Leu
1               5

<210>  36
<211>  9
<212>  PRT
<213>  Artificial Sequence

<220>
<223>  BestEPI Mage-A8

<400>  36

Ala Ile Trp Glu Ala Leu Ser Val Met
1               5


<210>  37
<211>  9
<212>  PRT
<213>  Artificial Sequence

<220>
<223>  BestEPI Mage-A8

<400>  37

Lys Val Ala Glu Leu Val Arg Phe Leu
1               5


<210>  38
<211>  9
<212>  PRT
<213>  Artificial Sequence

<220>
<223>  BestEPI Mage-A6

<400>  38

Phe Val Gln Glu Asn Tyr Leu Glu Tyr
1               5


<210>  39
<211>  9
<212>  PRT
<213>  Artificial Sequence

<220>
<223>  BestEPI Mage-A8

<400>  39

Arg Ala Leu Ala Glu Thr Ser Tyr Val
1               5


<210>  40
<211>  9
<212>  PRT
<213>  Artificial Sequence

<220>

<223>  BestEPI Mage-A6

<400>  40

Tyr Ile Phe Ala Thr Cys Leu Gly Leu
1               5


<210>  41
<211>  15
<212>  PRT
<213>  Artificial Sequence

<220>
<223>  15mer from Cancer Testis Antigen (CTA)

<400>  41

Asp Gln Val Asn Ile Asp Tyr Leu Met Asn Arg Pro Gln Asn Leu
1               5                   10                  15


<210>  42
<211>  15
<212>  PRT
<213>  Artificial Sequence

<220>
<223>  15mer from Cancer Testis Antigen (CTA)

<400>  42

Met Met Ala Tyr Ser Asp Thr Thr Met Met Ser Asp Asp Ile Asp
1               5                   10                  15


<210>  43
<211>  15
<212>  PRT
<213>  Artificial Sequence

<220>
<223>  15mer from Cancer Testis Antigen (CTA)

<400>  43

Met Phe Thr Ser Ser Arg Met Ser Ser Phe Asn Arg His Met Lys
1               5                   10                  15


<210>  44
<211>  15
<212>  PRT
<213>  Artificial Sequence

<220>
<223>  15mer from Cancer Testis Antigen (CTA)

<400>  44

Leu Gln Lys Tyr Ala Leu Gly Phe Gln His Ala Leu Ser Pro Ser
1               5                   10                  15

<210>  45
<211>  15
<212>  PRT
<213>  Artificial Sequence

<220>
<223>  15mer from Cancer Testis Antigen (CTA)

<400>  45

Ala Gln Lys Met Ser Ser Leu Leu Pro Thr Met Trp Leu Gly Ala
1               5                   10                  15


<210>  46
<211>  15
<212>  PRT
<213>  Artificial Sequence

<220>
<223>  15mer from Cancer Testis Antigen (CTA)

<400>  46

Gly Asn Ile Leu Asp Ser Phe Thr Val Cys Asn Ser His Val Leu
1               5                   10                  15


<210>  47
<211>  15
<212>  PRT
<213>  Artificial Sequence

<220>
<223>  15mer from Cancer Testis Antigen (CTA)

<400>  47

Asn Met Ala Phe Val Thr Ser Gly Glu Leu Val Arg His Arg Arg
1               5                   10                  15


<210>  48
<211>  15
<212>  PRT
<213>  Artificial Sequence

<220>
<223>  15mer from Cancer Testis Antigen (CTA)

<400>  48

Leu Glu Arg Leu Ala Tyr Leu His Ala Arg Leu Arg Glu Leu Leu
1               5                   10                  15


<210>  49
<211>  15
<212>  PRT
<213>  Artificial Sequence

<220>

<223>  15mer from Cancer Testis Antigen (CTA)

<400>  49

Ser Gly Ala Val Met Ser Glu Arg Val Ser Gly Leu Ala Gly Ser
1               5                   10                  15


<210>  50
<211>  15
<212>  PRT
<213>  Artificial Sequence

<220>
<223>  15mer from Cancer Testis Antigen (CTA)

<400>  50

His Thr Arg Phe Thr Gln Ser Gly Thr Met Lys Ile His Ile Leu
1               5                   10                  15


<210>  51
<211>  15
<212>  PRT
<213>  Artificial Sequence

<220>
<223>  15mer from Cancer Testis Antigen (CTA)

<400>  51

Phe Ser Ser Ser Gly Thr Thr Ser Phe Lys Cys Phe Ala Pro Phe
1               5                   10                  15


<210>  52
<211>  15
<212>  PRT
<213>  Artificial Sequence

<220>
<223>  15mer from Cancer Testis Antigen (CTA)

<400>  52

Gln Leu Glu Phe Met Phe Gln Glu Ala Leu Lys Leu Lys Val Ala
1               5                   10                  15


<210>  53
<211>  15
<212>  PRT
<213>  Artificial Sequence

<220>
<223>  15mer from Cancer Testis Antigen (CTA)

<400>  53

Cys Ser Gly Ser Ser Tyr Phe Val Leu Ala Asn Gly His Ile Leu
1               5                   10                  15

<210>  54
<211>  15
<212>  PRT
<213>  Artificial Sequence

<220>
<223>  15mer from Cancer Testis Antigen (CTA)

<400>  54

Arg His Ser Gln Thr Leu Lys Ala Met Val Gln Ala Trp Pro Phe
1               5                   10                  15


<210>  55
<211>  15
<212>  PRT
<213>  Artificial Sequence

<220>
<223>  15mer from Cancer Testis Antigen (CTA)

<400>  55

Tyr Ser Cys Asp Ser Arg Ser Leu Phe Glu Ser Ser Ala Lys Ile
1               5                   10                  15


<210>  56
<211>  15
<212>  PRT
<213>  Artificial Sequence

<220>
<223>  15mer from Cancer Testis Antigen (CTA)

<400>  56

Thr Ala Lys Lys Val Arg Arg Ala Ile Glu Gln Leu Ala Ala Met
1               5                   10                  15


<210>  57
<211>  15
<212>  PRT
<213>  Artificial Sequence

<220>
<223>  15mer from Cancer Testis Antigen (CTA)

<400>  57

Ser Pro Thr Ala Met Asp Ala Ile Phe Gly Ser Leu Ser Asp Glu
1               5                   10                  15


<210>  58
<211>  15
<212>  PRT
<213>  Artificial Sequence

<220>

<223>  15mer from Cancer Testis Antigen (CTA)

<400>  58

Met Ala Ser Phe Arg Lys Leu Thr Leu Ser Glu Lys Val Pro Pro
1               5                   10                  15


<210>  59
<211>  15
<212>  PRT
<213>  Artificial Sequence

<220>
<223>  15mer from Cancer Testis Antigen (CTA)

<400>  59

Ser Ser Ile Ser Val Tyr Tyr Thr Leu Trp Ser Gln Phe Asp Glu
1               5                   10                  15


<210>  60
<211>  15
<212>  PRT
<213>  Artificial Sequence

<220>
<223>  15mer from Cancer Testis Antigen (CTA)

<400>  60

Pro Gly Lys Pro Ser Ala Phe Glu Pro Ala Thr Glu Met Gln Lys
1               5                   10                  15


<210>  61
<211>  15
<212>  PRT
<213>  Artificial Sequence

<220>
<223>  15mer from Cancer Testis Antigen (CTA)

<400>  61

Arg Ser Cys Gly Phe Ser Tyr Glu Gln Asp Pro Thr Leu Arg Asp
1               5                   10                  15


<210>  62
<211>  15
<212>  PRT
<213>  Artificial Sequence

<220>
<223>  15mer from Cancer Testis Antigen (CTA)

<400>  62

Val Arg Thr Tyr Trp Ile Ile Ile Glu Leu Lys His Lys Ala Arg
1               5                   10                  15

<210>  63
<211>  15
<212>  PRT
<213>  Artificial Sequence

<220>
<223>  15mer from Cancer Testis Antigen (CTA)

<400>  63

Pro Ser Thr Thr Met Glu Thr Gln Phe Pro Val Ser Glu Gly Lys
1               5                   10                  15


<210>  64
<211>  15
<212>  PRT
<213>  Artificial Sequence

<220>
<223>  15mer from Cancer Testis Antigen (CTA)

<400>  64

Gly Thr Gly Lys Leu Gly Phe Ser Phe Val Arg Ile Thr Ala Leu
1               5                   10                  15


<210>  65
<211>  15
<212>  PRT
<213>  Artificial Sequence

<220>
<223>  15mer from Cancer Testis Antigen (CTA)

<400>  65

Ala Gln Lys Met Ser Ser Leu Leu Pro Thr Met Trp Leu Gly Ala
1               5                   10                  15


<210>  66
<211>  15
<212>  PRT
<213>  Artificial Sequence

<220>
<223>  15mer from Cancer Testis Antigen (CTA)

<400>  66

Leu Ala Ser Lys Met His Ser Leu Leu Ala Leu Met Val Gly Leu
1               5                   10                  15


<210>  67
<211>  15
<212>  PRT
<213>  Artificial Sequence

<220>

<223>  15mer from Cancer Testis Antigen (CTA)

<400>  67

Lys Phe Met Asn Pro Tyr Asn Ala Val Leu Thr Lys Lys Phe Gln
1               5                   10                  15


<210>  68
<211>  15
<212>  PRT
<213>  Artificial Sequence

<220>
<223>  15mer from Cancer Testis Antigen (CTA)

<400>  68

Pro Lys Ser Met Thr Met Met Pro Ala Leu Phe Lys Glu Asn Arg
1               5                   10                  15


<210>  69
<211>  15
<212>  PRT
<213>  Artificial Sequence

<220>
<223>  15mer from Cancer Testis Antigen (CTA)

<400>  69

Tyr Val Asp Glu Lys Ala Pro Glu Phe Ser Met Gln Gly Leu Lys
1               5                   10                  15


<210>  70
<211>  15
<212>  PRT
<213>  Artificial Sequence

<220>
<223>  15mer from Cancer Testis Antigen (CTA)

<400>  70

Phe Lys Lys Thr Met Ser Thr Phe His Asn Leu Val Ser Leu Asn
1               5                   10                  15


<210>  71
<211>  15
<212>  PRT
<213>  Artificial Sequence

<220>
<223>  15mer from Cancer Testis Antigen (CTA)

<400>  71

Thr Ala Lys Lys Val Arg Arg Ala Ile Glu Gln Leu Ala Ala Met
1               5                   10                  15

<210>  72
<211>  15
<212>  PRT
<213>  Artificial Sequence

<220>
<223>  15mer from Cancer Testis Antigen (CTA)

<400>  72

Ser Gly Ala Val Met Ser Glu Arg Val Ser Gly Leu Ala Gly Ser
1               5                   10                  15


<210>  73
<211>  15
<212>  PRT
<213>  Artificial Sequence

<220>
<223>  15mer from Cancer Testis Antigen (CTA)

<400>  73

Ser Arg Tyr Arg Ala Gln Arg Phe Trp Ser Trp Val Gly Gln Ala
1               5                   10                  15


<210>  74
<211>  15
<212>  PRT
<213>  Artificial Sequence

<220>
<223>  15mer from Cancer Testis Antigen (CTA)

<400>  74

Lys Val Asn Phe Phe Phe Glu Arg Ile Met Lys Tyr Glu Arg Leu
1               5                   10                  15


<210>  75
<211>  15
<212>  PRT
<213>  Artificial Sequence

<220>
<223>  15mer from Cancer Testis Antigen (CTA)

<400>  75

Lys Asp His Arg Ile Ser Thr Phe Lys Asn Trp Pro Phe Leu Glu
1               5                   10                  15


<210>  76
<211>  15
<212>  PRT
<213>  Artificial Sequence

<220>

<223>  15mer from Cancer Testis Antigen (CTA)

<400>  76

Ser Arg Ala Pro Glu Glu Ala Ile Trp Glu Ala Leu Ser Val Met
1               5                   10                  15


<210>  77
<211>  15
<212>  PRT
<213>  Artificial Sequence

<220>
<223>  15mer from Cancer Testis Antigen (CTA)

<400>  77

Asp Glu Lys Val Ala Glu Leu Val Arg Phe Leu Leu Arg Lys Tyr
1               5                   10                  15


<210>  78
<211>  15
<212>  PRT
<213>  Artificial Sequence

<220>
<223>  15mer from Cancer Testis Antigen (CTA)

<400>  78

Gln Tyr Phe Val Gln Glu Asn Tyr Leu Glu Tyr Arg Gln Val Pro
1               5                   10                  15


<210>  79
<211>  15
<212>  PRT
<213>  Artificial Sequence

<220>
<223>  15mer from Cancer Testis Antigen (CTA)

<400>  79

Glu Phe Leu Trp Gly Pro Arg Ala Leu Ala Glu Thr Ser Tyr Val
1               5                   10                  15


<210>  80
<211>  15
<212>  PRT
<213>  Artificial Sequence

<220>
<223>  15mer from Cancer Testis Antigen (CTA)

<400>  80

Ile Gly His Val Tyr Ile Phe Ala Thr Cys Leu Gly Leu Ser Tyr
1               5                   10                  15

<210>  81
<211>  30
<212>  PRT
<213>  Artificial Sequence

<220>
<223>  30mer AKAP4

<400>  81

Leu Gln Lys Tyr Ala Leu Gly Phe Gln His Ala Leu Ser Pro Ser Met
1               5                   10                  15

Met Ala Tyr Ser Asp Thr Thr Met Met Ser Asp Asp Ile Asp
            20                  25                  30

<210>  82
<211>  30
<212>  PRT
<213>  Artificial Sequence

<220>
<223>  30mer BORIS/AKAP4

<400>  82

Val Cys Met Phe Thr Ser Ser Arg Met Ser Ser Phe Asn Arg His Val
1               5                   10                  15

Asn Ile Asp Tyr Leu Met Asn Arg Pro Gln Asn Leu Arg Leu
            20                  25                  30

<210>  83
<211>  30
<212>  PRT
<213>  Artificial Sequence

<220>
<223>  30mer BORIS

<400>  83

Asn Met Ala Phe Val Thr Ser Gly Glu Leu Val Arg His Arg Arg His
1               5                   10                  15

Thr Arg Phe Thr Gln Ser Gly Thr Met Lys Ile His Ile Leu
            20                  25                  30

<210>  84
<211>  30
<212>  PRT
<213>  Artificial Sequence

<220>
<223>  30mer SPAG9

<400>  84

Leu Asp Ser Phe Thr Val Cys Asn Ser His Val Leu Cys Ile Ala Lys
1               5                   10                  15

Leu Gly Phe Ser Phe Val Arg Ile Thr Ala Leu Met Val Ser
            20                  25                  30

<210>  85
<211>  30
<212>  PRT
<213>  Artificial Sequence

<220>
<223>  30mer SPAG9/NY-SAR-35

<400>  85

Ala Gln Lys Met Ser Ser Leu Leu Pro Thr Met Trp Leu Gly Ala Met
1               5                   10                  15

Met Gln Met Phe Gly Leu Gly Ala Ile Ser Leu Ile Leu Val
            20                  25                  30

<210>  86
<211>  30
<212>  PRT
<213>  Artificial Sequence

<220>
<223>  30mer PRAME

<400>  86

Leu Glu Arg Leu Ala Tyr Leu His Ala Arg Leu Arg Glu Leu Leu Gln
1               5                   10                  15

Thr Leu Lys Ala Met Val Gln Ala Trp Pro Phe Thr Cys Leu
            20                  25                  30

<210>  87
<211>  30
<212>  PRT
<213>  Artificial Sequence

<220>
<223>  30mer NY-SAR-35

<400>  87

Ser Ser Tyr Phe Val Leu Ala Asn Gly His Ile Leu Pro Asn Ser Leu
1               5                   10                  15

Arg His Lys Cys Cys Phe Ser Ser Ser Gly Thr Thr Ser Phe
            20                  25                  30

<210>  88
<211>  30
<212>  PRT
<213>  Artificial Sequence

<220>
<223>  30mer Survivin/MAGE-A9

<400>  88

Thr Ala Lys Lys Val Arg Arg Ala Ile Glu Gln Leu Ala Ala Met Gln
1               5                   10                  15

Leu Glu Phe Met Phe Gln Glu Ala Leu Lys Leu Lys Val Ala
            20                  25                  30

<210>  89
<211>  30
<212>  PRT
<213>  Artificial Sequence

<220>
<223>  30mer MAGE-A11/NY-BR-1

<400>  89

Thr Ser His Ser Tyr Val Leu Val Thr Ser Leu Asn Leu Ser Tyr Tyr
1               5                   10                  15

Ser Cys Asp Ser Arg Ser Leu Phe Glu Ser Ser Ala Lys Ile
            20                  25                  30

<210>  90
<211>  30
<212>  PRT
<213>  Artificial Sequence

<220>
<223>  30mer SPAG9/BORIS

<400>  90

Leu Asp Ser Phe Thr Val Cys Asn Ser His Val Leu Cys Ile Ala Val
1               5                   10                  15

Cys Met Phe Thr Ser Ser Arg Met Ser Ser Phe Asn Arg His
            20                  25                  30

<210>  91
<211>  30
<212>  PRT
<213>  Artificial Sequence

<220>
<223>  30mer NY-SAR35/PRAME

<400>  91

Leu Arg His Lys Cys Cys Phe Ser Ser Ser Gly Thr Thr Ser Phe Gln
1               5                   10                  15

Thr Leu Lys Ala Met Val Gln Ala Trp Pro Phe Thr Cys Leu
            20                  25                  30

<210>  92
<211>  30
<212>  PRT
<213>  Artificial Sequence

<220>
<223>  30mer NY-BR-1/SURVIVIN

<400>  92

Tyr Ser Cys Asp Ser Arg Ser Leu Phe Glu Ser Ser Ala Lys Ile Thr
1               5                   10                  15

Ala Lys Lys Val Arg Arg Ala Ile Glu Gln Leu Ala Ala Met
            20                  25                  30

<210>  93
<211>  30
<212>  PRT
<213>  Artificial Sequence

<220>
<223>  30mer AKAP-4/BORIS

<400>  93

Met Met Ala Tyr Ser Asp Thr Thr Met Met Ser Asp Asp Ile Asp His
1               5                   10                  15

Thr Arg Phe Thr Gln Ser Gly Thr Met Lys Ile His Ile Leu
            20                  25                  30

<210>  94
<211>  30
<212>  PRT
<213>  Artificial Sequence

<220>
<223>  30mer SPAG9/AKAP-4

<400>  94

Ala Gln Lys Met Ser Ser Leu Leu Pro Thr Met Trp Leu Gly Ala Leu
1               5                   10                  15

Gln Lys Tyr Ala Leu Gly Phe Gln His Ala Leu Ser Pro Ser
            20                  25                  30

<210>  95
<211>  30
<212>  PRT
<213>  Artificial Sequence

<220>
<223>  30mer MAGE-A11/BORIS

<400>  95

Thr Ser His Ser Tyr Val Leu Val Thr Ser Leu Asn Leu Ser Tyr Asn
1               5                   10                  15

Met Ala Phe Val Thr Ser Gly Glu Leu Val Arg His Arg Arg
            20                  25                  30

<210>  96
<211>  30
<212>  PRT
<213>  Artificial Sequence

<220>
<223>  30mer NY-SAR-35/AKAP-4

<400>  96

Met Met Gln Met Phe Gly Leu Gly Ala Ile Ser Leu Ile Leu Val Val
1               5                   10                  15

Asn Ile Asp Tyr Leu Met Asn Arg Pro Gln Asn Leu Arg Leu
            20                  25                  30

<210>  97
<211>  30
<212>  PRT
<213>  Artificial Sequence

<220>
<223>  30mer NY-SAR-SPAG9

<400>  97

Ser Ser Tyr Phe Val Leu Ala Asn Gly His Ile Leu Pro Asn Ser Lys
1               5                   10                  15

Leu Gly Phe Ser Phe Val Arg Ile Thr Ala Leu Met Val Ser
            20                  25                  30

<210>  98
<211>  30
<212>  PRT
<213>  Artificial Sequence

<220>
<223>  30mer PRAME/MAGE-A9

<400>  98

Leu Glu Arg Leu Ala Tyr Leu His Ala Arg Leu Arg Glu Leu Leu Gln
1               5                   10                  15

Leu Glu Phe Met Phe Gln Glu Ala Leu Lys Leu Lys Val Ala
            20                  25                  30

<210>  99
<211>  30
<212>  PRT
<213>  Artificial Sequence

<220>
<223>  30mer SPAG9/AKAP4

<400>  99

Gly Asn Ile Leu Asp Ser Phe Thr Val Cys Asn Ser His Val Leu Leu
1               5                   10                  15

Gln Lys Tyr Ala Leu Gly Phe Gln His Ala Leu Ser Pro Ser
            20                  25                  30

<210>  100
<211>  30
<212>  PRT
<213>  Artificial Sequence

<220>
<223>  30mer BORIS/NY-SAR-35

<400>  100

Asn Met Ala Phe Val Thr Ser Gly Glu Leu Val Arg His Arg Arg Phe
1               5                   10                  15

Ser Ser Ser Gly Thr Thr Ser Phe Lys Cys Phe Ala Pro Phe
            20                  25                  30

<210>  101
<211>  30
<212>  PRT
<213>  Artificial Sequence

<220>
<223>  30mer SPAG9/BORIS

<400>  101

Ala Gln Lys Met Ser Ser Leu Leu Pro Thr Met Trp Leu Gly Ala Met
1               5                   10                  15

Phe Thr Ser Ser Arg Met Ser Ser Phe Asn Arg His Met Lys
            20                  25                  30

<210>  102
<211>  30
<212>  PRT
<213>  Artificial Sequence

<220>
<223>  30mer MAGE-A11/PRAME

<400>  102

Thr Ser His Ser Tyr Val Leu Val Thr Ser Leu Asn Leu Ser Tyr His
1               5                   10                  15

Ser Gln Thr Leu Lys Ala Met Val Gln Ala Trp Pro Phe Thr
            20                  25                  30

<210>  103
<211>  30
<212>  PRT
<213>  Artificial Sequence

<220>
<223>  30mer HomTes85/MageA11

<400>  103

Met Ala Ser Phe Arg Lys Leu Thr Leu Ser Glu Lys Val Pro Pro Ser
1               5                   10                  15

Pro Thr Ala Met Asp Ala Ile Phe Gly Ser Leu Ser Asp Glu
            20                  25                  30

<210>  104
<211>  30
<212>  PRT
<213>  Artificial Sequence

<220>
<223>  30mer AKAP4/MageA11

<400>  104

Asp Gln Val Asn Ile Asp Tyr Leu Met Asn Arg Pro Gln Asn Leu Arg
1               5                   10                  15

His Ser Gln Thr Leu Lys Ala Met Val Gln Ala Trp Pro Phe
            20                  25                  30

<210>  105
<211>  30
<212>  PRT
<213>  Artificial Sequence

<220>
<223>  30mer NYSAR/SPAG9

<400>  105

Cys Ser Gly Ser Ser Tyr Phe Val Leu Ala Asn Gly His Ile Leu Ser
1               5                   10                  15

Gly Ala Val Met Ser Glu Arg Val Ser Gly Leu Ala Gly Ser
            20                  25                  30

<210>  106
<211>  30
<212>  PRT
<213>  Artificial Sequence

<220>
<223>  30mer AKAP-4/MAGE-A9

<400>  106

Asp Leu Ser Phe Tyr Val Asn Arg Leu Ser Ser Leu Val Ile Gln Ser
1               5                   10                  15

Ser Ile Ser Val Tyr Tyr Thr Leu Trp Ser Gln Phe Asp Glu
            20                  25                  30

<210>  107
<211>  30
<212>  PRT
<213>  Artificial Sequence

<220>
<223>  30mer SPAG9/NY-ESO-1

<400>  107

Ser Gly Ala Val Met Ser Glu Arg Val Ser Gly Leu Ala Gly Ser Ser
1               5                   10                  15

Arg Leu Leu Glu Phe Tyr Leu Ala Met Pro Phe Ala Thr Pro
            20                  25                  30

<210>  108
<211>  30
<212>  PRT
<213>  Artificial Sequence

<220>
<223>  30mer HOM-TES-85/MAGE-A11

<400>  108

Met Ala Ser Phe Arg Lys Leu Thr Leu Ser Glu Lys Val Pro Pro Glu
1               5                   10                  15

Ser Phe Ser Pro Thr Ala Met Asp Ala Ile Phe Gly Ser Leu
            20                  25                  30

<210>  109
<211>  30
<212>  PRT
<213>  Artificial Sequence

<220>
<223>  30mer NY-ESO-1/NY-BR-1

<400>  109

Phe Tyr Leu Ala Met Pro Phe Ala Thr Pro Met Glu Ala Glu Leu Lys
1               5                   10                  15

Pro Ser Ala Phe Glu Pro Ala Thr Glu Met Gln Lys Ser Val
            20                  25                  30

<210>  110
<211>  30
<212>  PRT
<213>  Artificial Sequence

<220>
<223>  30mer MAGE-A11/PRAME

<400>  110

Ala Met Asp Ala Ile Phe Gly Ser Leu Ser Asp Glu Gly Ser Gly His
1               5                   10                  15

Ser Gln Thr Leu Lys Ala Met Val Gln Ala Trp Pro Phe Thr
            20                  25                  30

<210>  111
<211>  30
<212>  PRT
<213>  Artificial Sequence

<220>
<223>  30mer NY-SAR-35/SPAG9

<400>  111

Phe Val Leu Ala Asn Gly His Ile Leu Pro Asn Ser Glu Asn Ala Gly
1               5                   10                  15

Thr Gly Lys Leu Gly Phe Ser Phe Val Arg Ile Thr Ala Leu
            20                  25                  30

<210>  112
<211>  30
<212>  PRT
<213>  Artificial Sequence

<220>
<223>  30mer TSP50

<400>  112

Val Cys Ser Met Glu Gly Thr Trp Tyr Leu Val Gly Leu Val Ser Tyr
1               5                   10                  15

Arg Ser Cys Gly Phe Ser Tyr Glu Gln Asp Pro Thr Leu Arg
            20                  25                  30

<210>  113
<211>  30
<212>  PRT
<213>  Artificial Sequence

<220>
<223>  30mer EpCAM/TSP50

<400>  113

Val Arg Thr Tyr Trp Ile Ile Ile Glu Leu Lys His Lys Ala Arg Leu
1               5                   10                  15

Pro Ser Thr Thr Met Glu Thr Gln Phe Pro Val Ser Glu Gly
            20                  25                  30

<210>  114
<211>  30
<212>  PRT
<213>  Artificial Sequence

<220>
<223>  30mer Survivin

<400>  114

Thr Ala Lys Lys Val Arg Arg Ala Ile Glu Gln Leu Ala Ala Met Met
1               5                   10                  15

Gly Ala Pro Thr Leu Pro Pro Ala Trp Gln Pro Phe Leu Lys
            20                  25                  30

<210>  115
<211>  30
<212>  PRT
<213>  Artificial Sequence

<220>
<223>  30mer CAGE1

<400>  115

Leu Ala Ser Lys Met His Ser Leu Leu Ala Leu Met Val Gly Leu Pro
1               5                   10                  15

Lys Ser Met Thr Met Met Pro Ala Leu Phe Lys Glu Asn Arg
            20                  25                  30

<210>  116
<211>  30
<212>  PRT
<213>  Artificial Sequence

<220>
<223>  30mer Spag9

<400>  116

Lys Leu Gly Phe Ser Phe Val Arg Ile Thr Ala Leu Met Val Ser Leu
1               5                   10                  15

Asp Ser Phe Thr Val Cys Asn Ser His Val Leu Cys Ile Ala
            20                  25                  30

<210>  117
<211>  30
<212>  PRT
<213>  Artificial Sequence

<220>
<223>  30mer FBXO39

<400>  117

Lys Phe Met Asn Pro Tyr Asn Ala Val Leu Thr Lys Lys Phe Gln Phe
1               5                   10                  15

Lys Lys Thr Met Ser Thr Phe His Asn Leu Val Ser Leu Asn
            20                  25                  30

<210>  118
<211>  30
<212>  PRT
<213>  Artificial Sequence

<220>
<223>  30mer Spag9/FBXO39

<400>  118

Ala Gln Lys Met Ser Ser Leu Leu Pro Thr Met Trp Leu Gly Ala Lys
1               5                   10                  15

Val Asn Phe Phe Phe Glu Arg Ile Met Lys Tyr Glu Arg Leu
            20                  25                  30

<210>  119
<211>  30
<212>  PRT
<213>  Artificial Sequence

<220>
<223>  30mer Survivin/Mage-A8

<400>  119

Lys Asp His Arg Ile Ser Thr Phe Lys Asn Trp Pro Phe Leu Glu Pro
1               5                   10                  15

Glu Glu Ala Ile Trp Glu Ala Leu Ser Val Met Gly Leu Tyr
            20                  25                  30

<210>  120
<211>  30
<212>  PRT
<213>  Artificial Sequence

<220>
<223>  30mer TPS50

<400>  120

Tyr Arg Ser Cys Gly Phe Ser Tyr Glu Gln Asp Pro Thr Leu Arg Val
1               5                   10                  15

Cys Ser Met Glu Gly Thr Trp Tyr Leu Val Gly Leu Val Ser
            20                  25                  30

<210>  121
<211>  30
<212>  PRT
<213>  Artificial Sequence

<220>
<223>  30mer EpCAM/Survivin

<400>  121

Val Arg Thr Tyr Trp Ile Ile Ile Glu Leu Lys His Lys Ala Arg Thr
1               5                   10                  15

Ala Lys Lys Val Arg Arg Ala Ile Glu Gln Leu Ala Ala Met
            20                  25                  30

<210>  122
<211>  30
<212>  PRT
<213>  Artificial Sequence

<220>
<223>  30mer TSP50/Spag9

<400>  122

Leu Pro Ser Thr Thr Met Glu Thr Gln Phe Pro Val Ser Glu Gly Lys
1               5                   10                  15

Leu Gly Phe Ser Phe Val Arg Ile Thr Ala Leu Met Val Ser
            20                  25                  30

<210>  123
<211>  30
<212>  PRT
<213>  Artificial Sequence

<220>
<223>  30mer Survivin/Mage-A8

<400>  123

Met Gly Ala Pro Thr Leu Pro Pro Ala Trp Gln Pro Phe Leu Lys Pro
1               5                   10                  15

Glu Glu Ala Ile Trp Glu Ala Leu Ser Val Met Gly Leu Tyr
            20                  25                  30

<210>  124
<211>  30
<212>  PRT
<213>  Artificial Sequence

<220>
<223>  30mer CAGE1/Survivin

<400>  124

Leu Ala Ser Lys Met His Ser Leu Leu Ala Leu Met Val Gly Leu Lys
1               5                   10                  15

Asp His Arg Ile Ser Thr Phe Lys Asn Trp Pro Phe Leu Glu
            20                  25                  30

<210>  125
<211>  30
<212>  PRT
<213>  Artificial Sequence

<220>
<223>  30mer CAGE1/Spag9

<400>  125

Pro Lys Ser Met Thr Met Met Pro Ala Leu Phe Lys Glu Asn Arg Leu
1               5                   10                  15

Asp Ser Phe Thr Val Cys Asn Ser His Val Leu Cys Ile Ala
            20                  25                  30

<210>  126
<211>  30
<212>  PRT
<213>  Artificial Sequence

<220>
<223>  30mer FBXO39

<400>  126

Lys Phe Met Asn Pro Tyr Asn Ala Val Leu Thr Lys Lys Phe Gln Lys
1               5                   10                  15

Val Asn Phe Phe Phe Glu Arg Ile Met Lys Tyr Glu Arg Leu
            20                  25                  30

<210>  127
<211>  30
<212>  PRT
<213>  Artificial Sequence

<220>
<223>  30mer Spag9/FBXO39

<400>  127

Ala Gln Lys Met Ser Ser Leu Leu Pro Thr Met Trp Leu Gly Ala Phe
1               5                   10                  15

Lys Lys Thr Met Ser Thr Phe His Asn Leu Val Ser Leu Asn
            20                  25                  30

<210>  128
<211>  30
<212>  PRT
<213>  Artificial Sequence

<220>
<223>  30mer TSP50

<400>  128

Gly Phe Ser Tyr Glu Gln Asp Pro Thr Leu Arg Asp Pro Glu Ala Val
1               5                   10                  15

Cys Ser Met Glu Gly Thr Trp Tyr Leu Val Gly Leu Val Ser
            20                  25                  30

<210>  129
<211>  30
<212>  PRT
<213>  Artificial Sequence

<220>
<223>  30mer CAGE1/Spag9

<400>  129

Pro Lys Ser Met Thr Met Met Pro Ala Leu Phe Lys Glu Asn Arg Gly
1               5                   10                  15

Asn Ile Leu Asp Ser Phe Thr Val Cys Asn Ser His Val Leu
            20                  25                  30

<210>  130
<211>  30
<212>  PRT
<213>  Artificial Sequence

<220>
<223>  30mer TSP50

<400>  130

Pro Ser Thr Thr Met Glu Thr Gln Phe Pro Val Ser Glu Gly Lys Ser
1               5                   10                  15

Arg Tyr Arg Ala Gln Arg Phe Trp Ser Trp Val Gly Gln Ala
            20                  25                  30

<210>  131
<211>  30
<212>  PRT
<213>  Artificial Sequence

<220>
<223>  30mer EpCAM/MAGE-A8

<400>  131

Tyr Val Asp Glu Lys Ala Pro Glu Phe Ser Met Gln Gly Leu Lys Asp
1               5                   10                  15

Glu Lys Val Ala Glu Leu Val Arg Phe Leu Leu Arg Lys Tyr
            20                  25                  30

<210>  132
<211>  30
<212>  PRT
<213>  Artificial Sequence

<220>
<223>  30mer TSP50/Spag9

<400>  132

Arg Ser Cys Gly Phe Ser Tyr Glu Gln Asp Pro Thr Leu Arg Asp Gly
1               5                   10                  15

Thr Gly Lys Leu Gly Phe Ser Phe Val Arg Ile Thr Ala Leu
            20                  25                  30

<210>  133
<211>  30
<212>  PRT
<213>  Artificial Sequence

<220>
<223>  30mer Mage-A8/Mage-A6

<400>  133

Ser Arg Ala Pro Glu Glu Ala Ile Trp Glu Ala Leu Ser Val Met Gln
1               5                   10                  15

Tyr Phe Val Gln Glu Asn Tyr Leu Glu Tyr Arg Gln Val Pro
            20                  25                  30

<210>  134
<211>  30
<212>  PRT
<213>  Artificial Sequence

<220>
<223>  30mer CAGE1/Spag9

<400>  134

Pro Lys Ser Met Thr Met Met Pro Ala Leu Phe Lys Glu Asn Arg Ser
1               5                   10                  15

Gly Ala Val Met Ser Glu Arg Val Ser Gly Leu Ala Gly Ser
            20                  25                  30

<210>  135
<211>  30
<212>  PRT
<213>  Artificial Sequence

<220>
<223>  30mer SPAG9/FBXO39

<400>  135

Ser Gly Ala Val Met Ser Glu Arg Val Ser Gly Leu Ala Gly Ser Arg
1               5                   10                  15

Asn Ser Ile Arg Ser Ser Phe Ile Ser Ser Leu Ser Phe Phe
            20                  25                  30

<210>  136
<211>  30
<212>  PRT
<213>  Artificial Sequence

<220>
<223>  30mer CAGE11/MAGE-A8

<400>  136

Asn Ile Glu Asn Tyr Ser Thr Asn Ala Leu Ile Gln Pro Val Asp Glu
1               5                   10                  15

Lys Val Ala Glu Leu Val Arg Phe Leu Leu Arg Lys Tyr Gln
            20                  25                  30

<210>  137
<211>  30
<212>  PRT
<213>  Artificial Sequence

<220>
<223>  30mer CAGE11/MAGE-A6

<400>  137

Arg Gln Phe Glu Thr Val Cys Lys Phe His Trp Val Glu Ala Phe Lys
1               5                   10                  15

Leu Leu Thr Gln Tyr Phe Val Gln Glu Asn Tyr Leu Glu Tyr
            20                  25                  30

<210>  138
<211>  30
<212>  PRT
<213>  Artificial Sequence

<220>
<223>  30mer MAGE-A8/MAGE-A3

<400>  138

Glu Phe Leu Trp Gly Pro Arg Ala Leu Ala Glu Thr Ser Tyr Val Lys
1               5                   10                  15

Leu Leu Thr Gln His Phe Val Gln Glu Asn Tyr Leu Glu Tyr
            20                  25                  30

<210>  139
<211>  30
<212>  PRT
<213>  Artificial Sequence

<220>
<223>  30mer MAGE-A8/EpCAM

<400>  139

Ala Ser Ser Ser Ser Thr Leu Ile Met Gly Thr Leu Glu Glu Val Gln
1               5                   10                  15

Thr Leu Ile Tyr Tyr Val Asp Glu Lys Ala Pro Glu Phe Ser
            20                  25                  30

<210>  140
<211>  30
<212>  PRT
<213>  Artificial Sequence

<220>
<223>  30mer TSP50/MAGE-A3

<400>  140

Ser Arg Thr Leu Leu Leu Ala Leu Pro Leu Pro Leu Ser Leu Leu Ile
1               5                   10                  15

Gly His Leu Tyr Ile Phe Ala Thr Cys Leu Gly Leu Ser Tyr
            20                  25                  30

<210>  141
<211>  30
<212>  PRT
<213>  Artificial Sequence

<220>
<223>  30mer LEMD1/MAGE-A6

<400>  141

Phe Ile Ile Val Val Phe Val Tyr Leu Thr Val Glu Asn Lys Ser Ile
1               5                   10                  15

Gly His Val Tyr Ile Phe Ala Thr Cys Leu Gly Leu Ser Tyr
            20                  25                  30

<210>  142
<211>  30
<212>  PRT
<213>  Artificial Sequence

<220>
<223>  30mer EPCAM

<400>  142

Leu Leu Ala Ala Ala Thr Ala Thr Phe Ala Ala Ala Gln Glu Glu Gln
1               5                   10                  15

Thr Leu Ile Tyr Tyr Val Asp Glu Lys Ala Pro Glu Phe Ser
            20                  25                  30

<210>  143
<211>  1321
<212>  PRT
<213>  Homo sapiens

<400>  143

Met Glu Leu Glu Asp Gly Val Val Tyr Gln Glu Glu Pro Gly Gly Ser
1               5                   10                  15

Gly Ala Val Met Ser Glu Arg Val Ser Gly Leu Ala Gly Ser Ile Tyr
            20                  25                  30

Arg Glu Phe Glu Arg Leu Ile Gly Arg Tyr Asp Glu Glu Val Val Lys
        35                  40                  45

Glu Leu Met Pro Leu Val Val Ala Val Leu Glu Asn Leu Asp Ser Val
    50                  55                  60

Phe Ala Gln Asp Gln Glu His Gln Val Glu Leu Glu Leu Leu Arg Asp
65                  70                  75                  80

Asp Asn Glu Gln Leu Ile Thr Gln Tyr Glu Arg Glu Lys Ala Leu Arg
                85                  90                  95

Lys His Ala Glu Glu Lys Phe Ile Glu Phe Glu Asp Ser Gln Glu Gln
            100                 105                 110

Glu Lys Lys Asp Leu Gln Thr Arg Val Glu Ser Leu Glu Ser Gln Thr
        115                 120                 125

Arg Gln Leu Glu Leu Lys Ala Lys Asn Tyr Ala Asp Gln Ile Ser Arg
    130                 135                 140

Leu Glu Glu Arg Glu Ala Glu Leu Lys Lys Glu Tyr Asn Ala Leu His
145                 150                 155                 160

Gln Arg His Thr Glu Met Ile His Asn Tyr Met Glu His Leu Glu Arg
                165                 170                 175

Thr Lys Leu His Gln Leu Ser Gly Ser Asp Gln Leu Glu Ser Thr Ala
            180                 185                 190

His Ser Arg Ile Arg Lys Glu Arg Pro Ile Ser Leu Gly Ile Phe Pro
        195                 200                 205

Leu Pro Ala Gly Asp Gly Leu Leu Thr Pro Asp Ala Gln Lys Gly Gly
    210                 215                 220

Glu Thr Pro Gly Ser Glu Gln Trp Lys Phe Gln Glu Leu Ser Gln Pro
225                 230                 235                 240

Arg Ser His Thr Ser Leu Lys Val Ser Asn Ser Pro Glu Pro Gln Lys
                245                 250                 255

Ala Val Glu Gln Glu Asp Glu Leu Ser Asp Val Ser Gln Gly Gly Ser
            260                 265                 270

Lys Ala Thr Thr Pro Ala Ser Thr Ala Asn Ser Asp Val Ala Thr Ile
        275                 280                 285

Pro Thr Asp Thr Pro Leu Lys Glu Glu Asn Glu Gly Phe Val Lys Val

290                 295                 300

Thr Asp Ala Pro Asn Lys Ser Glu Ile Ser Lys His Ile Glu Val Gln
305                 310                 315                 320

Val Ala Gln Glu Thr Arg Asn Val Ser Thr Gly Ser Ala Glu Asn Glu
                325                 330                 335

Glu Lys Ser Glu Val Gln Ala Ile Ile Glu Ser Thr Pro Glu Leu Asp
            340                 345                 350

Met Asp Lys Asp Leu Ser Gly Tyr Lys Gly Ser Ser Thr Pro Thr Lys
        355                 360                 365

Gly Ile Glu Asn Lys Ala Phe Asp Arg Asn Thr Glu Ser Leu Phe Glu
    370                 375                 380

Glu Leu Ser Ser Ala Gly Ser Gly Leu Ile Gly Asp Val Asp Glu Gly
385                 390                 395                 400

Ala Asp Leu Leu Gly Met Gly Arg Glu Val Glu Asn Leu Ile Leu Glu
                405                 410                 415

Asn Thr Gln Leu Leu Glu Thr Lys Asn Ala Leu Asn Ile Val Lys Asn
            420                 425                 430

Asp Leu Ile Ala Lys Val Asp Glu Leu Thr Cys Glu Lys Asp Val Leu
        435                 440                 445

Gln Gly Glu Leu Glu Ala Val Lys Gln Ala Lys Leu Lys Leu Glu Glu
    450                 455                 460

Lys Asn Arg Glu Leu Glu Glu Glu Leu Arg Lys Ala Arg Ala Glu Ala
465                 470                 475                 480

Glu Asp Ala Arg Gln Lys Ala Lys Asp Asp Asp Asp Ser Asp Ile Pro
                485                 490                 495

Thr Ala Gln Arg Lys Arg Phe Thr Arg Val Glu Met Ala Arg Val Leu
            500                 505                 510

Met Glu Arg Asn Gln Tyr Lys Glu Arg Leu Met Glu Leu Gln Glu Ala
        515                 520                 525

Val Arg Trp Thr Glu Met Ile Arg Ala Ser Arg Glu Asn Pro Ala Met
    530                 535                 540

Gln Glu Lys Lys Arg Ser Ser Ile Trp Gln Phe Phe Ser Arg Leu Phe
545                 550                 555                 560

Ser Ser Ser Ser Asn Thr Thr Lys Lys Pro Glu Pro Pro Val Asn Leu
                565                 570                 575

Lys Tyr Asn Ala Pro Thr Ser His Val Thr Pro Ser Val Lys Lys Arg
            580                 585                 590

Ser Ser Thr Leu Ser Gln Leu Pro Gly Asp Lys Ser Lys Ala Phe Asp
        595                 600                 605

Phe Leu Ser Glu Glu Thr Glu Ala Ser Leu Ala Ser Arg Arg Glu Gln
    610                 615                 620

Lys Arg Glu Gln Tyr Arg Gln Val Lys Ala His Val Gln Lys Glu Asp
625                 630                 635                 640

Gly Arg Val Gln Ala Phe Gly Trp Ser Leu Pro Gln Lys Tyr Lys Gln
                645                 650                 655

Val Thr Asn Gly Gln Gly Glu Asn Lys Met Lys Asn Leu Pro Val Pro
            660                 665                 670

Val Tyr Leu Arg Pro Leu Asp Glu Lys Asp Thr Ser Met Lys Leu Trp
        675                 680                 685

Cys Ala Val Gly Val Asn Leu Ser Gly Gly Lys Thr Arg Asp Gly Gly
    690                 695                 700

Ser Val Val Gly Ala Ser Val Phe Tyr Lys Asp Val Ala Gly Leu Asp
705                 710                 715                 720

Thr Glu Gly Ser Lys Gln Arg Ser Ala Ser Gln Ser Ser Leu Asp Lys
                725                 730                 735

Leu Asp Gln Glu Leu Lys Glu Gln Gln Lys Glu Leu Lys Asn Gln Glu
            740                 745                 750

Glu Leu Ser Ser Leu Val Trp Ile Cys Thr Ser Thr His Ser Ala Thr
        755                 760                 765

Lys Val Leu Ile Ile Asp Ala Val Gln Pro Gly Asn Ile Leu Asp Ser
    770                 775                 780

Phe Thr Val Cys Asn Ser His Val Leu Cys Ile Ala Ser Val Pro Gly
785                 790                 795                 800

Ala Arg Glu Thr Asp Tyr Pro Ala Gly Glu Asp Leu Ser Glu Ser Gly
                805                 810                 815

Gln Val Asp Lys Ala Ser Leu Cys Gly Ser Met Thr Ser Asn Ser Ser
            820                 825                 830

Ala Glu Thr Asp Ser Leu Leu Gly Gly Ile Thr Val Val Gly Cys Ser
        835                 840                 845

Ala Glu Gly Val Thr Gly Ala Ala Thr Ser Pro Ser Thr Asn Gly Ala
    850                 855                 860

Ser Pro Val Met Asp Lys Pro Pro Glu Met Glu Ala Glu Asn Ser Glu
865                 870                 875                 880

Val Asp Glu Asn Val Pro Thr Ala Glu Glu Ala Thr Glu Ala Thr Glu
                885                 890                 895

Gly Asn Ala Gly Ser Ala Glu Asp Thr Val Asp Ile Ser Gln Thr Gly
            900                 905                 910

Val Tyr Thr Glu His Val Phe Thr Asp Pro Leu Gly Val Gln Ile Pro
        915                 920                 925

Glu Asp Leu Ser Pro Val Tyr Gln Ser Ser Asn Asp Ser Asp Ala Tyr
    930                 935                 940

Lys Asp Gln Ile Ser Val Leu Pro Asn Glu Gln Asp Leu Val Arg Glu
945                 950                 955                 960

Glu Ala Gln Lys Met Ser Ser Leu Leu Pro Thr Met Trp Leu Gly Ala
                965                 970                 975

Gln Asn Gly Cys Leu Tyr Val His Ser Ser Val Ala Gln Trp Arg Lys
            980                 985                 990

Cys Leu His Ser Ile Lys Leu Lys  Asp Ser Ile Leu Ser  Ile Val His
        995                 1000                1005

Val Lys  Gly Ile Val Leu Val  Ala Leu Ala Asp Gly  Thr Leu Ala
    1010                1015                1020

Ile Phe  His Arg Gly Val Asp  Gly Gln Trp Asp Leu  Ser Asn Tyr
    1025                1030                1035

His Leu  Leu Asp Leu Gly Arg  Pro His His Ser Ile  Arg Cys Met
    1040                1045                1050

Thr Val Val His Asp Lys Val Trp Cys Gly Tyr Arg Asn Lys Ile
    1055                1060                1065

Tyr Val Val Gln Pro Lys Ala Met Lys Ile Glu Lys Ser Phe Asp
    1070                1075                1080

Ala His Pro Arg Lys Glu Ser Gln Val Arg Gln Leu Ala Trp Val
    1085                1090                1095

Gly Asp Gly Val Trp Val Ser Ile Arg Leu Asp Ser Thr Leu Arg
    1100                1105                1110

Leu Tyr His Ala His Thr Tyr Gln His Leu Gln Asp Val Asp Ile
    1115                1120                1125

Glu Pro Tyr Val Ser Lys Met Leu Gly Thr Gly Lys Leu Gly Phe
    1130                1135                1140

Ser Phe Val Arg Ile Thr Ala Leu Met Val Ser Cys Asn Arg Leu
    1145                1150                1155

Trp Val Gly Thr Gly Asn Gly Val Ile Ile Ser Ile Pro Leu Thr
    1160                1165                1170

Glu Thr Asn Lys Thr Ser Gly Val Pro Gly Asn Arg Pro Gly Ser
    1175                1180                1185

Val Ile Arg Val Tyr Gly Asp Glu Asn Ser Asp Lys Val Thr Pro
    1190                1195                1200

Gly Thr Phe Ile Pro Tyr Cys Ser Met Ala His Ala Gln Leu Cys
    1205                1210                1215

Phe His Gly His Arg Asp Ala Val Lys Phe Phe Val Ala Val Pro
    1220                1225                1230

Gly Gln Val Ile Ser Pro Gln Ser Ser Ser Ser Gly Thr Asp Leu
    1235                1240                1245

Thr Gly Asp Lys Ala Gly Pro Ser Ala Gln Glu Pro Gly Ser Gln
    1250                1255                1260

Thr Pro Leu Lys Ser Met Leu Val Ile Ser Gly Gly Glu Gly Tyr
    1265                1270                1275

Ile Asp Phe Arg Met Gly Asp Glu Gly Gly Glu Ser Glu Leu Leu

1280                1285                1290

Gly Glu  Asp Leu Pro Leu Glu  Pro Ser Val Thr Lys  Ala Glu Arg
    1295                1300                1305

Ser His  Leu Ile Val Trp Gln  Val Met Tyr Gly Asn  Glu
    1310                1315                1320

<210>  144
<211>  854
<212>  PRT
<213>  Homo sapiens

<400>  144

Met Met Ala Tyr Ser Asp Thr Thr Met Met Ser Asp Asp Ile Asp Trp
1               5                   10                  15

Leu Arg Ser His Arg Gly Val Cys Lys Val Asp Leu Tyr Asn Pro Glu
            20                  25                  30

Gly Gln Gln Asp Gln Asp Arg Lys Val Ile Cys Phe Val Asp Val Ser
        35                  40                  45

Thr Leu Asn Val Glu Asp Lys Asp Tyr Lys Asp Ala Ala Ser Ser Ser
    50                  55                  60

Ser Glu Gly Asn Leu Asn Leu Gly Ser Leu Glu Glu Lys Glu Ile Ile
65                  70                  75                  80

Val Ile Lys Asp Thr Glu Lys Lys Asp Gln Ser Lys Thr Glu Gly Ser
                85                  90                  95

Val Cys Leu Phe Lys Gln Ala Pro Ser Asp Pro Val Ser Val Leu Asn
            100                 105                 110

Trp Leu Leu Ser Asp Leu Gln Lys Tyr Ala Leu Gly Phe Gln His Ala
        115                 120                 125

Leu Ser Pro Ser Thr Ser Thr Cys Lys His Lys Val Gly Asp Thr Glu
    130                 135                 140

Gly Glu Tyr His Arg Ala Ser Ser Glu Asn Cys Tyr Ser Val Tyr Ala
145                 150                 155                 160

Asp Gln Val Asn Ile Asp Tyr Leu Met Asn Arg Pro Gln Asn Leu Arg
                165                 170                 175

Leu Glu Met Thr Ala Ala Lys Asn Thr Asn Asn Asn Gln Ser Pro Ser

            180                 185                 190

Ala Pro Pro Ala Lys Pro Pro Ser Thr Gln Arg Ala Val Ile Ser Pro
        195                 200                 205

Asp Gly Glu Cys Ser Ile Asp Asp Leu Ser Phe Tyr Val Asn Arg Leu
    210                 215                 220

Ser Ser Leu Val Ile Gln Met Ala His Lys Glu Ile Lys Glu Lys Leu
225                 230                 235                 240

Glu Gly Lys Ser Lys Cys Leu His His Ser Ile Cys Pro Ser Pro Gly
                245                 250                 255

Asn Lys Glu Arg Ile Ser Pro Arg Thr Pro Ala Ser Lys Ile Ala Ser
            260                 265                 270

Glu Met Ala Tyr Glu Ala Val Glu Leu Thr Ala Ala Glu Met Arg Gly
        275                 280                 285

Thr Gly Glu Glu Ser Arg Glu Gly Gly Gln Lys Ser Phe Leu Tyr Ser
    290                 295                 300

Glu Leu Ser Asn Lys Ser Lys Ser Gly Asp Lys Gln Met Ser Gln Arg
305                 310                 315                 320

Glu Ser Lys Glu Phe Ala Asp Ser Ile Ser Lys Gly Leu Met Val Tyr
                325                 330                 335

Ala Asn Gln Val Ala Ser Asp Met Met Val Ser Leu Met Lys Thr Leu
            340                 345                 350

Lys Val His Ser Ser Gly Lys Pro Ile Pro Ala Ser Val Val Leu Lys
        355                 360                 365

Arg Val Leu Leu Arg His Thr Lys Glu Ile Val Ser Asp Leu Ile Asp
    370                 375                 380

Ser Cys Met Lys Asn Leu His Asn Ile Thr Gly Val Leu Met Thr Asp
385                 390                 395                 400

Ser Asp Phe Val Ser Ala Val Lys Arg Asn Leu Phe Asn Gln Trp Lys
                405                 410                 415

Gln Asn Ala Thr Asp Ile Met Glu Ala Met Leu Lys Arg Leu Val Ser
            420                 425                 430

Ala Leu Ile Gly Glu Glu Lys Glu Thr Lys Ser Gln Ser Leu Ser Tyr
        435                 440                 445

Ala Ser Leu Lys Ala Gly Ser His Asp Pro Lys Cys Arg Asn Gln Ser
    450                 455                 460

Leu Glu Phe Ser Thr Met Lys Ala Glu Met Lys Glu Arg Asp Lys Gly
465                 470                 475                 480

Lys Met Lys Ser Asp Pro Cys Lys Ser Leu Thr Ser Ala Glu Lys Val
                485                 490                 495

Gly Glu His Ile Leu Lys Glu Gly Leu Thr Ile Trp Asn Gln Lys Gln
            500                 505                 510

Gly Asn Ser Cys Lys Val Ala Thr Lys Ala Cys Ser Asn Lys Asp Glu
        515                 520                 525

Lys Gly Glu Lys Ile Asn Ala Ser Thr Asp Ser Leu Ala Lys Asp Leu
    530                 535                 540

Ile Val Ser Ala Leu Lys Leu Ile Gln Tyr His Leu Thr Gln Gln Thr
545                 550                 555                 560

Lys Gly Lys Asp Thr Cys Glu Glu Asp Cys Pro Gly Ser Thr Met Gly
                565                 570                 575

Tyr Met Ala Gln Ser Thr Gln Tyr Glu Lys Cys Gly Gly Gly Gln Ser
            580                 585                 590

Ala Lys Ala Leu Ser Val Lys Gln Leu Glu Ser His Arg Ala Pro Gly
        595                 600                 605

Pro Ser Thr Cys Gln Lys Glu Asn Gln His Leu Asp Ser Gln Lys Met
    610                 615                 620

Asp Met Ser Asn Ile Val Leu Met Leu Ile Gln Lys Leu Leu Asn Glu
625                 630                 635                 640

Asn Pro Phe Lys Cys Glu Asp Pro Cys Glu Gly Glu Asn Lys Cys Ser
                645                 650                 655

Glu Pro Arg Ala Ser Lys Ala Ala Ser Met Ser Asn Arg Ser Asp Lys
            660                 665                 670

Ala Glu Glu Gln Cys Gln Glu His Gln Glu Leu Asp Cys Thr Ser Gly
        675                 680                 685

Met Lys Gln Ala Asn Gly Gln Phe Ile Asp Lys Leu Val Glu Ser Val
    690                 695                 700

Met Lys Leu Cys Leu Ile Met Ala Lys Tyr Ser Asn Asp Gly Ala Ala
705                 710                 715                 720

Leu Ala Glu Leu Glu Glu Gln Ala Ala Ser Ala Asn Lys Pro Asn Phe
                725                 730                 735

Arg Gly Thr Arg Cys Ile His Ser Gly Ala Met Pro Gln Asn Tyr Gln
            740                 745                 750

Asp Ser Leu Gly His Glu Val Ile Val Asn Asn Gln Cys Ser Thr Asn
        755                 760                 765

Ser Leu Gln Lys Gln Leu Gln Ala Val Leu Gln Trp Ile Ala Ala Ser
    770                 775                 780

Gln Phe Asn Val Pro Met Leu Tyr Phe Met Gly Asp Lys Asp Gly Gln
785                 790                 795                 800

Leu Glu Lys Leu Pro Gln Val Ser Ala Lys Ala Ala Glu Lys Gly Tyr
                805                 810                 815

Ser Val Gly Gly Leu Leu Gln Glu Val Met Lys Phe Ala Lys Glu Arg
            820                 825                 830

Gln Pro Asp Glu Ala Val Gly Lys Val Ala Arg Lys Gln Leu Leu Asp
        835                 840                 845

Trp Leu Leu Ala Asn Leu
    850

<210>  145
<211>  663
<212>  PRT
<213>  Homo sapiens

<400>  145

Met Ala Ala Thr Glu Ile Ser Val Leu Ser Glu Gln Phe Thr Lys Ile
1               5                   10                  15

Lys Glu Leu Glu Leu Met Pro Glu Lys Gly Leu Lys Glu Glu Glu Lys
            20                  25                  30

Asp Gly Val Cys Arg Glu Lys Asp His Arg Ser Pro Ser Glu Leu Glu
        35                  40                  45

Ala Glu Arg Thr Ser Gly Ala Phe Gln Asp Ser Val Leu Glu Glu Glu
    50                  55                  60

Val Glu Leu Val Leu Ala Pro Ser Glu Glu Ser Glu Lys Tyr Ile Leu
65                  70                  75                  80

Thr Leu Gln Thr Val His Phe Thr Ser Glu Ala Val Glu Leu Gln Asp
                85                  90                  95

Met Ser Leu Leu Ser Ile Gln Gln Gln Glu Gly Val Gln Val Val Val
            100                 105                 110

Gln Gln Pro Gly Pro Gly Leu Leu Trp Leu Glu Glu Gly Pro Arg Gln
        115                 120                 125

Ser Leu Gln Gln Cys Val Ala Ile Ser Ile Gln Gln Glu Leu Tyr Ser
    130                 135                 140

Pro Gln Glu Met Glu Val Leu Gln Phe His Ala Leu Glu Glu Asn Val
145                 150                 155                 160

Met Val Ala Ser Glu Asp Ser Lys Leu Ala Val Ser Leu Ala Glu Thr
                165                 170                 175

Thr Gly Leu Ile Lys Leu Glu Glu Glu Gln Glu Lys Asn Gln Leu Leu
            180                 185                 190

Ala Glu Arg Thr Lys Glu Gln Leu Phe Phe Val Glu Thr Met Ser Gly
        195                 200                 205

Asp Glu Arg Ser Asp Glu Ile Val Leu Thr Val Ser Asn Ser Asn Val
    210                 215                 220

Glu Glu Gln Glu Asp Gln Pro Thr Ala Gly Gln Ala Asp Ala Glu Lys
225                 230                 235                 240

Ala Lys Ser Thr Lys Asn Gln Arg Lys Thr Lys Gly Ala Lys Gly Thr
                245                 250                 255

Phe His Cys Asp Val Cys Met Phe Thr Ser Ser Arg Met Ser Ser Phe
            260                 265                 270

Asn Arg His Met Lys Thr His Thr Ser Glu Lys Pro His Leu Cys His
        275                 280                 285

Leu Cys Leu Lys Thr Phe Arg Thr Val Thr Leu Leu Arg Asn His Val
    290                 295                 300

Asn Thr His Thr Gly Thr Arg Pro Tyr Lys Cys Asn Asp Cys Asn Met
305                 310                 315                 320

Ala Phe Val Thr Ser Gly Glu Leu Val Arg His Arg Arg Tyr Lys His
                325                 330                 335

Thr His Glu Lys Pro Phe Lys Cys Ser Met Cys Lys Tyr Ala Ser Val
            340                 345                 350

Glu Ala Ser Lys Leu Lys Arg His Val Arg Ser His Thr Gly Glu Arg
        355                 360                 365

Pro Phe Gln Cys Cys Gln Cys Ser Tyr Ala Ser Arg Asp Thr Tyr Lys
    370                 375                 380

Leu Lys Arg His Met Arg Thr His Ser Gly Glu Lys Pro Tyr Glu Cys
385                 390                 395                 400

His Ile Cys His Thr Arg Phe Thr Gln Ser Gly Thr Met Lys Ile His
                405                 410                 415

Ile Leu Gln Lys His Gly Glu Asn Val Pro Lys Tyr Gln Cys Pro His
            420                 425                 430

Cys Ala Thr Ile Ile Ala Arg Lys Ser Asp Leu Arg Val His Met Arg
        435                 440                 445

Asn Leu His Ala Tyr Ser Ala Ala Glu Leu Lys Cys Arg Tyr Cys Ser
    450                 455                 460

Ala Val Phe His Glu Arg Tyr Ala Leu Ile Gln His Gln Lys Thr His
465                 470                 475                 480

Lys Asn Glu Lys Arg Phe Lys Cys Lys His Cys Ser Tyr Ala Cys Lys
                485                 490                 495

Gln Glu Arg His Met Thr Ala His Ile Arg Thr His Thr Gly Glu Lys
            500                 505                 510

Pro Phe Thr Cys Leu Ser Cys Asn Lys Cys Phe Arg Gln Lys Gln Leu
        515                 520                 525

Leu Asn Ala His Phe Arg Lys Tyr His Asp Ala Asn Phe Ile Pro Thr
    530                 535                 540

Val Tyr Lys Cys Ser Lys Cys Gly Lys Gly Phe Ser Arg Trp Ile Asn

545                 550                 555                 560

Leu His Arg His Ser Glu Lys Cys Gly Ser Gly Glu Ala Lys Ser Ala
                565                 570                 575

Ala Ser Gly Lys Gly Arg Arg Thr Arg Lys Arg Lys Gln Thr Ile Leu
            580                 585                 590

Lys Glu Ala Thr Lys Gly Gln Lys Glu Ala Ala Lys Gly Trp Lys Glu
        595                 600                 605

Ala Ala Asn Gly Asp Glu Ala Ala Ala Glu Glu Ala Ser Thr Thr Lys
    610                 615                 620

Gly Glu Gln Phe Pro Gly Glu Met Phe Pro Val Ala Cys Arg Glu Thr
625                 630                 635                 640

Thr Ala Arg Val Lys Glu Glu Val Asp Glu Gly Val Thr Cys Glu Met
                645                 650                 655

Leu Leu Asn Thr Met Asp Lys
            660

<210>  146
<211>  255
<212>  PRT
<213>  Homo sapiens

<400>  146

Met Ser Ser His Arg Arg Lys Ala Lys Gly Arg Asn Arg Arg Ser His
1               5                   10                  15

Arg Ala Met Arg Val Ala His Leu Glu Leu Ala Thr Tyr Glu Leu Ala
            20                  25                  30

Ala Thr Glu Ser Asn Pro Glu Ser Ser His Pro Gly Tyr Glu Ala Ala
        35                  40                  45

Met Ala Asp Arg Pro Gln Pro Gly Trp Arg Glu Ser Leu Lys Met Arg
    50                  55                  60

Val Ser Lys Pro Phe Gly Met Leu Met Leu Ser Ile Trp Ile Leu Leu
65                  70                  75                  80

Phe Val Cys Tyr Tyr Leu Ser Tyr Tyr Leu Cys Ser Gly Ser Ser Tyr
                85                  90                  95

Phe Val Leu Ala Asn Gly His Ile Leu Pro Asn Ser Glu Asn Ala His

            100                 105                 110

Gly Gln Ser Leu Glu Glu Asp Ser Ala Leu Glu Ala Leu Leu Asn Phe
        115                 120                 125

Phe Phe Pro Thr Thr Cys Asn Leu Arg Glu Asn Gln Val Ala Lys Pro
    130                 135                 140

Cys Asn Glu Leu Gln Asp Leu Ser Glu Ser Glu Cys Leu Arg His Lys
145                 150                 155                 160

Cys Cys Phe Ser Ser Ser Gly Thr Thr Ser Phe Lys Cys Phe Ala Pro
                165                 170                 175

Phe Arg Asp Val Pro Lys Gln Met Met Gln Met Phe Gly Leu Gly Ala
            180                 185                 190

Ile Ser Leu Ile Leu Val Cys Leu Pro Ile Tyr Cys Arg Ser Leu Phe
        195                 200                 205

Trp Arg Ser Glu Pro Ala Asp Asp Leu Gln Arg Gln Asp Asn Arg Val
    210                 215                 220

Val Thr Gly Leu Lys Lys Gln Arg Arg Lys Arg Lys Arg Lys Ser Glu
225                 230                 235                 240

Met Leu Gln Lys Ala Ala Arg Gly Arg Glu Glu His Gly Asp Glu
                245                 250                 255

<210> 147
<211> 1341
<212> PRT
<213> Homo sapiens

<400> 147

Met Thr Lys Arg Lys Lys Thr Ile Asn Leu Asn Ile Gln Asp Ala Gln
1               5                   10                  15

Lys Arg Thr Ala Leu His Trp Ala Cys Val Asn Gly His Glu Glu Val
            20                  25                  30

Val Thr Phe Leu Val Asp Arg Lys Cys Gln Leu Asp Val Leu Asp Gly
        35                  40                  45

Glu His Arg Thr Pro Leu Met Lys Ala Leu Gln Cys His Gln Glu Ala
    50                  55                  60

Cys Ala Asn Ile Leu Ile Asp Ser Gly Ala Asp Ile Asn Leu Val Asp

65                  70                  75                  80

Val Tyr Gly Asn Thr Ala Leu His Tyr Ala Val Tyr Ser Glu Ile Leu
                85                  90                  95

Ser Val Val Ala Lys Leu Leu Ser His Gly Ala Val Ile Glu Val His
            100                 105                 110

Asn Lys Ala Ser Leu Thr Pro Leu Leu Leu Ser Ile Thr Lys Arg Ser
        115                 120                 125

Glu Gln Ile Val Glu Phe Leu Leu Ile Lys Asn Ala Asn Ala Asn Ala
    130                 135                 140

Val Asn Lys Tyr Lys Cys Thr Ala Leu Met Leu Ala Val Cys His Gly
145                 150                 155                 160

Ser Ser Glu Ile Val Gly Met Leu Leu Gln Gln Asn Val Asp Val Phe
                165                 170                 175

Ala Ala Asp Ile Cys Gly Val Thr Ala Glu His Tyr Ala Val Thr Cys
            180                 185                 190

Gly Phe His His Ile His Glu Gln Ile Met Glu Tyr Ile Arg Lys Leu
        195                 200                 205

Ser Lys Asn His Gln Asn Thr Asn Pro Glu Gly Thr Ser Ala Gly Thr
    210                 215                 220

Pro Asp Glu Ala Ala Pro Leu Ala Glu Arg Thr Pro Asp Thr Ala Glu
225                 230                 235                 240

Ser Leu Val Glu Lys Thr Pro Asp Glu Ala Ala Pro Leu Val Glu Arg
                245                 250                 255

Thr Pro Asp Thr Ala Glu Ser Leu Val Glu Lys Thr Pro Asp Glu Ala
            260                 265                 270

Ala Ser Leu Val Glu Gly Thr Ser Asp Lys Ile Gln Cys Leu Glu Lys
        275                 280                 285

Ala Thr Ser Gly Lys Phe Glu Gln Ser Ala Glu Glu Thr Pro Arg Glu
    290                 295                 300

Ile Thr Ser Pro Ala Lys Glu Thr Ser Glu Lys Phe Thr Trp Pro Ala
305                 310                 315                 320

Lys Gly Arg Pro Arg Lys Ile Ala Trp Glu Lys Lys Glu Asp Thr Pro
                325                 330                 335

Arg Glu Ile Met Ser Pro Ala Lys Glu Thr Ser Glu Lys Phe Thr Trp
            340                 345                 350

Ala Ala Lys Gly Arg Pro Arg Lys Ile Ala Trp Glu Lys Lys Glu Thr
        355                 360                 365

Pro Val Lys Thr Gly Cys Val Ala Arg Val Thr Ser Asn Lys Thr Lys
    370                 375                 380

Val Leu Glu Lys Gly Arg Ser Lys Met Ile Ala Cys Pro Thr Lys Glu
385                 390                 395                 400

Ser Ser Thr Lys Ala Ser Ala Asn Asp Gln Arg Phe Pro Ser Glu Ser
                405                 410                 415

Lys Gln Glu Glu Asp Glu Glu Tyr Ser Cys Asp Ser Arg Ser Leu Phe
            420                 425                 430

Glu Ser Ser Ala Lys Ile Gln Val Cys Ile Pro Glu Ser Ile Tyr Gln
        435                 440                 445

Lys Val Met Glu Ile Asn Arg Glu Val Glu Glu Pro Pro Lys Lys Pro
    450                 455                 460

Ser Ala Phe Lys Pro Ala Ile Glu Met Gln Asn Ser Val Pro Asn Lys
465                 470                 475                 480

Ala Phe Glu Leu Lys Asn Glu Gln Thr Leu Arg Ala Asp Pro Met Phe
                485                 490                 495

Pro Pro Glu Ser Lys Gln Lys Asp Tyr Glu Glu Asn Ser Trp Asp Ser
            500                 505                 510

Glu Ser Leu Cys Glu Thr Val Ser Gln Lys Asp Val Cys Leu Pro Lys
        515                 520                 525

Ala Thr His Gln Lys Glu Ile Asp Lys Ile Asn Gly Lys Leu Glu Glu
    530                 535                 540

Ser Pro Asn Lys Asp Gly Leu Leu Lys Ala Thr Cys Gly Met Lys Val
545                 550                 555                 560

Ser Ile Pro Thr Lys Ala Leu Glu Leu Lys Asp Met Gln Thr Phe Lys
                565                 570                 575

Ala Glu Pro Pro Gly Lys Pro Ser Ala Phe Glu Pro Ala Thr Glu Met
            580                 585                 590

Gln Lys Ser Val Pro Asn Lys Ala Leu Glu Leu Lys Asn Glu Gln Thr
        595                 600                 605

Leu Arg Ala Asp Glu Ile Leu Pro Ser Glu Ser Lys Gln Lys Asp Tyr
    610                 615                 620

Glu Glu Asn Ser Trp Asp Thr Glu Ser Leu Cys Glu Thr Val Ser Gln
625                 630                 635                 640

Lys Asp Val Cys Leu Pro Lys Ala Ala His Gln Lys Glu Ile Asp Lys
                645                 650                 655

Ile Asn Gly Lys Leu Glu Gly Ser Pro Val Lys Asp Gly Leu Leu Lys
            660                 665                 670

Ala Asn Cys Gly Met Lys Val Ser Ile Pro Thr Lys Ala Leu Glu Leu
        675                 680                 685

Met Asp Met Gln Thr Phe Lys Ala Glu Pro Pro Glu Lys Pro Ser Ala
    690                 695                 700

Phe Glu Pro Ala Ile Glu Met Gln Lys Ser Val Pro Asn Lys Ala Leu
705                 710                 715                 720

Glu Leu Lys Asn Glu Gln Thr Leu Arg Ala Asp Glu Ile Leu Pro Ser
                725                 730                 735

Glu Ser Lys Gln Lys Asp Tyr Glu Glu Ser Ser Trp Asp Ser Glu Ser
            740                 745                 750

Leu Cys Glu Thr Val Ser Gln Lys Asp Val Cys Leu Pro Lys Ala Thr
        755                 760                 765

His Gln Lys Glu Ile Asp Lys Ile Asn Gly Lys Leu Glu Glu Ser Pro
    770                 775                 780

Asp Asn Asp Gly Phe Leu Lys Ala Pro Cys Arg Met Lys Val Ser Ile
785                 790                 795                 800

Pro Thr Lys Ala Leu Glu Leu Met Asp Met Gln Thr Phe Lys Ala Glu
                805                 810                 815

Pro Pro Glu Lys Pro Ser Ala Phe Glu Pro Ala Ile Glu Met Gln Lys
            820                 825                 830

Ser Val Pro Asn Lys Ala Leu Glu Leu Lys Asn Glu Gln Thr Leu Arg
        835                 840                 845

Ala Asp Gln Met Phe Pro Ser Glu Ser Lys Gln Lys Lys Val Glu Glu
    850                 855                 860

Asn Ser Trp Asp Ser Glu Ser Leu Arg Glu Thr Val Ser Gln Lys Asp
865                 870                 875                 880

Val Cys Val Pro Lys Ala Thr His Gln Lys Glu Met Asp Lys Ile Ser
                885                 890                 895

Gly Lys Leu Glu Asp Ser Thr Ser Leu Ser Lys Ile Leu Asp Thr Val
            900                 905                 910

His Ser Cys Glu Arg Ala Arg Glu Leu Gln Lys Asp His Cys Glu Gln
        915                 920                 925

Arg Thr Gly Lys Met Glu Gln Met Lys Lys Lys Phe Cys Val Leu Lys
    930                 935                 940

Lys Lys Leu Ser Glu Ala Lys Glu Ile Lys Ser Gln Leu Glu Asn Gln
945                 950                 955                 960

Lys Val Lys Trp Glu Gln Glu Leu Cys Ser Val Arg Leu Thr Leu Asn
                965                 970                 975

Gln Glu Glu Glu Lys Arg Arg Asn Ala Asp Ile Leu Asn Glu Lys Ile
            980                 985                 990

Arg Glu Glu Leu Gly Arg Ile Glu Glu Gln His Arg Lys Glu Leu Glu
        995                 1000                1005

Val Lys Gln Gln Leu Glu Gln Ala Leu Arg Ile Gln Asp Ile Glu
    1010                1015                1020

Leu Lys Ser Val Glu Ser Asn Leu Asn Gln Val Ser His Thr His
    1025                1030                1035

Glu Asn Glu Asn Tyr Leu Leu His Glu Asn Cys Met Leu Lys Lys
    1040                1045                1050

Glu Ile Ala Met Leu Lys Leu Glu Ile Ala Thr Leu Lys His Gln
    1055                1060                1065

Tyr Gln Glu Lys Glu Asn Lys Tyr Phe Glu Asp Ile Lys Ile Leu

1070                1075                1080

Lys Glu Lys Asn Ala Glu Leu Gln Met Thr Leu Lys Leu Lys Glu
    1085                1090                1095

Glu Ser Leu Thr Lys Arg Ala Ser Gln Tyr Ser Gly Gln Leu Lys
    1100                1105                1110

Val Leu Ile Ala Glu Asn Thr Met Leu Thr Ser Lys Leu Lys Glu
    1115                1120                1125

Lys Gln Asp Lys Glu Ile Leu Glu Ala Glu Ile Glu Ser His His
    1130                1135                1140

Pro Arg Leu Ala Ser Ala Val Gln Asp His Asp Gln Ile Val Thr
    1145                1150                1155

Ser Arg Lys Ser Gln Glu Pro Ala Phe His Ile Ala Gly Asp Ala
    1160                1165                1170

Cys Leu Gln Arg Lys Met Asn Val Asp Val Ser Ser Thr Ile Tyr
    1175                1180                1185

Asn Asn Glu Val Leu His Gln Pro Leu Ser Glu Ala Gln Arg Lys
    1190                1195                1200

Ser Lys Ser Leu Lys Ile Asn Leu Asn Tyr Ala Gly Asp Ala Leu
    1205                1210                1215

Arg Glu Asn Thr Leu Val Ser Glu His Ala Gln Arg Asp Gln Arg
    1220                1225                1230

Glu Thr Gln Cys Gln Met Lys Glu Ala Glu His Met Tyr Gln Asn
    1235                1240                1245

Glu Gln Asp Asn Val Asn Lys His Thr Glu Gln Gln Glu Ser Leu
    1250                1255                1260

Asp Gln Lys Leu Phe Gln Leu Gln Ser Lys Asn Met Trp Leu Gln
    1265                1270                1275

Gln Gln Leu Val His Ala His Lys Lys Ala Asp Asn Lys Ser Lys
    1280                1285                1290

Ile Thr Ile Asp Ile His Phe Leu Glu Arg Lys Met Gln His His
    1295                1300                1305

Leu Leu Lys Glu Lys Asn Glu Glu Ile Phe Asn Tyr Asn Asn His
    1310                    1315                    1320

Leu Lys Asn Arg Ile Tyr Gln Tyr Glu Lys Glu Lys Ala Glu Thr
    1325                    1330                    1335

Glu Asn Ser
    1340

<210> 148
<211> 142
<212> PRT
<213> Homo sapiens

<400> 148

Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Ile Glu Glu His Lys Lys His
65                  70                  75                  80

Ser Ser Gly Cys Ala Phe Leu Ser Val Lys Lys Gln Phe Glu Glu Leu
                85                  90                  95

Thr Leu Gly Glu Phe Leu Lys Leu Asp Arg Glu Arg Ala Lys Asn Lys
            100                 105                 110

Ile Ala Lys Glu Thr Asn Asn Lys Lys Lys Glu Phe Glu Glu Thr Ala
        115                 120                 125

Lys Lys Val Arg Arg Ala Ile Glu Gln Leu Ala Ala Met Asp
    130                 135                 140

<210> 149
<211> 429
<212> PRT
<213> Homo sapiens

<400> 149

Met Glu Thr Gln Phe Arg Arg Gly Gly Leu Gly Cys Ser Pro Ala Ser

1               5                   10                  15

Ile Lys Arg Lys Lys Lys Arg Glu Asp Ser Gly Asp Phe Gly Leu Gln
            20                  25                  30

Val Ser Thr Met Phe Ser Glu Asp Asp Phe Gln Ser Thr Glu Arg Ala
        35                  40                  45

Pro Tyr Gly Pro Gln Leu Gln Trp Ser Gln Asp Leu Pro Arg Val Gln
    50                  55                  60

Val Phe Arg Glu Gln Ala Asn Leu Glu Asp Arg Ser Pro Arg Arg Thr
65                  70                  75                  80

Gln Arg Ile Thr Gly Gly Glu Gln Val Leu Trp Gly Pro Ile Thr Gln
                85                  90                  95

Ile Phe Pro Thr Val Arg Pro Ala Asp Leu Thr Arg Val Ile Met Pro
            100                 105                 110

Leu Glu Gln Arg Ser Gln His Cys Lys Pro Glu Glu Gly Leu Gln Ala
        115                 120                 125

Gln Glu Glu Asp Leu Gly Leu Val Gly Ala Gln Ala Leu Gln Ala Glu
    130                 135                 140

Glu Gln Glu Ala Ala Phe Phe Ser Ser Thr Leu Asn Val Gly Thr Leu
145                 150                 155                 160

Glu Glu Leu Pro Ala Ala Glu Ser Pro Ser Pro Pro Gln Ser Pro Gln
                165                 170                 175

Glu Glu Ser Phe Ser Pro Thr Ala Met Asp Ala Ile Phe Gly Ser Leu
            180                 185                 190

Ser Asp Glu Gly Ser Gly Ser Gln Glu Lys Glu Gly Pro Ser Thr Ser
        195                 200                 205

Pro Asp Leu Ile Asp Pro Glu Ser Phe Ser Gln Asp Ile Leu His Asp
    210                 215                 220

Lys Ile Ile Asp Leu Val His Leu Leu Leu Arg Lys Tyr Arg Val Lys
225                 230                 235                 240

Gly Leu Ile Thr Lys Ala Glu Met Leu Gly Ser Val Ile Lys Asn Tyr
                245                 250                 255

Glu Asp Tyr Phe Pro Glu Ile Phe Arg Glu Ala Ser Val Cys Met Gln
            260                 265                 270

Leu Leu Phe Gly Ile Asp Val Lys Glu Val Asp Pro Thr Ser His Ser
        275                 280                 285

Tyr Val Leu Val Thr Ser Leu Asn Leu Ser Tyr Asp Gly Ile Gln Cys
    290                 295                 300

Asn Glu Gln Ser Met Pro Lys Ser Gly Leu Leu Ile Ile Val Leu Gly
305                 310                 315                 320

Val Ile Phe Met Glu Gly Asn Cys Ile Pro Glu Glu Val Met Trp Glu
                325                 330                 335

Val Leu Ser Ile Met Gly Val Tyr Ala Gly Arg Glu His Phe Leu Phe
            340                 345                 350

Gly Glu Pro Lys Arg Leu Leu Thr Gln Asn Trp Val Gln Glu Lys Tyr
        355                 360                 365

Leu Val Tyr Arg Gln Val Pro Gly Thr Asp Pro Ala Cys Tyr Glu Phe
    370                 375                 380

Leu Trp Gly Pro Arg Ala His Ala Glu Thr Ser Lys Met Lys Val Leu
385                 390                 395                 400

Glu Tyr Ile Ala Asn Ala Asn Gly Arg Asp Pro Thr Ser Tyr Pro Ser
                405                 410                 415

Leu Tyr Glu Asp Ala Leu Arg Glu Glu Gly Glu Gly Val
            420                 425

<210>  150
<211>  509
<212>  PRT
<213>  Homo sapiens

<400>  150

Met Glu Arg Arg Arg Leu Trp Gly Ser Ile Gln Ser Arg Tyr Ile Ser
1               5                   10                  15

Met Ser Val Trp Thr Ser Pro Arg Arg Leu Val Glu Leu Ala Gly Gln
            20                  25                  30

Ser Leu Leu Lys Asp Glu Ala Leu Ala Ile Ala Ala Leu Glu Leu Leu
        35                  40                  45

Pro Arg Glu Leu Phe Pro Pro Leu Phe Met Ala Ala Phe Asp Gly Arg
    50                  55                  60

His Ser Gln Thr Leu Lys Ala Met Val Gln Ala Trp Pro Phe Thr Cys
65                  70                  75                  80

Leu Pro Leu Gly Val Leu Met Lys Gly Gln His Leu His Leu Glu Thr
                85                  90                  95

Phe Lys Ala Val Leu Asp Gly Leu Asp Val Leu Leu Ala Gln Glu Val
            100                 105                 110

Arg Pro Arg Arg Trp Lys Leu Gln Val Leu Asp Leu Arg Lys Asn Ser
        115                 120                 125

His Gln Asp Phe Trp Thr Val Trp Ser Gly Asn Arg Ala Ser Leu Tyr
    130                 135                 140

Ser Phe Pro Glu Pro Glu Ala Ala Gln Pro Met Thr Lys Lys Arg Lys
145                 150                 155                 160

Val Asp Gly Leu Ser Thr Glu Ala Glu Gln Pro Phe Ile Pro Val Glu
                165                 170                 175

Val Leu Val Asp Leu Phe Leu Lys Glu Gly Ala Cys Asp Glu Leu Phe
            180                 185                 190

Ser Tyr Leu Ile Glu Lys Val Lys Arg Lys Lys Asn Val Leu Arg Leu
        195                 200                 205

Cys Cys Lys Lys Leu Lys Ile Phe Ala Met Pro Met Gln Asp Ile Lys
    210                 215                 220

Met Ile Leu Lys Met Val Gln Leu Asp Ser Ile Glu Asp Leu Glu Val
225                 230                 235                 240

Thr Cys Thr Trp Lys Leu Pro Thr Leu Ala Lys Phe Ser Pro Tyr Leu
                245                 250                 255

Gly Gln Met Ile Asn Leu Arg Arg Leu Leu Leu Ser His Ile His Ala
            260                 265                 270

Ser Ser Tyr Ile Ser Pro Glu Lys Glu Glu Gln Tyr Ile Ala Gln Phe
        275                 280                 285

Thr Ser Gln Phe Leu Ser Leu Gln Cys Leu Gln Ala Leu Tyr Val Asp
    290                 295                 300

Ser Leu Phe Phe Leu Arg Gly Arg Leu Asp Gln Leu Leu Arg His Val
305                 310                 315                 320

Met Asn Pro Leu Glu Thr Leu Ser Ile Thr Asn Cys Arg Leu Ser Glu
                325                 330                 335

Gly Asp Val Met His Leu Ser Gln Ser Pro Ser Val Ser Gln Leu Ser
            340                 345                 350

Val Leu Ser Leu Ser Gly Val Met Leu Thr Asp Val Ser Pro Glu Pro
        355                 360                 365

Leu Gln Ala Leu Leu Glu Arg Ala Ser Ala Thr Leu Gln Asp Leu Val
    370                 375                 380

Phe Asp Glu Cys Gly Ile Thr Asp Asp Gln Leu Leu Ala Leu Leu Pro
385                 390                 395                 400

Ser Leu Ser His Cys Ser Gln Leu Thr Thr Leu Ser Phe Tyr Gly Asn
                405                 410                 415

Ser Ile Ser Ile Ser Ala Leu Gln Ser Leu Leu Gln His Leu Ile Gly
            420                 425                 430

Leu Ser Asn Leu Thr His Val Leu Tyr Pro Val Pro Leu Glu Ser Tyr
        435                 440                 445

Glu Asp Ile His Gly Thr Leu His Leu Glu Arg Leu Ala Tyr Leu His
    450                 455                 460

Ala Arg Leu Arg Glu Leu Leu Cys Glu Leu Gly Arg Pro Ser Met Val
465                 470                 475                 480

Trp Leu Ser Ala Asn Pro Cys Pro His Cys Gly Asp Arg Thr Phe Tyr
                485                 490                 495

Asp Pro Glu Pro Ile Leu Cys Pro Cys Phe Met Pro Asn
            500                 505

<210>  151
<211>  315
<212>  PRT
<213>  Homo sapiens

<400>  151

Met Ser Leu Glu Gln Arg Ser Pro His Cys Lys Pro Asp Glu Asp Leu
1               5                   10                  15

Glu Ala Gln Gly Glu Asp Leu Gly Leu Met Gly Ala Gln Glu Pro Thr
            20                  25                  30

Gly Glu Glu Glu Glu Thr Thr Ser Ser Ser Asp Ser Lys Glu Glu Glu
        35                  40                  45

Val Ser Ala Ala Gly Ser Ser Ser Pro Pro Gln Ser Pro Gln Gly Gly
    50                  55                  60

Ala Ser Ser Ser Ile Ser Val Tyr Tyr Thr Leu Trp Ser Gln Phe Asp
65                  70                  75                  80

Glu Gly Ser Ser Ser Gln Glu Glu Glu Glu Pro Ser Ser Ser Val Asp
                85                  90                  95

Pro Ala Gln Leu Glu Phe Met Phe Gln Glu Ala Leu Lys Leu Lys Val
            100                 105                 110

Ala Glu Leu Val His Phe Leu Leu His Lys Tyr Arg Val Lys Glu Pro
        115                 120                 125

Val Thr Lys Ala Glu Met Leu Glu Ser Val Ile Lys Asn Tyr Lys Arg
    130                 135                 140

Tyr Phe Pro Val Ile Phe Gly Lys Ala Ser Glu Phe Met Gln Val Ile
145                 150                 155                 160

Phe Gly Thr Asp Val Lys Glu Val Asp Pro Ala Gly His Ser Tyr Ile
                165                 170                 175

Leu Val Thr Ala Leu Gly Leu Ser Cys Asp Ser Met Leu Gly Asp Gly
            180                 185                 190

His Ser Met Pro Lys Ala Ala Leu Leu Ile Ile Val Leu Gly Val Ile
        195                 200                 205

Leu Thr Lys Asp Asn Cys Ala Pro Glu Glu Val Ile Trp Glu Ala Leu
    210                 215                 220

Ser Val Met Gly Val Tyr Val Gly Lys Glu His Met Phe Tyr Gly Glu
225                 230                 235                 240

Pro Arg Lys Leu Leu Thr Gln Asp Trp Val Gln Glu Asn Tyr Leu Glu
                245                 250                 255

Tyr Arg Gln Val Pro Gly Ser Asp Pro Ala His Tyr Glu Phe Leu Trp
            260                 265                 270

Gly Ser Lys Ala His Ala Glu Thr Ser Tyr Glu Lys Val Ile Asn Tyr
        275                 280                 285

Leu Val Met Leu Asn Ala Arg Glu Pro Ile Cys Tyr Pro Ser Leu Tyr
    290                 295                 300

Glu Glu Val Leu Gly Glu Glu Gln Glu Gly Val
305                 310                 315

<210>  152
<211>  313
<212>  PRT
<213>  Homo sapiens

<400>  152

Met Ala Ser Phe Arg Lys Leu Thr Leu Ser Glu Lys Val Pro Pro Asn
1               5                   10                  15

His Pro Ser Arg Lys Lys Val Asn Phe Leu Asp Met Ser Leu Asp Asp
            20                  25                  30

Ile Ile Ile Tyr Lys Glu Leu Glu Gly Thr Asn Ala Glu Glu Glu Lys
        35                  40                  45

Asn Lys Arg Gln Asn His Ser Lys Lys Glu Ser Pro Ser Arg Gln Gln
    50                  55                  60

Ser Lys Ala His Arg His Arg His Arg Arg Gly Tyr Ser Arg Cys Arg
65                  70                  75                  80

Ser Asn Ser Glu Glu Gly Asn His Asp Lys Lys Pro Ser Gln Lys Pro
                85                  90                  95

Ser Gly Phe Lys Ser Gly Gln His Pro Leu Asn Gly Gln Pro Leu Ile
            100                 105                 110

Glu Gln Glu Lys Cys Ser Asp Asn Tyr Glu Ala Gln Ala Glu Lys Asn
        115                 120                 125

Gln Gly Gln Ser Glu Gly Asn Gln His Gln Ser Glu Gly Asn Pro Asp
    130                 135                 140

Lys Ser Glu Glu Ser Gln Gly Gln Pro Glu Glu Asn His His Ser Glu
145                 150                 155                 160

Arg Ser Arg Asn His Leu Glu Arg Ser Leu Ser Gln Ser Asp Arg Ser
                165                 170                 175

Gln Gly Gln Leu Lys Arg His His Pro Gln Tyr Glu Arg Ser His Gly
        180                 185                 190

Gln Tyr Lys Arg Ser His Gly Gln Ser Glu Arg Ser His Gly His Ser
    195                 200                 205

Glu Arg Ser His Gly His Ser Glu Arg Ser His Gly His Ser Glu Arg
    210                 215                 220

Ser His Gly His Ser Lys Arg Ser Arg Ser Gln Gly Asp Leu Val Asp
225                 230                 235                 240

Thr Gln Ser Asp Leu Ile Ala Thr Gln Arg Asp Leu Ile Ala Thr Gln
                245                 250                 255

Lys Asp Leu Ile Ala Thr Gln Arg Asp Leu Ile Ala Thr Gln Arg Asp
            260                 265                 270

Leu Ile Val Thr Gln Arg Asp Leu Val Ala Thr Glu Arg Asp Leu Ile
        275                 280                 285

Asn Gln Ser Gly Arg Ser His Gly Gln Ser Glu Arg His Gln Arg Tyr
    290                 295                 300

Ser Thr Gly Lys Asn Thr Ile Thr Thr
305                 310

<210>  153
<211>  385
<212>  PRT
<213>  Homo sapiens

<400>  153

Met Gly Arg Trp Cys Gln Thr Val Ala Arg Gly Gln Arg Pro Arg Thr
1               5                   10                  15

Ser Ala Pro Ser Arg Ala Gly Ala Leu Leu Leu Leu Leu Leu Leu Leu
            20                  25                  30

Arg Ser Ala Gly Cys Trp Gly Ala Gly Glu Ala Pro Gly Ala Leu Ser
        35                  40                  45

Thr Ala Asp Pro Ala Asp Gln Ser Val Gln Cys Val Pro Lys Ala Thr
    50                  55                  60

Cys Pro Ser Ser Arg Pro Arg Leu Leu Trp Gln Thr Pro Thr Thr Gln
65                  70                  75                  80

Thr Leu Pro Ser Thr Thr Met Glu Thr Gln Phe Pro Val Ser Glu Gly
                85                  90                  95

Lys Val Asp Pro Tyr Arg Ser Cys Gly Phe Ser Tyr Glu Gln Asp Pro
            100                 105                 110

Thr Leu Arg Asp Pro Glu Ala Val Ala Arg Arg Trp Pro Trp Met Val
        115                 120                 125

Ser Val Arg Ala Asn Gly Thr His Ile Cys Ala Gly Thr Ile Ile Ala
    130                 135                 140

Ser Gln Trp Val Leu Thr Val Ala His Cys Leu Ile Trp Arg Asp Val
145                 150                 155                 160

Ile Tyr Ser Val Arg Val Gly Ser Pro Trp Ile Asp Gln Met Thr Gln
                165                 170                 175

Thr Ala Ser Asp Val Pro Val Leu Gln Val Ile Met His Ser Arg Tyr
            180                 185                 190

Arg Ala Gln Arg Phe Trp Ser Trp Val Gly Gln Ala Asn Asp Ile Gly
        195                 200                 205

Leu Leu Lys Leu Lys Gln Glu Leu Lys Tyr Ser Asn Tyr Val Arg Pro
    210                 215                 220

Ile Cys Leu Pro Gly Thr Asp Tyr Val Leu Lys Asp His Ser Arg Cys
225                 230                 235                 240

Thr Val Thr Gly Trp Gly Leu Ser Lys Ala Asp Gly Met Trp Pro Gln
                245                 250                 255

Phe Arg Thr Ile Gln Glu Lys Glu Val Ile Ile Leu Asn Asn Lys Glu
            260                 265                 270

Cys Asp Asn Phe Tyr His Asn Phe Thr Lys Ile Pro Thr Leu Val Gln
        275                 280                 285

Ile Ile Lys Ser Gln Met Met Cys Ala Glu Asp Thr His Arg Glu Lys
    290                 295                 300

Phe Cys Tyr Glu Leu Thr Gly Glu Pro Leu Val Cys Ser Met Glu Gly
305                 310                 315                 320

Thr Trp Tyr Leu Val Gly Leu Val Ser Trp Gly Ala Gly Cys Gln Lys

                325                 330                 335

Ser Glu Ala Pro Pro Ile Tyr Leu Gln Val Ser Ser Tyr Gln His Trp
            340                 345                 350

Ile Trp Asp Cys Leu Asn Gly Gln Ala Leu Ala Leu Pro Ala Pro Ser
        355                 360                 365

Arg Thr Leu Leu Leu Ala Leu Pro Leu Pro Leu Ser Leu Leu Ala Ala
    370                 375                 380

Leu
385

<210> 154
<211> 314
<212> PRT
<213> Homo sapiens

<400> 154

Met Ala Pro Pro Gln Val Leu Ala Phe Gly Leu Leu Leu Ala Ala Ala
1               5                   10                  15

Thr Ala Thr Phe Ala Ala Ala Gln Glu Glu Cys Val Cys Glu Asn Tyr
            20                  25                  30

Lys Leu Ala Val Asn Cys Phe Val Asn Asn Asn Arg Gln Cys Gln Cys
        35                  40                  45

Thr Ser Val Gly Ala Gln Asn Thr Val Ile Cys Ser Lys Leu Ala Ala
    50                  55                  60

Lys Cys Leu Val Met Lys Ala Glu Met Asn Gly Ser Lys Leu Gly Arg
65                  70                  75                  80

Arg Ala Lys Pro Glu Gly Ala Leu Gln Asn Asn Asp Gly Leu Tyr Asp
                85                  90                  95

Pro Asp Cys Asp Glu Ser Gly Leu Phe Lys Ala Lys Gln Cys Asn Gly
            100                 105                 110

Thr Ser Met Cys Trp Cys Val Asn Thr Ala Gly Val Arg Arg Thr Asp
        115                 120                 125

Lys Asp Thr Glu Ile Thr Cys Ser Glu Arg Val Arg Thr Tyr Trp Ile
    130                 135                 140

Ile Ile Glu Leu Lys His Lys Ala Arg Glu Lys Pro Tyr Asp Ser Lys

145                 150                 155                 160

Ser Leu Arg Thr Ala Leu Gln Lys Glu Ile Thr Thr Arg Tyr Gln Leu
                165                 170                 175

Asp Pro Lys Phe Ile Thr Ser Ile Leu Tyr Glu Asn Asn Val Ile Thr
            180                 185                 190

Ile Asp Leu Val Gln Asn Ser Ser Gln Lys Thr Gln Asn Asp Val Asp
        195                 200                 205

Ile Ala Asp Val Ala Tyr Tyr Phe Glu Lys Asp Val Lys Gly Glu Ser
    210                 215                 220

Leu Phe His Ser Lys Lys Met Asp Leu Thr Val Asn Gly Glu Gln Leu
225                 230                 235                 240

Asp Leu Asp Pro Gly Gln Thr Leu Ile Tyr Tyr Val Asp Glu Lys Ala
                245                 250                 255

Pro Glu Phe Ser Met Gln Gly Leu Lys Ala Gly Val Ile Ala Val Ile
            260                 265                 270

Val Val Val Val Ile Ala Val Val Ala Gly Ile Val Val Leu Val Ile
        275                 280                 285

Ser Arg Lys Lys Arg Met Ala Lys Tyr Glu Lys Ala Glu Ile Lys Glu
    290                 295                 300

Met Gly Glu Met His Arg Glu Leu Asn Ala
305                 310

<210>  155
<211>  777
<212>  PRT
<213>  Homo sapiens

<400>  155

Met Asn Lys Asp Tyr Gln Lys Phe Trp Ser Ser Pro Ser Asp Pro Val
1               5                   10                  15

His Phe Glu Val Asp Thr Ser His Glu Lys Val Glu Ser Met Ser Glu
            20                  25                  30

Ser Asp Thr Met Asn Val Ser Asn Leu Ser Gln Gly Val Met Leu Ser
        35                  40                  45

His Ser Pro Ile Cys Met Glu Thr Thr Gly Thr Thr Cys Asp Leu Pro

50                  55                  60

Gln Asn Glu Ile Lys Asn Phe Glu Arg Glu Asn Glu Tyr Glu Ser Thr
65                  70                  75                  80

Leu Cys Glu Asp Ala Tyr Gly Thr Leu Asp Asn Leu Leu Asn Asp Asn
                85                  90                  95

Asn Ile Glu Asn Tyr Ser Thr Asn Ala Leu Ile Gln Pro Val Asp Thr
            100                 105                 110

Ile Ser Ile Ser Ser Leu Arg Gln Phe Glu Thr Val Cys Lys Phe His
        115                 120                 125

Trp Val Glu Ala Phe Asp Asp Glu Met Thr Glu Lys Pro Glu Phe Gln
    130                 135                 140

Ser Gln Val Tyr Asn Tyr Ala Lys Asp Asn Asn Ile Lys Gln Asp Ser
145                 150                 155                 160

Phe Lys Glu Glu Asn Pro Met Glu Thr Ser Val Ser Ala Asn Thr Asp
                165                 170                 175

Gln Leu Gly Asn Glu Tyr Phe Arg Gln Pro Pro Pro Arg Ser Pro Pro
            180                 185                 190

Leu Ile His Cys Ser Gly Glu Met Leu Lys Phe Thr Glu Lys Ser Leu
        195                 200                 205

Ala Lys Ser Ile Ala Lys Glu Ser Ala Leu Asn Pro Ser Gln Pro Pro
    210                 215                 220

Ser Phe Leu Cys Lys Thr Ala Val Pro Ser Lys Glu Ile Gln Asn Tyr
225                 230                 235                 240

Gly Glu Ile Pro Glu Met Ser Val Ser Tyr Glu Lys Glu Val Thr Ala
                245                 250                 255

Glu Gly Val Glu Arg Pro Glu Ile Val Ser Thr Trp Ser Ser Ala Gly
            260                 265                 270

Ile Ser Trp Arg Ser Glu Ala Cys Arg Glu Asn Cys Glu Met Pro Asp
        275                 280                 285

Trp Glu Gln Ser Ala Glu Ser Leu Gln Pro Val Gln Glu Asp Met Ala
    290                 295                 300

Leu Asn Glu Val Leu Gln Lys Leu Lys His Thr Asn Arg Lys Gln Glu
305                 310                 315                 320

Val Arg Ile Gln Glu Leu Gln Cys Ser Asn Leu Tyr Leu Glu Lys Arg
                325                 330                 335

Val Lys Glu Leu Gln Met Lys Ile Thr Lys Gln Gln Val Phe Ile Asp
            340                 345                 350

Val Ile Asn Lys Leu Lys Glu Asn Val Glu Glu Leu Ile Glu Asp Lys
        355                 360                 365

Tyr Lys Ile Ile Leu Glu Lys Asn Asp Thr Lys Lys Thr Leu Gln Asn
    370                 375                 380

Leu Glu Glu Val Leu Ala Asn Thr Gln Lys His Leu Gln Glu Ser Arg
385                 390                 395                 400

Asn Asp Lys Glu Met Leu Gln Leu Gln Phe Lys Lys Ile Lys Ala Asn
                405                 410                 415

Tyr Val Cys Leu Gln Glu Arg Tyr Met Thr Glu Met Gln Gln Lys Asn
            420                 425                 430

Lys Ser Val Ser Gln Tyr Leu Glu Met Asp Lys Thr Leu Ser Lys Lys
        435                 440                 445

Glu Glu Glu Val Glu Arg Leu Gln Gln Leu Lys Lys Glu Leu Glu Lys
    450                 455                 460

Ala Thr Ala Ser Ala Leu Asp Leu Leu Lys Arg Glu Lys Glu Ala Gln
465                 470                 475                 480

Glu Gln Glu Phe Leu Ser Leu Gln Glu Glu Phe Gln Lys Leu Glu Lys
                485                 490                 495

Glu Asn Leu Glu Glu Arg Gln Lys Leu Lys Ser Arg Leu Glu Lys Leu
            500                 505                 510

Leu Thr Gln Val Arg Asn Leu Gln Phe Met Ser Glu Asn Glu Arg Thr
        515                 520                 525

Lys Asn Ile Lys Leu Gln Gln Gln Ile Asn Glu Val Lys Asn Glu Asn
    530                 535                 540

Ala Lys Leu Lys Gln Gln Val Ala Arg Ser Glu Glu Gln Asn Tyr Val
545                 550                 555                 560

Pro Lys Phe Glu Thr Ala Gln Leu Lys Asp Gln Leu Glu Glu Val Leu
                565                 570                 575

Lys Ser Asp Ile Thr Lys Asp Thr Lys Thr Thr His Ser Asn Leu Leu
            580                 585                 590

Pro Asp Cys Ser Pro Cys Glu Glu Arg Leu Asn Pro Ala Asp Ile Lys
        595                 600                 605

Arg Ala Ser Gln Leu Ala Ser Lys Met His Ser Leu Leu Ala Leu Met
    610                 615                 620

Val Gly Leu Leu Thr Cys Gln Asp Ile Ile Asn Ser Asp Ala Glu His
625                 630                 635                 640

Phe Lys Glu Ser Glu Lys Val Ser Asp Ile Met Leu Gln Lys Leu Lys
                645                 650                 655

Ser Leu His Leu Lys Lys Lys Thr Leu Asp Lys Glu Val Ile Asp Cys
            660                 665                 670

Asp Ser Asp Glu Ala Lys Ser Ile Arg Asp Val Pro Thr Leu Leu Gly
        675                 680                 685

Ala Lys Leu Asp Lys Tyr His Ser Leu Asn Glu Glu Leu Asp Phe Leu
    690                 695                 700

Val Thr Ser Tyr Glu Glu Ile Ile Glu Cys Ala Asp Gln Arg Leu Ala
705                 710                 715                 720

Ile Ser His Ser Gln Ile Ala His Leu Glu Glu Arg Asn Lys His Leu
                725                 730                 735

Glu Asp Leu Ile Arg Lys Pro Arg Glu Lys Ala Arg Lys Pro Arg Ser
            740                 745                 750

Lys Ser Leu Glu Asn His Pro Lys Ser Met Thr Met Met Pro Ala Leu
        755                 760                 765

Phe Lys Glu Asn Arg Asn Asp Leu Asp
    770                 775

<210>  156
<211>  442
<212>  PRT
<213>  Homo sapiens

<400>  156

Met Asp Glu Glu Ser Glu Leu Ile Gln Pro Gln Asp Gln Ser Cys Trp
1               5                   10                  15

Ala Phe Leu Pro Asp Leu Cys Leu Cys Arg Val Phe Trp Trp Leu Gly
            20                  25                  30

Asp Arg Asp Arg Ser Arg Ala Ala Leu Val Cys Arg Lys Trp Asn Gln
        35                  40                  45

Met Met Tyr Ser Ala Glu Leu Trp Arg Tyr Arg Thr Ile Thr Phe Ser
    50                  55                  60

Gly Arg Pro Ser Arg Val His Ala Ser Glu Val Glu Ser Ala Val Trp
65                  70                  75                  80

Tyr Val Lys Lys Phe Gly Arg Tyr Leu Glu His Leu Glu Val Lys Phe
                85                  90                  95

Met Asn Pro Tyr Asn Ala Val Leu Thr Lys Lys Phe Gln Val Thr Met
            100                 105                 110

Arg Gly Leu Leu Ser Cys Leu Ser Lys Ser Asn Asn Arg Leu Lys Ser
        115                 120                 125

Leu Ser Ile Gln Tyr Leu Glu Leu Asp Arg Leu Val Trp Arg Asn Ser
    130                 135                 140

Ile Arg Ser Ser Phe Ile Ser Ser Leu Ser Phe Phe Leu Lys Lys Met
145                 150                 155                 160

Gly Lys Arg Leu Asp Tyr Leu Asn Leu Lys Gly Ala Arg Leu Thr Val
                165                 170                 175

Glu Gln Gly Cys Gln Ile Leu Asp Ser Leu Ser Tyr Met Arg Asn Glu
            180                 185                 190

Asn Val Ile Ser Glu Leu Asn Ile Glu Asp Tyr Phe Ser His His Leu
        195                 200                 205

Ala Val Tyr Asn Ser Pro Gln Phe Lys Lys Thr Met Ser Thr Phe His
    210                 215                 220

Asn Leu Val Ser Leu Asn Leu Asn Tyr Asn Cys Ile Ser Asp Glu Leu
225                 230                 235                 240

Leu Glu Asn Leu Cys Glu Asn Ala Ser Thr Leu Arg Thr Ile Asn Ile
                245                 250                 255

Lys Cys His Val His Asp Pro His Gly Gln Val Ile Trp Gly Met Ser
            260                 265                 270

Trp Ala Lys Leu Ala Arg Gln Ala Thr Asn Leu Lys Val Asn Phe Phe
        275                 280                 285

Phe Glu Arg Ile Met Lys Tyr Glu Arg Leu Ala Arg Ile Leu Leu Gln
    290                 295                 300

Glu Ile Pro Ile Arg Ser Ile Ser Leu Arg Ser Cys Tyr Phe Ser Asp
305                 310                 315                 320

Pro Asp Cys Ser Met Arg Pro Thr Leu Ile Asp Leu Leu Pro Thr Phe
                325                 330                 335

Arg His Thr Leu Gln Lys Leu Thr Cys Glu Phe Asn Asn Asn His Glu
            340                 345                 350

Ser Leu Asp Glu Glu Leu His Leu Leu Ile Ile Ser Cys Arg Lys Leu
        355                 360                 365

Phe Tyr Phe Lys Ile Trp Ala Phe Leu Asp Val Ser Phe Val Glu Arg
    370                 375                 380

Ile Leu Lys Ser Gln Lys Glu Arg Gln Cys Ala Leu Arg Val Phe Lys
385                 390                 395                 400

Ala Arg Ile Tyr Thr Asn Arg Tyr Glu Thr Asn Glu Glu Asp Lys Thr
                405                 410                 415

Leu Gln Glu Ile Tyr Arg Lys Tyr Arg Lys Leu Ile Glu Ser Glu Leu
            420                 425                 430

Ser Tyr Phe Val Ile Val Tyr Ser Val Met
        435                 440

<210> 157
<211> 318
<212> PRT
<213> Homo sapiens

<400> 157
   Met Leu Leu Gly Gln Lys Ser Gln Arg Tyr Lys Ala Glu Glu Gly Leu
   1 5 10 15
   Gln Ala Gln Gly Glu Ala Pro Gly Leu Met Asp Val Gln Ile Pro Thr
   20 25 30
   Ala Glu Glu Gln Lys Ala Ala Ser Ser Ser Ser Thr Leu Ile Met Gly
           35                  40                  45
   Thr Leu Glu Glu Val Thr Asp Ser Gly Ser Pro Ser Pro Pro Gln Ser
       50                  55                  60
   Pro Glu Gly Ala Ser Ser Ser Leu Thr Val Thr Asp Ser Thr Leu Trp
   65                  70                  75                  80
   Ser Gln Ser Asp Glu Gly Ser Ser Ser Asn Glu Glu Glu Gly Pro Ser
                   85                  90                  95
   Thr Ser Pro Asp Pro Ala His Leu Glu Ser Leu Phe Arg Glu Ala Leu
               100                 105                 110
   Asp Glu Lys Val Ala Glu Leu Val Arg Phe Leu Leu Arg Lys Tyr Gln
           115                 120                 125
   Ile Lys Glu Pro Val Thr Lys Ala Glu Met Leu Glu Ser Val Ile Lys
       130                 135                 140
   Asn Tyr Lys Asn His Phe Pro Asp Ile Phe Ser Lys Ala Ser Glu Cys
   145                 150                 155                 160
   Met Gln Val Ile Phe Gly Ile Asp Val Lys Glu Val Asp Pro Ala Gly
                   165                 170                 175
   His Ser Tyr Ile Leu Val Thr Cys Leu Gly Leu Ser Tyr Asp Gly Leu
               180                 185                 190
   Leu Gly Asp Asp Gln Ser Thr Pro Lys Thr Gly Leu Leu Ile Ile Val
           195                 200                 205
   Leu Gly Met Ile Leu Met Glu Gly Ser Arg Ala Pro Glu Glu Ala Ile
       210                 215                 220
   Trp Glu Ala Leu Ser Val Met Gly Leu Tyr Asp Gly Arg Glu His Ser
   225                 230                 235                 240
   Val Tyr Trp Lys Leu Arg Lys Leu Leu Thr Gln Glu Trp Val Gln Glu
                   245                 250                 255
   Asn Tyr Leu Glu Tyr Arg Gln Ala Pro Gly Ser Asp Pro Val Arg Tyr
               260                 265                 270
   Glu Phe Leu Trp Gly Pro Arg Ala Leu Ala Glu Thr Ser Tyr Val Lys
   275                 280                 285
   Val Leu Glu His Val Val Arg Val Asn Ala Arg Val Arg Ile Ser Tyr
       290                 295                 300
   Pro Ser Leu His Glu Glu Ala Leu Gly Glu Glu Lys Gly Val
   305                 310                 315

<210>  158
<211>  314
<212>  PRT
<213>  Homo sapiens

<400>  158

Met Pro Leu Glu Gln Arg Ser Gln His Cys Lys Pro Glu Glu Gly Leu
1               5                   10                  15

Glu Ala Arg Gly Glu Ala Leu Gly Leu Val Gly Ala Gln Ala Pro Ala
            20                  25                  30

Thr Glu Glu Gln Glu Ala Ala Ser Ser Ser Ser Thr Leu Val Glu Val
        35                  40                  45

Thr Leu Gly Glu Val Pro Ala Ala Glu Ser Pro Asp Pro Pro Gln Ser
    50                  55                  60

Pro Gln Gly Ala Ser Ser Leu Pro Thr Thr Met Asn Tyr Pro Leu Trp
65                  70                  75                  80

Ser Gln Ser Tyr Glu Asp Ser Ser Asn Gln Glu Glu Glu Gly Pro Ser
                85                  90                  95

Thr Phe Pro Asp Leu Glu Ser Glu Phe Gln Ala Ala Leu Ser Arg Lys
            100                 105                 110

Val Ala Lys Leu Val His Phe Leu Leu Leu Lys Tyr Arg Ala Arg Glu
        115                 120                 125

Pro Val Thr Lys Ala Glu Met Leu Gly Ser Val Val Gly Asn Trp Gln
    130                 135                 140

Tyr Phe Phe Pro Val Ile Phe Ser Lys Ala Ser Asp Ser Leu Gln Leu
145                 150                 155                 160

Val Phe Gly Ile Glu Leu Met Glu Val Asp Pro Ile Gly His Val Tyr
                165                 170                 175

Ile Phe Ala Thr Cys Leu Gly Leu Ser Tyr Asp Gly Leu Leu Gly Asp

180                 185                 190

Asn Gln Ile Met Pro Lys Thr Gly Phe Leu Ile Ile Ile Leu Ala Ile
        195                 200                 205

Ile Ala Lys Glu Gly Asp Cys Ala Pro Glu Glu Lys Ile Trp Glu Glu
    210                 215                 220

Leu Ser Val Leu Glu Val Phe Glu Gly Arg Glu Asp Ser Ile Phe Gly
225                 230                 235                 240

Asp Pro Lys Lys Leu Leu Thr Gln Tyr Phe Val Gln Glu Asn Tyr Leu
                245                 250                 255

Glu Tyr Arg Gln Val Pro Gly Ser Asp Pro Ala Cys Tyr Glu Phe Leu
            260                 265                 270

Trp Gly Pro Arg Ala Leu Ile Glu Thr Ser Tyr Val Lys Val Leu His
        275                 280                 285

His Met Val Lys Ile Ser Gly Gly Pro Arg Ile Ser Tyr Pro Leu Leu
    290                 295                 300

His Glu Trp Ala Leu Arg Glu Gly Glu Glu
305                 310

<210>  159
<211>  16
<212>  PRT
<213>  Artificial Sequence

<220>
<223>  JUP Peptide Vaccine

<400>  159

Met Met Asn Leu Met Gln Pro Lys Thr Gln Gln Thr Tyr Thr Tyr Asp
1               5                   10                  15

<210>  160
<211>  21
<212>  PRT
<213>  Artificial Sequence

<220>
<223>  ADA17 Peptide Vaccine

<400>  160

Gly Arg Gly Ser Thr Thr Thr Asn Tyr Leu Leu Asp Arg Asp Asp Tyr
1               5                   10                  15

Arg Asn Thr Ser Asp
            20

<210>  161
<211>  22
<212>  PRT
<213>  Artificial Sequence

<220>
<223>  BAP31 Peptide Vaccine

<400>  161

Leu Lys Lys Gly Ala Ala Asp Gly Gly Lys Leu Asp Gly Asn Ala Lys
1               5                   10                  15

Leu Asn Arg Ser Leu Lys
            20

<210>  162
<211>  22
<212>  PRT
<213>  Artificial Sequence

<220>
<223>  TOP2A Peptide Vaccine

<400>  162

Phe Pro Pro Lys Asp Asp His Thr Leu Lys Phe Leu Tyr Asp Asp Asn
1               5                   10                  15

Gln Arg Pro Tyr Pro Pro
            20

<210>  163
<211>  21
<212>  PRT
<213>  Artificial Sequence

<220>
<223>  Abl-2 Peptide Vaccine

<400>  163

Arg Tyr Arg Lys Pro Asp Tyr Thr Leu Asp Asp Gly His Gly Leu Leu
1               5                   10                  15

Arg Phe Lys Ser Thr
            20

<210>  164
<211>  18
<212>  PRT
<213>  Artificial Sequence

<220>
<223>  DDR1 Peptide Vaccine

<400>  164

Gln Arg Pro Pro Phe Ser Gln Leu His Arg Phe Leu Ala Asp Ala Leu
1               5                   10                  15

Asn Thr

<210>  165
<211>  25
<212>  PRT
<213>  Artificial Sequence

<220>
<223>  ITGB8 Peptide Vaccine

<400>  165

Ala Leu Asp Gln Cys Lys Thr Ser Cys Ala Leu Met Gln Gln His Tyr
1               5                   10                  15

Asp Gln Thr Ser Cys Phe Ser Ser Pro
            20                  25

<210>  166
<211>  25
<212>  PRT
<213>  Artificial Sequence

<220>
<223>  MUC-1 Peptide Vaccine

<400>  166

Ser Thr Ala Pro Pro Ala His Gly Val Thr Ser Ala Pro Asp Thr Arg
1               5                   10                  15

Pro Ala Pro Gly Ser Thr Ala Pro Pro
            20                  25

<210>  167
<211>  9
<212>  PRT
<213>  Artificial Sequence

<220>
<223>  gp100 Peptide Vaccine

<400>  167

Tyr Leu Glu Pro Gly Pro Val Thr Ala
1               5

<210>  168
<211>  21
<212>  PRT
<213>  Artificial Sequence

<220>
<223>  MUC-1 Peptide Vaccine

<400>  168

Met Thr Pro Gly Thr Gln Ser Pro Phe Phe Leu Leu Leu Leu Leu Thr
1               5                   10                  15

Val Leu Thr Val Val
            20

<210>  169
<211>  9
<212>  PRT
<213>  Artificial Sequence

<220>
<223>  Ber-Abl Peptide Vaccine

<400>  169

Ser Ser Lys Ala Leu Gln Arg Pro Val
1               5

<210>  170
<211>  9
<212>  PRT
<213>  Artificial Sequence

<220>
<223>  WT-1 Peptide Vaccine

<400>  170

Arg Met Phe Pro Asn Ala Pro Tyr Leu
1               5

<210>  171
<211>  9
<212>  PRT
<213>  Artificial Sequence

<220>
<223>  WT-1, HLA-A 0201 Peptide Vaccine

<400>  171

Arg Met Phe Pro Asn Ala Pro Tyr Leu
1               5